(19)

**Europäisches Patentamt**

**European Patent Office**

**Office européen des brevets**

(11) **EP 4 624 489 A1**

(12) **EUROPEAN PATENT APPLICATION**
published in accordance with Art. 153(4) EPC

(43) Date of publication:
01.10.2025 Bulletin 2025/40

(21) Application number: 23894661.0

(22) Date of filing: 24.11.2023

(51) International Patent Classification (IPC):
C07K 16/00 (2006.01)     A61K 39/395 (2006.01)
A61P 35/00 (2006.01)     C07K 16/28 (2006.01)
C07K 16/46 (2006.01)     C12N 15/13 (2006.01)

(52) Cooperative Patent Classification (CPC):
A61K 39/395; A61P 35/00; C07K 16/00;
C07K 16/28; C07K 16/46

(86) International application number:
PCT/JP2023/042131

(87) International publication number:
WO 2024/111657 (30.05.2024 Gazette 2024/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.11.2022 JP 2022187883

(71) Applicant: CHUGAI SEIYAKU KABUSHIKI KAISHA
Tokyo 115-8543 (JP)

(72) Inventors:
• FARUKSHINA, Katerina
Tokyo 115-8543 (JP)

• KAWADE, Raiji
Tokyo 115-8543 (JP)
• KOBAYASHI, Shohei
Tokyo 115-8543 (JP)
• NAKAYAMA, Junichi
Tokyo 115-8543 (JP)
• NONAKA, Takahiro
Tokyo 115-8543 (JP)

(74) Representative: Vossius & Partner
Patentanwälte Rechtsanwälte mbB
Siebertstraße 3
81675 München (DE)

Remarks:
The complete document including Reference
Table(s) and the Sequence Listing(s) can be
downloaded from the EPO website

(54) **METHOD FOR PRODUCING PROTEIN**

(57)     In one embodiment, it was discovered that a preparation containing antigen-binding molecules that have at least one disulfide bond formed between amino acid residues in regions other than the hinge region can be produced more efficiently and with high reproducibility by carrying out the following in chromatography:
contacting a mixture containing antigen-binding molecules having at least one disulfide bond formed between amino acid residues in regions other than the hinge region and mis-disulfide bonded forms and/or non-disulfide bonded forms of the antigen-binding molecules with a solution containing a reducing agent; and subsequently, removing the reducing agent.

| Cys concentration | [mM] | Skip | 0.1 | 1 | 10 | 100 | 0.1 | 1 | 10 | 100 |
|---|---|---|---|---|---|---|---|---|---|---|
| pH | [-] | Skip | 7 | 7 | 7 | 7 | 8 | 8 | 8 | 8 |
| LINC percentage | [%] | 62.2 | 96.2 | 97.6 | 79.1 | - | 97.2 | 99.4 | 93.9 | - |

FIG. 1

EP 4 624 489 A1

**Description**

[Technical Field]

The present disclosure relates to methods for producing antigen-binding molecules having at least one disulfide bond formed between amino acid residues in regions other than the hinge region. The present disclosure also relates to methods for increasing or enriching such antigen-binding molecules, methods for eliminating heterogeneity of the disulfide bonds of the antigen-binding molecules, and such.

[Background Art]

Antigen-binding molecules (LINC-Ig) having an artificial disulfide bond between amino acid residues in regions other than the hinge region ("LINC") are known (PTL 1 to PTL 4). For example, a LINC-Ig has a disulfide bond between the CH1 region of one of two antibody heavy chains constituting an antigen-binding molecule and the CH1 region of the other one of the antibody heavy chains. However, when such an antigen-binding molecule is expressed in a cell culture solution to obtain the molecule, two kinds of formats, molecules having appropriate disulfide bonds between the heavy chains (LINC format) and molecules without appropriate disulfide bonds such as mis-disulfide bonded forms and non-disulfide bonded forms of the molecules (unLINC format), will coexist in the cell culture solution. When preparing LINC-Ig, molecules in which appropriate disulfide bonds are not formed between the heavy chains, such as open-type molecules with capped sulfur atoms, included in the cell culture solution, must be converted to molecules having appropriate disulfide bonds.

The following are known as methods for obtaining LINC-Ig in which appropriate disulfide bond is formed between heavy chains *via* amino acid residues in regions other than the hinge region.

PTL 2 and PTL 4 disclose methods for efficiently obtaining antibodies that have LINC, comprising treating a preparation containing molecules having disulfide bonds between the heavy chains and molecules in which appropriate disulfide bonds are not formed with a reducing agent, and then forming disulfide bonds by performing re-oxidization by buffer exchange and such.

PTL 5 discloses the use of a redox buffer (for example, Cysteine/Cystine) so that a reduction-oxidation reaction can be performed in the Down Stream Process for the purpose of preventing fragmentation of antibodies.

PTL 6 discloses a method for refolding recombinant antibodies by contacting them with a reduction/oxidation coupling reagent.

NPL 1 discloses suppression of non-uniformity among lots caused by cysteinylation of a specific antibody (MAB007) using a redox buffer.

[Citation List]

[Patent Literature]

[PTL 1] WO2020/027330
[PTL 2] WO2021/157679
[PTL 3] WO2021/200898
[PTL 4] WO2021/201087
[PTL 5] WO2020/037016
[PTL 6] WO2006/047340

[Non-Patent Literature]

[NPL 1] Pharm Sci. 2008 Feb;97(2):775-90, Removal of Cysteinylation from an Unpaired Sulfhydryl in the Variable Region of a Recombinant Monoclonal IgG1 Antibody Improves Homogeneity, Stability, and Biological Activity

[Summary of Invention]

[Technical Problem]

As described above, a method for efficiently obtaining antigen-binding molecules that have LINC, by treating a mixture comprising antigen-binding molecules having appropriate disulfide bonds between heavy chains and antigen-binding molecules in which appropriate disulfide bonds are not formed (for example, capped molecules) with a reducing agent and then performing re-oxidation, is known. However, there were problems in this method from the perspective of productivity and ease of manufacture, including low yield and long reaction time.

In order to solve the above-mentioned problems, the present disclosure aims to provide methods for efficient and easy production and purification of antigen-binding molecules having appropriate disulfide bonds between antibody heavy chains. The present disclosure relates to methods for increasing structural homogeneity and relative abundance of antigen-binding molecules having one or more disulfide bonds formed between amino acid residues which are in regions other than the hinge region at each of the two antibody heavy chains. In other words, the present disclosure relates to methods for decreasing the relative abundance of antigen-binding molecules that do not have appropriate disulfide bonds between amino acid residues in regions other than the hinge region.

[Solution to Problem]

As a result of performing examinations to solve the above-mentioned problems, the present inventors discovered that it is possible to more efficiently and highly reproducibly form at least one disulfide bond between amino acid residues in regions other than the hinge region by contacting antigen-binding molecules with a reducing agent and then removing the reducing agent, wherein the antigen-binding molecules are bound to an affinity column and have amino acid residues that may form at least one disulfide bond between amino acid residues in regions other than the hinge region (these antigen-binding molecules include antigen-binding molecules in which appropriate disulfide bond is formed between amino acid residues in regions other than the hinge region and antigen-binding molecules in which at least one appropriate disulfide bond is not formed between amino acid residues in regions other than the hinge region). Specifically, the present inventors developed techniques to steadily obtain in high-yield antigen-binding molecules having at least one disulfide bond between amino acid residues in regions other than the hinge region (LINC-Ig format) by inserting two steps, which are allowing a solution comprising a reducing agent to flow and removing the reducing agent, into the step of purification by Protein A affinity chromatography, a universally used technique for primary purification from a cell culture solution (Harvested cell culture fluid: HCCF).

The present disclosure is based on these findings, and specifically relates to the following:

[1] A method for producing a preparation comprising an antigen-binding molecule having at least one disulfide bond formed between amino acid residues in regions other than a hinge region, wherein the method comprises subjecting to chromatography in the presence of a reducing agent an antigen-binding molecule having amino acid residues that may form at least one disulfide bond between amino acid residues in regions other than a hinge region.

[2] A method for producing a preparation comprising an antigen-binding molecule having at least one disulfide bond formed between amino acid residues in regions other than a hinge region, wherein the method comprises:

(a) contacting an antigen-binding molecule having amino acid residues that may form at least one disulfide bond between amino acid residues in regions other than a hinge region with a solution comprising a reducing agent in chromatography, and
(b) removing the reducing agent.

[3] A method for producing a preparation comprising an antigen-binding molecule having at least one disulfide bond formed between amino acid residues in regions other than a hinge region, wherein the method comprises subjecting to chromatography in the presence of a reducing agent a mixture which comprises:

an antigen-binding molecule having at least one disulfide bond formed between amino acid residues in regions other than a hinge region, and
a mis-disulfide bonded form and/or a non-disulfide bonded form of the antigen-binding molecule.

[4] A method for producing a preparation comprising an antigen-binding molecule having at least one disulfide bond formed between amino acid residues in regions other than a hinge region, wherein the method comprises:

(a) contacting a mixture which comprises:

an antigen-binding molecule having at least one disulfide bond formed between amino acid residues in regions other than a hinge region, and
a mis-disulfide bonded form and/or a non-disulfide bonded form of the antigen-binding molecule

with a solution comprising a reducing agent in chromatography; and

(b) removing the reducing agent.

[5] The method of any one of [1] to [4], wherein the antigen-binding molecule comprises two or more polypeptide chains, and the at least one disulfide bond formed between amino acid residues in regions other than a hinge region is formed between the polypeptide chains.

[6] The method of [5], wherein either or both of the polypeptide chains comprise mutated, substituted, or introduced cysteine residues in a region other than a hinge region.

[7] The method of any one of [1] to [6], wherein the antigen-binding molecule comprises a first antigen-binding domain and a second antigen-binding domain, and wherein the at least one disulfide bond formed between amino acid residues in regions other than a hinge region is formed between the first antigen-binding domain and the second antigen-binding domain.

[8] The method of any one of [1] to [7], wherein the antigen-binding molecule comprises a first antigen-binding domain and a second antigen-binding domain, and wherein the at least one disulfide bond formed between amino acid residues in regions other than a hinge region is formed between a heavy chain of the first antigen-binding domain and a heavy chain of the second antigen-binding domain.

[9] The method of any one of [1] to [8], wherein the antigen-binding molecule comprises a first antigen-binding domain and a second antigen-binding domain, and wherein the at least one disulfide bond formed between amino acid residues in regions other than a hinge region is formed between a CH1 region of the first antigen-binding domain and a CH1 region of the second antigen-binding domain.

[10] The method of any one of [1] to [9], wherein the antigen-binding molecule comprises a first antigen-binding domain and a second antigen-binding domain, and wherein the at least one disulfide bond formed between amino acid residues in regions other than a hinge region is formed between an amino acid residue at EU numbering position 191 in a heavy chain of the first antigen-binding domain and an amino acid residue at EU numbering position 191 in a heavy chain of the second antigen-binding domain.

[11] The method of any one of [1] to [10], wherein the chromatography comprises an affinity chromatography matrix, an ion exchange chromatography matrix, a hydrophobic interaction chromatography matrix, a multimodal chromatography matrix including both ion exchange chromatography and hydrophobic interaction chromatography, or a hydroxyapatite matrix.

[12] The method of [11], wherein the affinity chromatography matrix is selected from the group consisting of a protein A matrix, a protein G matrix, a protein L matrix, a sequence-selective peptide matrix, and a matrix that selectively binds to the antigen-binding molecule.

[13] The method of [11], wherein the ion exchange chromatography matrix is a cation exchange ligand or an anion exchange ligand.

[14] The method of [11], wherein the hydrophobic interaction chromatography matrix is a hydrophobic ligand.

[15] The method of [11], wherein the multimodal chromatography matrix is a matrix with a combination of a cation exchange ligand and a hydrophobic ligand, or a matrix with a combination of an anion exchange ligand and a hydrophobic ligand.

[16] The method of [11], wherein the hydroxyapatite matrix is hydroxyapatite or a derivative thereof (such as fluoroapatite).

[17] The method of any one of [1] to [16], wherein the method comprises:

(a) contacting a mixture which comprises:

an antigen-binding molecule having at least one disulfide bond formed between amino acid residues in regions other than a hinge region, and
a mis-disulfide bonded form and/or a non-disulfide bonded form of the antigen-binding molecule

with a chromatography matrix loaded into a column for column chromatography or applied onto a membrane for membrane chromatography.

[18] The method of any one of [1] to [17], wherein the reducing agent is selected from the group consisting of a monothiol, a dithiol, a phosphine, and an inorganic reagent, and a combination of two or more thereof.

[19] The method of any one of [1] to [18], wherein the reducing agent is cysteine or TCEP.

[20] The method of any one of [1] to [18], wherein the reducing agent is a monothiol and the concentration of the reducing agent is about 0.01 mM to about 100 mM.

[21] The method of any one of [1] to [19], wherein the reducing agent is cysteine and the concentration of the reducing agent is about 0.01 mM to about 100 mM, about 0.0001 mM to about 100.0 mM, about 0.001 mM to about 100.0 mM, about 0.005 mM to about 75.0 mM, about 0.01 mM to about 50.0 mM, about 0.05 mM to about 25.0 mM, or about 0.1 mM to about 10 mM.

[22] The method of any one of [1] to [18], wherein the reducing agent is a dithiol and the concentration of the reducing agent is about 0.001 mM to about 10 mM.

[23] The method of any one of [1] to [18], wherein the reducing agent is a phosphine and the concentration of the reducing agent is about 0.0001 mM to about 1 mM or about 0.001 mM to about 0.01 mM.

[24] The method of any one of [1] to [19], wherein the reducing agent is TCEP and the concentration of the reducing agent is about 0.00001 mM to about 10.0 mM, about 0.00005 mM to about 5.0 mM, about 0.0001 mM to about 1 mM, about 0.0005 mM to about 0.5 mM, about 0.001 mM to about 0.1 mM, or about 0.001 mM to about 0.01 mM.

[25] The method of any one of [1] to [18], wherein the reducing agent is an inorganic reagent and the concentration of the reducing agent is about 0.0001 mM to about 10 mM or about 0.001 mM to about 0.1 mM.

[26] The method of any one of [1] to [19], wherein the reducing agent is cysteine and the concentration of the reducing agent is about 0.1 mM, about 0.15 mM, about 1.0 mM, about 10.0 mM, or about 100 mM.

[27] The method of any one of [1] to [19], wherein the reducing agent is TCEP and the concentration of the reducing agent is about 0.001 mM, about 0.01 mM, about 0.1 mM, or about 1.0 mM.

[28] The method of any one of [2] and [4] to [27], wherein the pH of the solution comprising the reducing agent is about 4.5 to about 10.0, about 5.0 to about 9.0, about 6.5 to about 8.5, or about 7.0 to about 8.0.

[29] The method of any one of [2] and [4] to [28], wherein the pH of the solution comprising the reducing agent is about 7.0, about 7.5, or about 8.0.

[30] The method of any one of [1] to [29], wherein the chromatography is column chromatography or membrane chromatography.

[31] The method of [30], wherein the solution comprising the reducing agent is allowed to flow through a column for column chromatography or a device for membrane chromatography with a residence time of about 2 seconds to about 80 minutes or about 3 seconds to about 24 minutes, or the flow is temporarily stopped when the column or device is filled with the reducing agent.

[32] The method of [30] or [31], wherein the solution comprising the reducing agent is allowed to flow through a column for column chromatography or a device for membrane chromatography with a residence time of about 12 minutes or about 30 seconds, or the flow is temporarily stopped when the column or device is filled with the solution comprising the reducing agent.

[33] The method of any one of [2] and [4] to [32], wherein the mixture is contacted with the solution comprising the reducing agent for about 6 seconds to about 1440 minutes, or about 18 seconds to about 300 minutes.

[34] The method of any one of [2] and [4] to [33], wherein the mixture is contacted with the solution comprising the reducing agent for about 120 minutes or about 7.5 minutes.

[35] The method of any one of [1] to [34], wherein the removal of the reducing agent comprises contacting the antigen-binding molecule with a solution that does not contain the reducing agent.

[36] The method of [35], wherein the chromatography is column chromatography or membrane chromatography, and wherein the contacting of the mixture with the solution that does not contain the reducing agent comprises allowing the solution that does not contain the reducing agent to flow through a column for column chromatography or a device for membrane chromatography.

[37] The method of [35] or [36], wherein the solution that does not contain the reducing agent is allowed to flow through a column for column chromatography or a device for membrane chromatography with a residence time of about 2 seconds to about 80 minutes or about 3 seconds to about 24 minutes, and optionally the flow is temporarily stopped when the column or device is filled with the solution.

[38] The method of any one of [35] to [37], wherein the solution that does not contain the reducing agent is allowed to flow through a column for column chromatography or a device for membrane chromatography with a residence time of about 4 minutes or about 30 seconds, and optionally the flow is temporarily stopped when the column or device is filled with the solution.

[39] The method of any one of [35] to [38], wherein the mixture is contacted with the solution that does not contain the reducing agent for about 6 seconds to about 1440 minutes, or about 18 seconds to about 300 minutes.

[40] The method of any one of [35] to [39], wherein the mixture is contacted with the solution that does not contain the reducing agent for about 20 minutes or about 7.5 minutes.

[41] The method of any one of [1] to [40], wherein the mixture is contacted with the solution comprising the reducing agent at about 4°C to about 37°C, preferably at about 15°C to about 37°C.

[42] The method of any one of [1] to [41], wherein the method further comprises, prior to step (a),

contacting, with a chromatography matrix, a mixture which comprises:

an antigen-binding molecule having at least one disulfide bond formed between amino acid residues in regions other than a hinge region and

a mis-disulfide bonded form and/or a non-disulfide bonded form of the antigen-binding molecule

to load the antigen-binding molecule into a column for column chromatography or to immobilize the antigen-binding molecule onto a membrane for membrane chromatography.

[43] The method of any one of [1] to [42], wherein the method further comprises, prior to step (a), removing an impurity in chromatography.

[44] The method of any one of [1] to [43], wherein the method further comprises, prior to or after step (b), recovering the antigen-binding molecule having at least one disulfide bond formed between amino acid residues in regions other than a hinge region.

[45] The method of any one of [1] to [44], wherein the contacting of the antigen-binding molecule with the solution comprising the reducing agent results in cleavage of a disulfide bond that has been formed between amino acid residues that may form a disulfide bond, and/or decapping of a sulfur atom in amino acid residues that may form a disulfide bond.

[46] The method of any one of [2] and [4] to [45], wherein the removal of the reducing agent results in formation of a disulfide bond between amino acid residues that may form a disulfide bond.

[47] The method of any one of [1] to [46], wherein the amino acid residues that may form a disulfide bond are introduced or engineered cysteine residues.

[48] The method of any one of [1] to [47], wherein the at least one disulfide bond in regions other than a hinge region is an interchain disulfide bond.

[49] The method of any one of [1] to [48], wherein the at least one disulfide bond formed between amino acid residues in regions other than a hinge region is one, two, three, four, or more interchain disulfide bonds.

[50] The method of any one of [1] to [49], wherein the at least one disulfide bond formed between amino acid residues in regions other than a hinge region is an engineered disulfide bond that does not exist in wild-type IgG.

[51] The method of any one of [1] to [50], which is for increasing the ratio (LINC ratio) of the antigen-binding molecule having at least one disulfide bond in regions other than a hinge region (LINC form) to the sum of:

(i) the antigen-binding molecule having at least one disulfide bond formed between amino acid residues in regions other than a hinge region (LINC form), and
(ii) a mis-disulfide bonded form and/or a non-disulfide bonded form of the antigen-binding molecule (unLINC forms).

[52] The method of any one of [1] to [51], which is for producing at least 50%, at least 60%, at least 70%, at least 80%, at least 90%, or at least 95%, by molar ratio, of the antigen-binding molecule having at least one disulfide bond in regions other than a hinge region (LINC form) relative to the sum of:

(i) the antigen-binding molecule having at least one disulfide bond formed between amino acid residues in regions other than a hinge region (LINC form) and
(ii) a mis-disulfide bonded form and/or a non-disulfide bonded form of the antigen-binding molecule (unLINC forms).

The present disclosure also relates to the following inventions:

[A1] The method of any one of [1] to [52], wherein the chromatography is column chromatography.
[A2] The method of [A1], wherein the method comprises:

(a) contacting a mixture which comprises:

an antigen-binding molecule having at least one disulfide bond formed between amino acid residues in regions other than a hinge region and
a mis-disulfide bonded form and/or a non-disulfide bonded form of the antigen-binding molecule

with a chromatography matrix loaded into a column.

[A3] The method of [A1] or [A2], wherein the solution comprising the reducing agent is allowed to flow through a column with a residence time of about 2 minutes to about 80 minutes, and optionally the flow is temporarily stopped when the column is filled with the solution comprising the reducing agent.
[A4] The method of any one of [A1] to [A3], wherein the solution comprising the reducing agent is allowed to flow through a column with a residence time of about 4 minutes to about 24 minutes, and optionally the flow is temporarily stopped when the column is filled with the solution comprising the reducing agent.

[A5] The method of any one of [A1] to [A4], wherein the solution comprising the reducing agent is allowed to flow through a column with a residence time of about 12 minutes, and optionally the flow is temporarily stopped when the column is filled with the solution comprising the reducing agent.

[A6] The method of any one of [A1] to [A5], wherein the solution comprising the reducing agent is allowed to flow through a column for column chromatography at a rate of about 25 cm/h to about 500 cm/h, or about 50 cm/h to about 400 cm/h, and optionally the flow is temporarily stopped when the column is filled with the solution comprising the reducing agent.

[A7] The method of any one of [A1] to [A6], wherein the solution comprising the reducing agent is allowed to flow through a column for column chromatography at a rate of about 100 cm/h, and optionally the flow is temporarily stopped when the column is filled with the solution comprising the reducing agent.

[A8] The method of any one of [A1] to [A7], wherein the flow volume of the solution comprising the reducing agent is about 0.1 times the column volume (about 0.1 CV (column volume)) to about 100 times the column volume (about 100 CV).

[A9] The method of any one of [A1] to [A8], wherein the flow volume of the solution comprising the reducing agent is about 1 times the column volume (1 CV) to about 20 times the column volume (about 20 CV).

[A10] The method of any one of [A1] to [A9], wherein the flow volume of the solution comprising the reducing agent is about 10.0 times the column volume (about 10.0 CV) .

[A11] The method of any one of [A1] to [A10], wherein the mixture is contacted with the solution comprising the reducing agent for about 4 minutes to about 1440 minutes.

[A12] The method of any one of [A1] to [A11], wherein the mixture is contacted with the solution comprising the reducing agent for about 8 minutes to about 300 minutes.

[A13] The method of any one of [A1] to [A12], wherein the mixture is contacted with the solution comprising the reducing agent for about 120 minutes.

[A14] The method of any one of [A1] to [A13], wherein the amount of the antigen-binding molecule carried by the matrix is about 5 g to about 80 g, about 7 g to about 50 g, or about 10 g to about 40 g, per 1L of matrix.

[A15] The method of any one of [A1] to [A14], wherein a solution that does not contain the reducing agent is allowed to flow through a column with a residence time of about 2 minutes to about 80 minutes, or the flow is temporarily stopped when the column is filled with the solution.

[A16] The method of any one of [A1] to [A15], wherein a solution that does not contain the reducing agent is allowed to flow through a column with a residence time of about 4 minutes to about 24 minutes, or the flow is temporarily stopped when the column is filled with the solution.

[A17] The method of any one of [A1] to [A16], wherein the solution that does not contain the reducing agent is allowed to flow through a column with a residence time of about 4 minutes, or the flow is temporarily stopped when the column is filled with the solution.

[A18] The method of any one of [A1] to [A17], wherein the solution that does not contain the reducing agent is allowed to flow through a column for column chromatography at a rate of about 25 cm/h to about 500 cm/h, or about 50 cm/h to about 400 cm/h, or the flow is temporarily stopped when the column is filled with the solution.

[A19] The method of any one of [A1] to [A18], wherein the solution that does not contain the reducing agent is allowed to flow through a column for column chromatography at a rate of about 300 cm/h, or the flow is temporarily stopped when the column is filled with the solution.

[A20] The method of any one of [A1] to [A19], wherein the flow volume of the solution that does not contain the reducing agent is about 0.1 times the column volume (about 0.1 CV) to about 100 times the column volume (about 100 CV), or about 1 times the column volume (about 1 CV) to about 20 times the column volume (about 20 CV).

[A21] The method of any one of [A1] to [A20], wherein the flow volume of the solution that does not contain the reducing agent is about 5.0 times the column volume (about 5.0 CV) .

[A22] The method of any one of [A1] to [A21], wherein the mixture is contacted with the solution that does not contain the reducing agent for about 4 minutes to about 1440 minutes.

[A23] The method of any one of [A1] to [A22], wherein the mixture is contacted with the solution that does not contain the reducing agent for about 8 minutes to about 300 minutes.

[A24] The method of any one of [A1] to [A23], wherein the mixture is contacted with the solution that does not contain the reducing agent for about 20 minutes.

The present disclosure also relates to the following inventions:

[B1] The method of any one of [1] to [52], wherein the chromatography is membrane chromatography.
[B2] The method of [B1], wherein the method comprises:

(a) contacting a mixture which comprises:

an antigen-binding molecule having at least one disulfide bond formed between amino acid residues in regions other than a hinge region and
a mis-disulfide bonded form and/or a non-disulfide bonded form of the antigen-binding molecule

with a chromatography matrix applied or immobilized onto a membrane for membrane chromatography.

[B3] The method of [B1] or [B2], wherein the solution comprising the reducing agent is allowed to flow through a membrane device with a residence time of about 2 seconds to about 60 minutes, or the flow is temporarily stopped when the membrane device is filled with the solution comprising the reducing agent.

[B4] The method of any one of [B1] to [B3], wherein the solution comprising the reducing agent is allowed to flow through a membrane device with a residence time of about 3 seconds to about 6 minutes, for example about 30 seconds, or the flow is temporarily stopped when the membrane device is filled with the solution comprising the reducing agent.

[B5] The method of any one of [B1] to [B4], wherein the flow volume of the solution comprising the reducing agent is about 1 times the membrane device volume (about 1 MV (membrane volume)) to about 500 times the membrane device volume (about 500 MV).

[B6] The method of any one of [B1] to [B5], wherein the flow volume of the solution comprising the reducing agent is about 2 times the membrane device volume (about 2 MV) to about 100 times the membrane device volume (about 100 MV), for example about 15 times the membrane device volume (about 15 MV).

[B7] The method of any one of [B1] to [B6], wherein the mixture is contacted with the solution comprising the reducing agent for about 6 seconds to about 600 minutes.

[B8] The method of any one of [B1] to [B7], wherein the mixture is contacted with the solution comprising the reducing agent for about 18 seconds to about 120 minutes, for example about 7.5 minutes.

[B9] The method of any one of [B1] to [B8], wherein the amount of the antigen-binding molecule carried by the matrix is about 5 g to about 100 g or about 7 g to about 70 g, for example about 25 g, per 1L of membrane device volume.

[B10] The method of any one of [B1] to [B9], wherein the solution that does not contain the reducing agent is allowed to flow through a membrane device with a residence time of about 2 seconds to about 60 minutes, or the flow is temporarily stopped when the membrane device is filled with the solution.

[B11] The method of any one of [B1] to [B10], wherein the solution that does not contain the reducing agent is allowed to flow through a membrane device with a residence time of about 3 seconds to about 6 minutes, for example about 30 seconds, or the flow is temporarily stopped when the membrane device is filled with the solution.

[B12] The method of any one of [B1] to [B11], wherein the flow volume of the solution that does not contain the reducing agent is about 1 times the membrane device volume (about 1 MV) to about 500 times the membrane device volume (about 500 MV).

[B13] The method of any one of [B1] to [B12], wherein the flow volume of the solution that does not contain the reducing agent is about 2 times the membrane device volume (about 2 MV) to about 100 times the membrane device volume (about 100 MV), for example about 15 times the membrane device volume (about 15MV).

[B14] The method of any one of [B1] to [B13], wherein the mixture is contacted with the solution that does not contain the reducing agent for about 6 seconds to about 600 minutes.

[B15] The method of any one of [B1] to [B14], wherein the mixture is contacted with the solution that does not contain the reducing agent for about 18 seconds to about 120 minutes, for example about 7.5 minutes.

The present disclosure also relates to the following inventions:

[101] The method of any one of [1] to [52], [A1] to [A24], and [B1] to [B15], wherein the antigen-binding molecule comprises a first antigen-binding domain and a second antigen-binding domain which can be linked with each other via at least one disulfide bond.

[102] The method of [101], wherein the first antigen-binding domain and the second antigen-binding domain each comprise a Fab, Fab', scFab, Fv, scFv, or VHH structure.

[103] The method of [101] or [102], wherein the first antigen-binding domain and the second antigen-binding domain each comprise or do not comprise a hinge region.

[104] The method of any one of [101] to [103], wherein the first antigen-binding domain and the second antigen-binding domain each comprise a Fab and a hinge region that form a F(ab')2 structure.

[105] The method of any one of [101] to [104], wherein both the first antigen-binding domain and the second antigen-binding domain bind to the same antigen.

[106] The method of any one of [101] to [105], wherein both the first antigen-binding domain and the second antigen-binding domain bind to the same epitope on the same antigen.

[107] The method of any one of [101] to [105], wherein each of the first antigen-binding domain and the second antigen-binding domain binds to a different epitope on the same antigen.

[108] The method of any one of [101] to [104], wherein each of the first antigen-binding domain and the second antigen-binding domain binds to a different antigen.

[109] The method of any one of [101] to [106], wherein both the first antigen-binding domain and the second antigen-binding domain have the same amino acid sequence.

[110] The method of any one of [101] to [108], wherein each of the first antigen-binding domain and the second antigen-binding domain has a different amino acid sequence.

[111] The method of any one of [101] to [110], wherein at least one of the first antigen-binding domain and the second antigen-binding domain binds to a soluble protein.

[112] The method of any one of [101] to [111], wherein at least one of the first antigen-binding domain and the second antigen-binding domain binds to a membrane protein.

[113] The method of any one of [1] to [52], [A1] to [A24], [B1] to [B15], and [101] to [112], wherein the antigen-binding molecule has activity of modulating interaction between two antigen molecules.

[114] The method of any one of [101] to [104], wherein the first antigen-binding domain and the second antigen-binding domain bind to a ligand and a receptor thereof, respectively, and wherein the antigen-binding molecule has activity of promoting activation of the receptor mediated by the ligand.

[115] The method of any one of [101] to [104], wherein the first antigen-binding domain and the second antigen-binding domain bind to an enzyme and a substrate thereof, respectively, and wherein the antigen-binding molecule has activity of promoting catalytic reaction between the enzyme and the substrate.

[116] The method of any one of [101] to [104], wherein both the first antigen-binding domain and the second antigen-binding domain bind to a protein present on the surface of a cell (a first antigen and a second antigen, respectively), and wherein the antigen-binding molecule has activity of promoting interaction between the cell expressing the first antigen and the cell expressing the second antigen.

[117] The method of [116], wherein the cell expressing the first antigen is a cell with cytotoxic activity and the cell expressing the second antigen is a target cell thereof, and wherein the antigen-binding molecule promotes damage to the target cell by the cell with cytotoxic activity.

[118] The method of [117], wherein the cell with cytotoxic activity is a T cell, a NK cell, a monocyte, or a macrophage.

[119] The method of any one of [105] to [118], wherein the antigen is selected from the group consisting of a receptor belonging to the cytokine receptor superfamily, a G protein-coupled receptor, an ion channel-type receptor, a tyrosine kinase-type receptor, an immune checkpoint receptor, an antigen receptor, a CD antigen, a costimulatory molecule, and a cell adhesion molecule.

[120] The method of any one of [101] to [104], wherein the first antigen-binding domain and the second antigen-binding domain are each capable of binding to CD3 and/or CD137.

[121] The method of any one of [1] to [52], [A1] to [A24], [B1] to [B15], and [101] to [120], wherein the antigen-binding molecule further comprises a third antigen-binding domain.

[122] The method of any one of [101] to [121], wherein the third antigen-binding domain is fused to either the first antigen-binding domain or the second antigen-binding domain.

[123] The method of [121] or [122], wherein the third antigen-binding domain is a Fab or an scFv.

[124] The method of any one of [102] to [123], wherein the third antigen-binding domain is fused, at its C-terminus, to the N-terminus of the Fab heavy chain (VH region) of either the first antigen-binding domain or the second antigen-binding domain, optionally via a peptide linker.

[125] The method of any one of [121] to [124], wherein the first antigen-binding domain, the second antigen-binding domain, and the third antigen-binding domain are each a Fab molecule, wherein the third antigen-binding domain is fused, at the C-terminus of its Fab heavy chain (CH1 region), to the N-terminus of the Fab heavy chain (VH region) of either the first antigen-binding domain or the second antigen-binding domain, optionally via a peptide linker.

[126] The method of [124] or [125], wherein the peptide linker comprises an amino acid sequence selected from the group consisting of the amino acid sequences of SEQ ID NO: 18, SEQ ID NO: 19, and SEQ ID NO: 20.

[127] The method of any one of [121] to [126], wherein the third antigen-binding domain is a crossover Fab molecule in which the variable regions of the Fab light chain and the Fab heavy chain are exchanged, and wherein the first antigen-binding domain and the second antigen-binding domain are conventional Fab molecules.

[128] The method of any one of [121] to [127], wherein the third antigen-binding domain is capable of binding to an antigen expressed on a cancer cell or cancer tissue.

[129] The method of any one of [121] to [128], wherein the third antigen-binding domain is capable of binding to DLL3, preferably human DLL3.

[130] The method of any one of [1] to [52], [A1] to [A24], [B1] to [B15], and [101] to [129], wherein the antigen-binding molecule further comprises an Fc region.

[131] The method of [130], wherein the Fc region is composed of a first Fc region subunit and a second Fc region subunit which can be stably associated with each other.

[132] The method of [131], wherein the first antigen-binding domain and the second antigen-binding domain are Fabs, wherein the first antigen-binding domain is fused at the C-terminus of the Fab heavy chain to the N-terminus of either one of the first Fc region subunit and the second Fc region subunit of the Fc region, and wherein the second antigen-binding domain is fused at the C-terminus of the Fab heavy chain to the N-terminus of the other subunit of the Fc region.

[133] The method of any one of [130] to [132], wherein the Fc region is derived from a human.

[134] The method of any one of [130] to [133], wherein the Fc region is an IgG Fc region, preferably a human IgG Fc region, or more preferably a human IgG1 Fc region.

[135] The method of any one of [130] to [134], wherein the Fc region shows a reduced binding affinity for a human Fcγ receptor as compared to a native human IgG1 Fc region.

[136] The method of any one of [130] to [135], wherein the Fc region shows an enhanced FcRn-binding activity under acidic pH conditions (e.g., pH 5.8) as compared to a native IgG Fc region.

[137] The method of any one of [130] to [136], wherein the Fc region comprises Ala at position 434; Glu, Arg, Ser, or Lys at position 438; and Glu, Asp, or Gln at position 440, according to EU numbering.

[138] The method of any one of [130] to [137], wherein the Fc region comprises Ala at position 434; Arg or Lys at position 438; and Glu or Asp at position 440, according to EU numbering.

[139] The method of any one of [130] to [138], wherein the Fc region further comprises Ile or Leu at position 428; and/or Ile, Leu, Val, Thr, or Phe at position 436, according to EU numbering.

[140] The method of any one of [130] to [139], wherein the Fc region comprises a combination of amino acid substitutions selected from the group consisting of:

> (a) N434A/Q438R/S440E;
> (b) N434A/Q438R/S440D;
> (c) N434A/Q438K/S440E;
> (d) N434A/Q438K/S440D;
> (e) N434A/Y436T/Q438R/S440E;
> (f) N434A/Y436T/Q438R/S440D;
> (g) N434A/Y436T/Q438K/S440E;
> (h) N434A/Y436T/Q438K/S440D;
> (i) N434A/Y436V/Q438R/S440E;
> (j) N434A/Y436V/Q438R/S440D;
> (k) N434A/Y436V/Q438K/S440E;
> (1) N434A/Y436V/Q438K/S440D;
> (m) N434A/R435H/F436T/Q438R/S440E;
> (n) N434A/R435H/F436T/Q438R/S440D;
> (o) N434A/R435H/F436T/Q438K/S440E;
> (p) N434A/R435H/F436T/Q438K/S440D;
> (q) N434A/R435H/F436V/Q438R/S440E;
> (r) N434A/R435H/F436V/Q438R/S440D;
> (s) N434A/R435H/F436V/Q438K/S440E;
> (t) N434A/R435H/F436V/Q438K/S440D;
> (u) M428L/N434A/Q438R/S440E;
> (v) M428L/N434A/Q438R/S440D;
> (w) M428L/N434A/Q438K/S440E;
> (x) M428L/N434A/Q438K/S440D;
> (y) M428L/N434A/Y436T/Q438R/S440E;
> (z) M428L/N434A/Y436T/Q438R/S440D;
> (aa) M428L/N434A/Y436T/Q438K/S440E;
> (ab) M428L/N434A/Y436T/Q438K/S440D;
> (ac) M428L/N434A/Y436V/Q438R/S440E;
> (ad) M428L/N434A/Y 436V/Q438R/S440D;
> (ae) M428L/N434A/Y436V/Q438K/S440E;
> (af) M428L/N434A/Y436V/Q438K/S440D;
> (ag) L235R/G236R/S239K/M428L/N434A/Y436T/Q438R/S440E; and
> (ah) L235R/G236R/A327G/A330S/P331S/M428L/N434A/Y436T/Q438R/S440E, according to EU numbering.

[141] The method of any one of [130] to [136], wherein the Fc region comprises the combination of amino acid substitutions M428L/N434A/Q438R/S440E.

[142] The method of any one of [130] to [141], wherein the Fc region comprises a combination of one or more amino acid substitutions that promote multimerization of Fc regions.

[143] The method of [142], wherein the amino acid substitutions that promote multimerization comprise an amino acid substitution at at least one position selected from the group consisting of EU numbering positions 247, 248, 253, 254, 310, 311, 338, 345, 356, 359, 382, 385, 386, 430, 433, 434, 436, 437, 438, 439, 440, and 447.

[144] The method of [142] or [143], wherein the multimerization is hexamerization.

[145] The method of any one of [1] to [52], [A1] to [A24], [B1] to [B15], and [101] to [144], wherein the antigen-binding molecule comprises an amino acid residue resulting from substitution of at least one cysteine residue in its hinge region.

[146] The method of [145], wherein the cysteine residue is present at EU numbering position 226 and/or position 229 in the hinge region.

[147] The method of any one of [1] to [52], [A1] to [A24], [B1] to [B15], and [101] to [146], wherein the antigen-binding molecule is a multispecific antigen-binding molecule.

[148] The method of [147], wherein the multispecific antigen-binding molecule is a bispecific antigen-binding molecule or a trispecific antigen-binding molecule.

[149] The method of any one of [1] to [52], [A1] to [A24], [B1] to [B15], and [101] to [148], wherein the antigen-binding molecule is an antibody.

[150] The method of [149], wherein the antibody is an IgG antibody, preferably an IgG1, IgG2, IgG3, or IgG4 antibody.

The present disclosure also relates to the following inventions:

[201] The method of any one of [101] to [150], wherein the at least one disulfide bond in regions other than a hinge region is formed between amino acid residues present at the same position in the first antigen-binding domain and the second antigen-binding domain.

[202] The method of any one of [101] to [150], wherein the at least one disulfide bond in regions other than a hinge region is formed between amino acid residues present at different positions in the first antigen-binding domain and the second antigen-binding domain.

[203] The method of any one of [101] to [150], [201], and [202], wherein the at least one disulfide bond in regions other than a hinge region is formed between a heavy chain of the first antigen-binding domain and a heavy chain of the second antigen-binding domain, between a light chain of the first antigen-binding domain and a light chain of the second antigen-binding domain, or between any combination of a CH1 region, CL region, VL region, VH region, or VHH region of the first antigen-binding domain and a CH1 region, CL region, VL region, VH region, or VHH region of the second antigen-binding domain.

[204] The method of [203], wherein the at least one disulfide bond is formed between a CH1 region of the first antigen-binding domain and a CH1 region of the second antigen-binding domain.

[205] The method of [204], wherein the positions of the amino acid residues in the CH1 regions of the first antigen-binding domain and the second antigen-binding domain are each independently selected from the group consisting of EU numbering positions 119 to 123, 131 to 140, 148 to 150, 155 to 167, 174 to 178, 188 to 197, 201 to 214, and 218 to 219.

[206] The method of [204], wherein the positions of the amino acid residues in the CH1 regions of the first antigen-binding domain and the second antigen-binding domain are each independently selected from the group consisting of EU numbering positions 119, 122, 123, 131, 132, 133, 134, 135, 136, 137, 138, 139, 140, 148, 150, 155, 156, 157, 159, 160, 161, 162, 163, 164, 165, 167, 174, 176, 177, 178, 188, 189, 190, 191, 192, 193, 194, 195, 196, 197, 201, 203, 205, 206, 207, 208, 211, 212, 213, 214, 218, and 219.

[207] The method of [204], wherein the positions of the amino acid residues in the CH1 regions of the first antigen-binding domain and the second antigen-binding domain are each independently selected from the group consisting of EU numbering positions 134, 135, 136, 137, 191, 192, 193, 194, 195, and 196.

[208] The method of [204], wherein the positions of the amino acid residues in the CH1 regions of the first antigen-binding domain and the second antigen-binding domain are each independently selected from the group consisting of EU numbering positions 135, 136, and 191.

[209] The method of [204], wherein the positions of the amino acid residues in the CH1 regions of the first antigen-binding domain and the second antigen-binding domain are each independently selected from the group consisting of EU numbering positions 119, 120, 121, 122, and 123.

[210] The method of [204], wherein the positions of the amino acid residues in the CH1 regions of the first antigen-binding domain and the second antigen-binding domain are each independently selected from the group consisting of EU numbering positions 131, 132, 133, 134, 135, 136, 137, 138, 139, and 140.

[211] The method of [204], wherein the positions of the amino acid residues in the CH1 regions of the first antigen-binding domain and the second antigen-binding domain are each independently selected from the group consisting of EU numbering positions 148, 149, and 150.

[212] The method of [204], wherein the positions of the amino acid residues in the CH1 regions of the first antigen-binding domain and the second antigen-binding domain are each independently selected from the group consisting of EU numbering positions 155, 156, 157, 158, 159, 160, 161, 162, 163, 164, 165, 166, and 167.

[213] The method of [204], wherein the positions of the amino acid residues in the CH1 regions of the first antigen-binding domain and the second antigen-binding domain are each independently selected from the group consisting of EU numbering positions 174, 175, 176, 177, and 178.

[214] The method of [204], wherein the positions of the amino acid residues in the CH1 regions of the first antigen-binding domain and the second antigen-binding domain are each independently selected from the group consisting of EU numbering positions 188, 189, 190, 191, 192, 193, 194, 195, 196, and 197.

[215] The method of [204], wherein the positions of the amino acid residues in the CH1 regions of the first antigen-binding domain and the second antigen-binding domain are each independently selected from the group consisting of EU numbering positions 201, 202, 203, 204, 205, 206, 207, 208, 209, 210, 211, 212, 213, and 214.

[216] The method of [204], wherein the positions of the amino acid residues in the CH1 regions of the first antigen-binding domain and the second antigen-binding domain are each independently selected from the group consisting of EU numbering positions 218 and 219.

[217] The method of any one of [204] to [216], wherein the positions of the amino acid residues in the CH1 regions of the first antigen-binding domain and the second antigen-binding domain differ by 3 amino acids or less.

[218] The method of [204], wherein the amino acid residue in the CH1 region of the first antigen-binding domain is an amino acid residue at EU numbering position 135, and the amino acid residue in the CH1 region of the second antigen-binding domain is an amino acid residue at any one of EU numbering positions 132 to 138.

[219] The method of [204], wherein the amino acid residue in the CH1 region of the first antigen-binding domain is an amino acid residue at EU numbering position 136, and the amino acid residue in the CH1 region of the second antigen-binding domain is an amino acid residue at any one of EU numbering positions 133 to 139.

[220] The method of [204], wherein the amino acid residue in the CH1 region of the first antigen-binding domain is an amino acid residue at EU numbering position 191, and the amino acid residue in the CH1 region of the second antigen-binding domain is an amino acid residue at any one of EU numbering positions 188 to 194.

[221] The method of [204], wherein the amino acid residue in the CH1 region of the first antigen-binding domain is an amino acid residue at EU numbering position 135, and the amino acid residue in the CH1 region of the second antigen-binding domain is an amino acid residue at EU numbering position 135.

[222] The method of [204], wherein the amino acid residue in the CH1 region of the first antigen-binding domain is an amino acid residue at EU numbering position 136, and the amino acid residue in the CH1 region of the second antigen-binding domain is an amino acid residue at EU numbering position 136.

[223] The method of [204], wherein the amino acid residue in the CH1 region of the first antigen-binding domain is an amino acid residue at EU numbering position 191, and the amino acid residue in the CH1 region of the second antigen-binding domain is an amino acid residue at EU numbering position 191.

[224] The method of any one of [204] to [223], wherein the subclass of the CH1 region is $\gamma$1, $\gamma$2, y3, y4, $\alpha$1, $\alpha$2, $\mu$, $\delta$, or $\varepsilon$.

[225] The method of any one of [101] to [150] and [201] to [224], wherein the antigen-binding molecule comprises one, two, or more additional disulfide bonds between the first antigen-binding domain and the second antigen-binding domain, wherein the additional disulfide bonds are formed via amino acid residues at the following positions according to EU numbering in each CH1 region of the first antigen-binding domain and the second antigen-binding domain:

> (a) between amino acid residues at any one of positions 131 to 138, 194, and 195 in each of the two antigen-binding domains;
> (b) between amino acid residues at position 131 in each of the two antigen-binding domains, and between amino acid residues at position 194 in each of the two antigen-binding domains;
> (c) between amino acid residues at position 132 in each of the two antigen-binding domains, and between amino acid residues at position 194 in each of the two antigen-binding domains;
> (d) between amino acid residues at position 133 in each of the two antigen-binding domains, and between amino acid residues at position 194 in each of the two antigen-binding domains;
> (e) between amino acid residues at position 134 in each of the two antigen-binding domains, and between amino acid residues at position 194 in each of the two antigen-binding domains;
> (f) between amino acid residues at position 135 in each of the two antigen-binding domains, and between amino acid residues at position 194 in each of the two antigen-binding domains;
> (g) between amino acid residues at position 136 in each of the two antigen-binding domains, and between amino acid residues at position 194 in each of the two antigen-binding domains;

(h) between amino acid residues at position 137 in each of the two antigen-binding domains, and between amino acid residues at position 194 in each of the two antigen-binding domains;

(i) between amino acid residues at position 138 in each of the two antigen-binding domains, and between amino acid residues at position 194 in each of the two antigen-binding domains;

(j) between amino acid residues at position 131 in each of the two antigen-binding domains, and between amino acid residues at position 195 in each of the two antigen-binding domains;

(k) between amino acid residues at position 132 in each of the two antigen-binding domains, and between amino acid residues at position 195 in each of the two antigen-binding domains;

(l) between amino acid residues at position 133 in each of the two antigen-binding domains, and between amino acid residues at position 195 in each of the two antigen-binding domains;

(m) between amino acid residues at position 134 in each of the two antigen-binding domains, and between amino acid residues at position 195 in each of the two antigen-binding domains;

(n) between amino acid residues at position 135 in each of the two antigen-binding domains, and between amino acid residues at position 195 in each of the two antigen-binding domains;

(o) between amino acid residues at position 136 in each of the two antigen-binding domains, and between amino acid residues at position 195 in each of the two antigen-binding domains;

(p) between amino acid residues at position 137 in each of the two antigen-binding domains, and between amino acid residues at position 195 in each of the two antigen-binding domains; and

(q) between amino acid residues at position 138 in each of the two antigen-binding domains, and between amino acid residues at position 195 in each of the two antigen-binding domains.

[226] The method of any one of [101] to [150] and [201] to [224], wherein either one of the first antigen-binding domain and the second antigen-binding domain comprises one, two, or more charged amino acid residues at EU numbering positions 136-138 in the CH1 region; and the other of the first antigen-binding domain and the second antigen-binding domain comprises one, two, or more oppositely charged amino acid residues at EU numbering positions 193-195 in the CH1 region.

[227] The method of any one of [101] to [150] and [201] to [224], wherein either one of the first antigen-binding domain and the second antigen-binding domain comprises one, two, or more positively charged amino acid residues at EU numbering positions 136-138 in the CH1 region; and the other of the first antigen-binding domain and the second antigen-binding domain comprises one, two, or more negatively charged amino acid residues at EU numbering positions 193-195 in the CH1 region.

[228] The method of any one of [101] to [150] and [201] to [224], wherein either one of the first antigen-binding domain and the second antigen-binding domain comprises one, two, or more negatively charged amino acid residues at EU numbering positions 136-138 in the CH1 region; and the other of the first antigen-binding domain and the second antigen-binding domain comprises one, two, or more positively charged amino acid residues at EU numbering positions 193-195 in the CH1 region.

[229] The method of any one of [101] to [150] and [201] to [224], wherein either one of the first antigen-binding domain and the second antigen-binding domain comprises one, two, or more of the following amino acid residues in the CH1 region:

(a) the amino acid residue at EU numbering position 136 is glutamic acid (E) or aspartic acid (D);
(b) the amino acid residue at EU numbering position 137 is glutamic acid (E) or aspartic acid (D);and
(c) the amino acid residue at EU numbering position 138 is glutamic acid (E) or aspartic acid (D);

and wherein the other of the first antigen-binding domain and the second antigen-binding domain comprises one, two, or more of the following amino acid residues in the CH1 region:

(d) the amino acid residue at EU numbering position 193 is lysine (K), arginine (R), or histidine (H);
(e) the amino acid residue at EU numbering position 194 is lysine (K), arginine (R), or histidine (H); and
(f) the amino acid residue at EU numbering position 195 is lysine (K), arginine (R), or histidine (H).

[230] The method of any one of [101] to [150] and [201] to [224], wherein either one of the first antigen-binding domain and the second antigen-binding domain comprises one, two, or more of the following amino acid residues in the CH1 region:

(a) the amino acid residue at EU numbering position 136 is lysine (K), arginine (R), or histidine (H);
(b) the amino acid residue at EU numbering position 137 is lysine (K), arginine (R), or histidine (H);and
(c) the amino acid residue at EU numbering position 138 is lysine (K), arginine (R), or histidine (H);

and wherein the other of the first antigen-binding domain and the second antigen-binding domain comprises one, two, or more of the following amino acid residues in the CH1 region:

(d) the amino acid residue at EU numbering position 193 is glutamic acid (E) or aspartic acid (D);
(e) the amino acid residue at EU numbering position 194 is glutamic acid (E) or aspartic acid (D); and
(f) the amino acid residue at EU numbering position 195 is glutamic acid (E) or aspartic acid (D).

[231] The method of any one of [101] to [150] and [201] to [224], wherein each of the first antigen-binding domain and the second antigen-binding domain comprises any of the combinations of specific charged amino acids in the CH1 region (according to EU numbering) as set forth in Table 1, 2, or 3.

[232] The method of any one of [101] to [150] and [201] to [224], wherein either one of the first antigen-binding domain and the second antigen-binding domain comprises one, two, or more hydrophobic amino acid residues at EU numbering positions 136-138 in the CH1 region; and the other of the first antigen-binding domain and the second antigen-binding domain comprises one, two, or more hydrophobic amino acid residues at EU numbering positions 193-195 in the CH1 region.

[233] The method of [232], wherein the hydrophobic amino acid residue(s) is(are) alanine (Ala), valine (Val), leucine (Leu), isoleucine (Ile), phenylalanine (Phe), and/or tryptophan (Trp).

[234] The method of any one of [101] to [150] and [201] to [224], wherein each of the first antigen-binding domain and the second antigen-binding domain comprises any of the combinations of specific hydrophobic amino acids in the CH1 region (according to EU numbering) as set forth in Table 4.

[235] The method of any one of [101] to [150] and [201] to [224], wherein either one of the first antigen-binding domain and the second antigen-binding domain comprises one "knob" amino acid residue at EU numbering positions 136-138 in the CH1 region; and the other of the first antigen-binding domain and the second antigen-binding domain comprises one, two, or more "hole" amino acid residues at EU numbering positions 193-195 in the CH1 region.

[236] The method of any one of [101] to [150] and [201] to [224], wherein either one of the first antigen-binding domain and the second antigen-binding domain comprises one, two, or more "hole" amino acid residues at EU numbering positions 136-138 in the CH1 region; and the other of the first antigen-binding domain and the second antigen-binding domain comprises one "knob" amino acid residue at EU numbering positions 193-195 in the CH1 region.

[237] The method of [236], wherein the "knob" amino acid residue is selected from the group consisting of tryptophan (Trp) and phenylalanine (Phe); and the "hole" amino acid residue(s) is(are) selected from the group consisting of alanine (Ala), valine (Val), threonine (Thr), and serine (Ser).

[238] The method of any one of [101] to [150] and [201] to [224], wherein either one of the first antigen-binding domain and the second antigen-binding domain comprises one, two, or more aromatic amino acid residues at EU numbering positions 136-138 in the CH1 region; and the other of the first antigen-binding domain and the second antigen-binding domain comprises one, two, or more positively charged amino acid residues at EU numbering positions 193-195 in the CH1 region.

[239] The method of any one of [101] to [150] and [201] to [224], wherein either one of the first antigen-binding domain and the second antigen-binding domain comprises one, two, or more positively charged amino acid residues at EU numbering positions 136-138 in the CH1 region; and the other of the first antigen-binding domain and the second antigen-binding domain comprises one, two, or more aromatic amino acid residues at EU numbering positions 193-195 in the CH1 region.

[240] The method of any one of [101] to [150] and [201] to [224], wherein the aromatic amino acid residue(s) is(are) selected from the group consisting of tryptophan (Trp), tyrosine (Tyr), histidine (His), and phenylalanine (Phe); and the positively charged amino acid residue(s) is(are) selected from the group consisting of lysine (Lys), arginine (Arg), and histidine (His).

[241] The method of [203], wherein the at least one disulfide bond in regions other than a hinge region is formed between a CL region of the first antigen-binding domain and a CL region of the second antigen-binding domain.

[242] The method of [241], wherein the positions of the amino acid residues in the CL regions of the first antigen-binding domain and the second antigen-binding domain are each independently selected from the group consisting of Kabat numbering positions 108 to 112, 121 to 128, 151 to 156, 184 to 190, 195 to 196, 200 to 203, and 208 to 213.

[243] The method of [241], wherein the positions of the amino acid residues in the CL regions of the first antigen-binding domain and the second antigen-binding domain are each independently selected from the group consisting of Kabat numbering positions 108, 109, 112, 121, 123, 126, 128, 151, 152, 153, 156, 184, 186, 188, 189, 190, 195, 196, 200, 201, 202, 203, 208, 210, 211, 212, and 213.

[244] The method of [241], wherein the positions of the amino acid residues in the CL regions of the first antigen-binding domain and the second antigen-binding domain are each independently selected from the group consisting of Kabat numbering positions 108, 109, 110, 111, and 112.

[245] The method of [241], wherein the positions of the amino acid residues in the CL regions of the first antigen-binding domain and the second antigen-binding domain are each independently selected from the group consisting of Kabat numbering positions 121, 122, 123, 124, 125, 126, 127, and 128.

[246] The method of [241], wherein the positions of the amino acid residues in the CL regions of the first antigen-binding domain and the second antigen-binding domain are each independently selected from the group consisting of Kabat numbering positions 151, 152, 153, 154, 155, and 156.

[247] The method of [241], wherein the positions of the amino acid residues in the CL regions of the first antigen-binding domain and the second antigen-binding domain are each independently selected from the group consisting of Kabat numbering positions 184, 185, 186, 187, 188, 189, and 190.

[248] The method of [241], wherein the positions of the amino acid residues in the CL regions of the first antigen-binding domain and the second antigen-binding domain are each independently selected from the group consisting of Kabat numbering positions 195 and 196.

[249] The method of [241], wherein the positions of the amino acid residues in the CL regions of the first antigen-binding domain and the second antigen-binding domain are each independently selected from the group consisting of Kabat numbering positions 200, 201, 202, and 203.

[250] The method of [241], wherein the positions of the amino acid residues in the CL regions of the first antigen-binding domain and the second antigen-binding domain are each independently selected from the group consisting of Kabat numbering positions 208, 209, 210, 211, 212, and 213.

[251] The method of any one of [241] to [250], wherein the positions of the amino acid residues in the CL regions of the first antigen-binding domain and the second antigen-binding domain differ by 3 amino acids or less.

[252] The method of [241], wherein the amino acid residue in the CL region of the first antigen-binding domain is an amino acid residue at Kabat numbering position 126, and the amino acid residue in the CL region of the second antigen-binding domain is an amino acid residue at Kabat numbering position 126.

[253] The method of [203], wherein the at least one disulfide bond in regions other than a hinge region is formed between an amino acid residue in a CH1 region of the first antigen-binding domain and an amino acid residue in a CL region of the second antigen-binding domain.

[254] The method of [253], wherein the position of the amino acid residue in the CH1 region is selected from the group consisting of EU numbering positions 188, 189, 190, 191, 192, 193, 194, 195, 196, and 197, and the position of the amino acid residue in the CL region is selected from the group consisting of Kabat numbering positions 121, 122, 123, 124, 125, 126, 127, and 128.

[255] The method of [253], wherein the amino acid residue in the CH1 region is an amino acid residue at EU numbering position 191, and the amino acid residue in the CL region is an amino acid residue at Kabat numbering position 126.

[256] The method of any one of [101] to [150] and [201] to [224], wherein each of the first antigen-binding domain and the second antigen-binding domain comprises lysine (K), arginine (R), or histidine (H) independently at Kabat numbering positions 123 and/or 124 in the CL region, and comprises glutamic acid (E) or aspartic acid (D) independently at EU numbering positions 147 and/or 213 in the CH1 region.

[257] The method of [255], wherein each of the first antigen-binding domain and the second antigen-binding domain comprises arginine (R) and lysine (K) at Kabat numbering positions 123 and 124 in the CL region, respectively, and comprises glutamic acid (E) at EU numbering positions 147 and 213 in the CH1 region.

[258] The method of any one of [241] to [257], wherein the subclass of the CL region is κ or λ.

[259] The method of [203], wherein the at least one disulfide bond in regions other than a hinge region is formed between an amino acid residue in a VH region of the first antigen-binding domain and an amino acid residue in a VH region of the second antigen-binding domain.

[260] The method of [259], wherein the positions of the amino acid residues in the VH regions of the first antigen-binding domain and the second antigen-binding domain are each independently selected from the group consisting of EU numbering positions 6, 8, 16, 20, 25, 26, 28, 74, and 82b.

[261] The method of [203], wherein the at least one disulfide bond in regions other than a hinge region is formed between an amino acid residue in a VL region of the first antigen-binding domain and an amino acid residue in a VL region of the second antigen-binding domain.

[262] The method of [261], wherein the positions of the amino acid residues in the VL regions (subclass κ) of the first antigen-binding domain and the second antigen-binding domain are each independently selected from the group consisting of Kabat numbering positions 21, 27, 58, 77, 100, 105, and 107.

[263] The method of [261], wherein the positions of the amino acid residues in the VL regions (subclass λ) of the first antigen-binding domain and the second antigen-binding domain are each independently selected from the group consisting of Kabat numbering positions 6, 19, 33, and 34.

[264] The method of [203], wherein the at least one disulfide bond in regions other than a hinge region is formed between an amino acid residue in a VHH region of the first antigen-binding domain and an amino acid residue in a VHH region of the second antigen-binding domain.

[265] The method of [264], wherein the positions of the amino acid residues in the VHH regions of the first antigen-binding domain and the second antigen-binding domain are each independently selected from the group consisting of Kabat numbering positions 4, 6, 7, 8, 9, 10, 11, 12, 14, 15, 17, 20, 24, 27, 29, 38, 39, 40, 41, 43, 44, 45, 46, 47, 48, 49, 67, 69, 71, 78, 80, 82, 82c, 85, 88, 91, 93, 94, and 107.

The present disclosure also relates to the following inventions:

[301] The method of any one of [1] to [52], [A1] to [A24], [B1] to [B15], [101] to [150], and [201] to [265], wherein the antigen-binding molecule comprises:

a first antigen-binding domain and a second antigen-binding domain that are capable of binding to CD3 and CD137 but do not bind to CD3 and CD137 simultaneously, and
a third antigen-binding domain that is capable of binding to DLL3, preferably human DLL3.

[302] The method of [301], wherein the first antigen-binding domain and the second antigen-binding domain each comprise an antibody variable region that may be identical to or different from each other, and comprise an antibody variable region independently selected from the group consisting of (a1) to (a4) below:

(a1) an antibody variable region comprising:

a heavy chain variable region comprising:

heavy chain complementarity determining region (CDR) 1 comprising the amino acid sequence of SEQ ID NO: 27,
heavy chain CDR 2 comprising the amino acid sequence of SEQ ID NO: 28, and
heavy chain CDR 3 comprising the amino acid sequence of SEQ ID NO: 29; and

a light chain variable region comprising:

light chain CDR 1 comprising the amino acid sequence of SEQ ID NO: 30,
light chain CDR 2 comprising the amino acid sequence of SEQ ID NO: 31, and
light chain CDR 3 comprising the amino acid sequence of SEQ ID NO: 32;

(a2) an antibody variable region comprising:

a heavy chain variable region comprising:

heavy chain complementarity determining region (CDR) 1 comprising the amino acid sequence of SEQ ID NO: 33,
heavy chain CDR 2 comprising the amino acid sequence of SEQ ID NO: 34, and
heavy chain CDR 3 comprising the amino acid sequence of SEQ ID NO: 35; and

a light chain variable region comprising:

light chain CDR 1 comprising the amino acid sequence of SEQ ID NO: 30,
light chain CDR 2 comprising the amino acid sequence of SEQ ID NO: 31, and
light chain CDR 3 comprising the amino acid sequence of SEQ ID NO: 32;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

[303] The method of [301] or [302], wherein the first antigen-binding domain and the second antigen-binding domain each comprise an antibody variable region that may be identical to or different from each other, and comprise an antibody variable region independently selected from the group consisting of (a1) to (a4) below:

(a1) an antibody variable region comprising:

a heavy chain variable region comprising the amino acid sequence of SEQ ID NO: 36, and
a light chain variable region comprising the amino acid sequence of SEQ ID NO: 37; and

(a2) an antibody variable region comprising:

a heavy chain variable region comprising the amino acid sequence of SEQ ID NO: 38, and
a light chain variable region comprising the amino acid sequence of SEQ ID NO: 37;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

[304] The method of any one of [301] to [303], wherein the third antigen-binding domain comprises an antibody variable region independently selected from the group consisting of any one of (a1) to (a4) below:

(a1) an antibody variable region comprising:

a heavy chain variable region comprising:

heavy chain complementarity determining region (CDR) 1 comprising the amino acid sequence of SEQ ID NO: 46,
heavy chain CDR 2 comprising the amino acid sequence of SEQ ID NO: 47, and
heavy chain CDR 3 comprising the amino acid sequence of SEQ ID NO: 48; and

a light chain variable region comprising:

light chain CDR 1 comprising the amino acid sequence of SEQ ID NO: 49,
light chain CDR 2 comprising the amino acid sequence of SEQ ID NO: 50, and
light chain CDR 3 comprising the amino acid sequence of SEQ ID NO: 51;

(a2) an antibody variable region comprising:

a heavy chain variable region comprising the amino acid sequence of SEQ ID NO: 52, and
a light chain variable region comprising the amino acid sequence of SEQ ID NO: 53;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

[305] The method of any one of [301] to [304], wherein the first antigen-binding domain and the second antigen-binding domain each comprise an antibody variable region comprising:

a heavy chain variable region comprising:

heavy chain complementarity determining region (CDR) 1 comprising the amino acid sequence of SEQ ID NO: 27,
heavy chain CDR 2 comprising the amino acid sequence of SEQ ID NO: 28, and
heavy chain CDR 3 comprising the amino acid sequence of SEQ ID NO: 29; and

a light chain variable region comprising:

light chain CDR 1 comprising the amino acid sequence of SEQ ID NO: 30,
light chain CDR 2 comprising the amino acid sequence of SEQ ID NO: 31, and
light chain CDR 3 comprising the amino acid sequence of SEQ ID NO: 32.

[306] The method of any one of [301] to [305], wherein the first antigen-binding domain and the second antigen-binding domain each comprise an antibody variable region comprising:

a heavy chain variable region comprising the amino acid sequence of SEQ ID NO: 36, and
a light chain variable region comprising the amino acid sequence of SEQ ID NO: 37.

[307] The method of any one of [301] to [306], wherein the third antigen-binding domain comprises an antibody variable region comprising:

a heavy chain variable region comprising:

heavy chain complementarity determining region (CDR) 1 comprising the amino acid sequence of SEQ ID NO: 46,
heavy chain CDR 2 comprising the amino acid sequence of SEQ ID NO: 47, and
heavy chain CDR 3 comprising the amino acid sequence of SEQ ID NO: 48; and

a light chain variable region comprising:

light chain CDR 1 comprising the amino acid sequence of SEQ ID NO: 49,
light chain CDR 2 comprising the amino acid sequence of SEQ ID NO: 50, and
light chain CDR 3 comprising the amino acid sequence of SEQ ID NO: 51.

[308] The method of any one of [301] to [307], wherein the third antigen-binding domain comprises an antibody variable region comprising:

a heavy chain variable region comprising the amino acid sequence of SEQ ID NO: 52, and
a light chain variable region comprising the amino acid sequence of SEQ ID NO: 53.

[309] The method of any one of [301] to [308], wherein the first antigen-binding domain and the second antigen-binding domain each comprise an antibody variable region comprising:

a heavy chain variable region comprising the amino acid sequence of SEQ ID NO: 36, and
a light chain variable region comprising the amino acid sequence of SEQ ID NO: 37, and wherein the third antigen-binding domain comprises an antibody variable region comprising:

a heavy chain variable region comprising SEQ ID NO: 52, and
a light chain variable region comprising SEQ ID NO: 53.

[310] The method of any one of [301] to [309], wherein the first antigen-binding domain and the second antigen-binding domain are each a Fab having a cysteine residue at EU numbering position 191 in the heavy chain, and have a disulfide bond formed by two cysteine residues.
[311] The method of any one of [301] to [310], wherein each of the first, second and third antigen-binding domains is a Fab which comprises:

a heavy chain comprising a VH region and a CH1 region, and
a light chain comprising a VL region and a CL region,

wherein the C-terminus of the CH1 region of the heavy chain of the third antigen-binding domain is fused to the N-terminus of the VH region of the Fab heavy chain of either the first antigen-binding domain or the second antigen-binding domain directly or via a peptide linker.
[312] The method of [311], wherein the peptide linker comprises an amino acid sequence selected from the group consisting of the amino acid sequences of SEQ ID NO: 18, SEQ ID NO: 19, and SEQ ID NO: 20.
[313] The method of any one of [301] to [312], wherein the third antigen-binding domain is a crossover Fab in which an VH region is linked to a CL region and a VL region is linked to a CH1 region, and wherein each of the first and second antigen-binding domains is a conventional Fab in which a VH region is linked to a CH1 region and a VL region is linked to a CL region.
[314] The method of any one of [301] to [313], wherein:

in the CL region of each of the first antigen-binding domain and the second antigen-binding domain, the amino acid residues at Kabat numbering positions 123 and 124 are arginine and lysine, respectively, and
in the CH1 region of each of the first and second antigen-binding domains, the amino acid residues at EU
numbering positions 147 and 213 are glutamic acid.

[315] The method of any one of [301] to [314], wherein the antigen-binding molecule further comprises an Fc region.
[316] The method of [315], wherein the Fc region comprises a first Fc region subunit and a second Fc region subunit,

wherein the first Fc region subunit is selected from the group consisting of:

an Fc region polypeptide comprising alanine at each of positions 234 and 235;
an Fc region polypeptide comprising alanine at each of positions 234, 235, and 297; and
an Fc region polypeptide comprising alanine at each of positions 234, 235, and 297, cysteine at position 354,
and tryptophan at position 366,and

the second Fc region subunit is selected from the group consisting of:

an Fc region polypeptide comprising alanine at each of positions 234 and 235;
an Fc region polypeptide comprising alanine at each of positions 234, 235, and 297; and
an Fc region polypeptide comprising alanine at each of positions 234, 235, and 297, cysteine at position 349,
and serine at position 366, alanine at position 368, and valine at position 407,

wherein all positions are according to EU numbering.

[317] The method of any one of [316] or [317], wherein the Fc region comprises any one of the following:

(a) a first Fc region subunit comprising the amino acid sequence of SEQ ID NO: 23, and a second Fc region
subunit comprising the amino acid sequence of SEQ ID NO: 24;
(b) a first Fc region subunit comprising the amino acid sequence of SEQ ID NO: 25, and a second Fc region
subunit comprising the amino acid sequence of SEQ ID NO: 26; or
(c) a first Fc region subunit comprising the amino acid sequence of SEQ ID NO: 58, and a second Fc region subunit
comprising the amino acid sequence of SEQ ID NO: 59.

[318] The method of any one of [301] to [317], wherein the antigen-binding molecule comprises any one combination
of five polypeptide chains selected from the group consisting of:

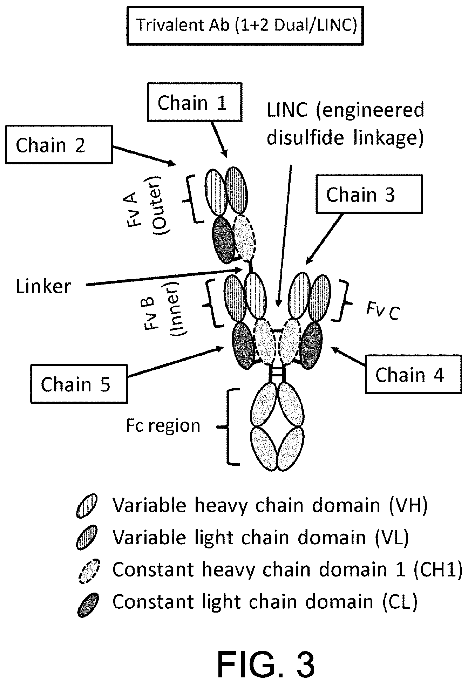

(a1) a polypeptide chain comprising the amino acid sequence of SEQ ID NO: 39 (chain 1), a polypeptide chain
comprising the amino acid sequence of SEQ ID NO: 40 (chain 2), a polypeptide chain comprising the amino acid
sequence of SEQ ID NO: 41 (chain 3), and two polypeptide chains each comprising the amino acid sequence of
SEQ ID NO: 42 (chain 4 and chain 5);
(a2) a polypeptide chain comprising the amino acid sequence of SEQ ID NO: 43 (chain 1), a polypeptide chain
comprising the amino acid sequence of SEQ ID NO: 40 (chain 2), a polypeptide chain comprising the amino acid
sequence of SEQ ID NO: 44 (chain 3), and two polypeptide chains each comprising the amino acid sequence of
SEQ ID NO: 42 (chain 4 and chain 5); and
(a3) a polypeptide chain comprising the amino acid sequence of SEQ ID NO: 45 (chain 1), a polypeptide chain
comprising the amino acid sequence of SEQ ID NO: 40 (chain 2), a polypeptide chain comprising the amino acid
sequence of SEQ ID NO: 44 (chain 3), and two polypeptide chains each comprising the amino acid sequence of
SEQ ID NO: 42 (chain 4 and chain 5);
wherein, preferably, the five polypeptide chains (chains 1 to 5) are connected and/or associated with each other
according to the orientation shown in Fig. 3.

[319] The method of [301], wherein the antigen-binding molecule comprises the following five polypeptide chains:

a polypeptide chain comprising the amino acid sequence of SEQ ID NO: 54 (chain 1), a polypeptide chain
comprising the amino acid sequence of SEQ ID NO: 55 (chain 2), a polypeptide chain comprising the amino acid
sequence of SEQ ID NO: 56 (chain 3), and two polypeptide chains each comprising the amino acid sequence of
SEQ ID NO: 57 (chain 4 and chain 5);
wherein, preferably, the five polypeptide chains (chains 1 to 5) are connected and/or associated with each other
according to the orientation shown in Fig. 3.

[320] The method of [301], wherein the antigen-binding molecule comprises the following five polypeptide chains:

a polypeptide chain comprising the amino acid sequence of SEQ ID NO: 54 (chain 1),
a polypeptide chain comprising the amino acid sequence of SEQ ID NO: 55 (chain 2),
a polypeptide chain comprising the amino acid sequence of SEQ ID NO: 60 (chain 3), and
two polypeptide chains each comprising the amino acid sequence of SEQ ID NO: 57 (chain 4 and chain 5);

wherein, preferably, the five polypeptide chains (chains 1 to 5) are connected and/or associated with each other according to the orientation shown in Fig. 3.

The present disclosure also relates to the following inventions:

[401] The method of any one of [1] to [52], [A1] to [A24], [B1] to [B15], [101] to [150], [201] to [265], and [301] to [320], which further comprises quantifying the ratio (LINC ratio) of the antigen-binding molecule having at least one disulfide bond in regions other than a hinge region (LINC form) to the sum of:

(i) the antigen-binding molecule having at least one disulfide bond formed between amino acid residues in regions other than a hinge region (LINC form), and
(ii) a mis-disulfide bonded form and/or a non-disulfide bonded form of the antigen-binding molecule (unLINC forms)

in the preparation.
[402] The method of [401], wherein the step of quantifying comprises electrophoresis and chromatography.
[403] The method of [402], wherein the electrophoresis is selected from the group consisting of non-reducing SDS-polyacrylamide gel electrophoresis (SDS-PAGE) and non-reducing capillary SDS gel electrophoresis (CE-SDS).
[404] The method of [402], wherein the chromatography is hydrophobic interaction chromatography (HIC).
[405] The method of any one of [401] to [404], wherein the method further comprises, prior to the quantifying step, adding a protease to the preparation.
[406] The method of [405], wherein the protease digests the antigen-binding molecule between T and H in the amino acid sequence KSCDKT/HTCPPCP of the antigen-binding molecule.
[407] The method of [406], wherein the antigen-binding molecule is a human IgG1 antibody.
[408] The method of [406] or [407], wherein the protease is IdgE.
[409] The method of [408], wherein the IdgE is derived from *Streptococcus agalactiae.*
[410] The method of any one of [405] to [409], wherein the step of quantifying comprises non-reducing capillary SDS gel electrophoresis (CE-SDS) or hydrophobic interaction chromatography (HIC).
[411] The method of [410], wherein the step of quantifying comprises non-reducing capillary SDS gel electrophoresis (CE-SDS), and wherein the method further comprises performing non-reducing capillary SDS gel electrophoresis (CE-SDS) for a preparation to which the protease has not been added and a sample that contains the protease only.
[412] The method of [411], which further comprises preparing an electropherogram for a preparation to which the protease has been added, a preparation to which the protease has not been added, and a sample that contains the protease only.
[413] The method of [412], wherein the LINC ratio is determined by the ratio of (a peak from the LINC form) to (peaks from the preparation to which the protease has been added, including peaks from the unLINC forms and peaks from the protease) - (peaks from the preparation to which the protease has not been added which do not include peaks from the unLINK forms) - (peaks from the sample that contains the protease only) + (the peak from the LINC form).
[414] The method of [410], wherein the step of quantifying comprises hydrophobic interaction chromatography, wherein the LINC ratio is determined by the ratio of a peak from the LINC form to the sum of the peak from the LINC form and peaks from all unLINC forms.
[415] The method of any one of [405] to [414], wherein the chromatography in [1] to [52], [B1] to [B15], [101] to [150], [201] to [265], and [301] to [320] is membrane chromatography.
[416] The method of any one of [405] to [415], wherein the method further comprises, after adding the protease, culturing the preparation.

The present disclosure also relates to the following inventions:

[501] A preparation comprising an antigen-binding molecule having at least one disulfide bond formed between amino acid residues in regions other than a hinge region, produced by the method of any one of [1] to [52], [A1] to [A24], [B1] to [B15], [101] to [150], [201] to [265], [301] to [320], and [401] to [416].

[502] A pharmaceutical composition comprising a preparation comprising an antigen-binding molecule having at least one disulfide bond formed between amino acid residues in hinge regions other than a hinge region, wherein the preparation is produced by the method of any one of [1] to [52], [A1] to [A24], [B1] to [B15], [101] to [150], [201] to [265], [301] to [320], and [401] to [416].

[Effects of the Invention]

The present disclosure provides efficient and easy methods for producing and purifying antigen-binding molecules having appropriate disulfide bonds in regions other than the hinge region. Methods in the present disclosure enable the LINC-Ig format to be obtained easily as well as quickly and in high yield. The methods in the present disclosure have the advantage that a wide range of the type, concentration, pH, and reaction time of a reducing agent is applicable.

Furthermore, production and purification of the LINC-Ig format inside a column, like those in the methods of the present disclosure, have advantages compared to such production and purification in a tank, such as the possibility of reducing aggregation of antigen-binding molecules, short reaction time, easy operativity, the possibility of automation by using programmed liquid chromatography (LC), the possibility of maintaining the concentration of the reducing agent in the reaction at a constant level, and ease of reproduction across scales.

[Brief Description of Drawings]

Fig. 1 shows the results of analyzing the samples reduced by Cys. Starting from the left, the Ctrl which skipped the reduction/oxidation step and performed affinity chromatography (1 sample), the 0.1-100 mmol/L Cys solutions at pH 7.0 (4 samples), and the 0.1-100 mmol/L Cys solutions at pH 8.0 (4 samples) are shown. At both pH 7.0 and 8.0, effective increase in LINC ratio was confirmed particularly at the concentration range of 0.1-10 mmol/L. Bands could not be confirmed on the gel for the results at 100 mmol/L, and since accurate LINC ratio cannot be calculated, the notation "-" is used.

Fig. 2 shows the results of analyzing the samples reduced by TCEP. Starting from the left, the Ctrl which skipped the reduction/oxidation step and performed affinity chromatography (1 sample), the 0.001-1 mmol/L TCEP solutions at pH 7.0 (4 samples), and the 0.001-1 mmol/L TCEP solutions at pH 8.0 (4 samples) are shown. Bands could not be confirmed on the gel for the results at 0.1-1 mmol/L, and since accurate LINC ratio cannot be calculated, the notation "-" is used.

Fig. 3 is a diagram showing the design and naming rule for a trivalent antibody in the DUAL/LINC (1+2) format.

Fig. 4 shows electropherograms from CE-SDS.

Fig. 5 shows chromatograms from HIS analysis, including peak names. A standard chromatogram (upper panel) and an enlarged chromatogram (lower panel) are shown.

[Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

The definitions and detailed description below are provided to facilitate understanding of the present disclosure illustrated herein.

I. Definitions

Herein, the meaning of the term "and/or" when describing the site of amino acid alteration includes every combination where "and" and "or" are suitably combined. Specifically, for example, "the amino acids at positions 33, 55, and/or 96 are substituted" includes the following variation of amino acid alterations: amino acid(s) at (a) position 33, (b) position 55, (c) position 96, (d) positions 33 and 55, (e) positions 33 and 96, (f) positions 55 and 96, and (g) positions 33, 55, and 96.

Unless otherwise indicated, amino acid residues in the light chain constant region are numbered herein according to Kabat et al., and numbering of amino acid residues in the heavy chain constant region is according to the EU numbering system, also called the EU index, as described in Kabat et al., Sequences of Proteins of Immunological Interest, 5th Ed. Public Health Service, National Institutes of Health, Bethesda, MD, 1991.

Amino acids

Herein, amino acids are described by one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Alteration of Amino Acids

For amino acid alteration (also herein described as "amino acid substitution" or "amino acid mutation") in the amino acid sequence of an antigen-binding molecule, known methods such as site-directed mutagenesis methods (Kunkel et al. (Proc. Natl. Acad. Sci. USA (1985) 82, 488-492)) and overlap extension PCR may be appropriately employed. Furthermore, several known methods may also be employed as amino acid alteration methods for substitution to non-natural amino acids (Annu Rev. Biophys. Biomol. Struct. (2006) 35, 225-249; and Proc. Natl. Acad. Sci. U.S.A. (2003) 100 (11), 6353-6357). For example, it is suitable to use a cell-free translation system (Clover Direct (Protein Express)) containing a tRNA which has a non-natural amino acid bound to a complementary amber suppressor tRNA of one of the stop codons, the UAG codon (amber codon).

Furthermore, herein, as an expression showing alteration of amino acids, an expression that shows before and after a number indicating a specific position, one-letter or three-letter codes for amino acids before and after alteration, respectively, may be used appropriately. For example, the alteration N100bL or Asn100bLeu used when substituting an amino acid contained in an antibody variable region indicates substitution of Asn at position 100b (according to Kabat numbering) with Leu. That is, the number shows the amino acid position according to Kabat numbering, the one-letter or three-letter amino-acid code written before the number shows the amino acid before substitution, and the one-letter or three-letter amino-acid code written after the number shows the amino acid after substitution. Similarly the alteration P238D or Pro238Asp used when substituting an amino acid of the Fc region contained in an antibody constant region indicates substitution of Pro at position 238 (according to EU numbering) with Asp. That is, the number shows the amino acid position according to EU numbering, the one-letter or three-letter amino-acid code written before the number shows the amino acid before substitution, and the one-letter or three-letter amino-acid code written after the number shows the amino acid after substitution.

Polypeptides

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide as described herein may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

Percent (%) amino acid sequence identity

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

Antigen-binding molecules

As used herein, the term "antigen-binding molecule" refers to, in its broadest sense, any molecule that specifically binds to an antigenic determinant (epitope), and comprises an antigen-binding domain or has binding activity to an antigen, and may further refers to molecules such as a peptide or protein having a length of about five amino acids or more. The peptide and protein are not limited to those derived from a living organism, and for example, they may be a polypeptide produced from an artificially designed sequence. They may also be any of a naturally-occurring polypeptide, synthetic polypeptide, recombinant polypeptide, and such. A scaffold molecule comprising known stable conformational structure such as alpha/beta barrel as scaffold, and in which part of the molecule is made into antigen-binding site, is also one embodiment of the antigen binding molecule described herein. In one embodiment, an antigen-binding molecule may comprise 2 or more (e.g., 2, 3, 4, 5, or more) polypeptide chains. In one embodiment, polypeptide chains constituting an antigen-binding molecule may be antibody heavy chains or antibody light chains. Specifically, in one embodiment, an antigen binding molecule is an antibody, an antibody fragment, or an antibody derivative. In one embodiment, an antigen-binding molecule is a trivalent antibody comprising chains 1 to 5 as shown in Fig. 3 of the present application. In one embodiment, an antigen-binding molecule is a non-antibody protein, or a fragment thereof or a derivative thereof.

Multispecific antigen-binding molecules

"Multispecific antigen-binding molecules" refers to antigen-binding molecules that bind specifically to more than one antigen. The term "bispecific" means that the antigen binding molecule is able to specifically bind to at least two distinct antigenic determinants. The term "trispecific" means that the antigen binding molecule is able to specifically bind to at least three distinct antigenic determinants.
In certain embodiments, the multispecific antigen binding molecule of the present application is a trispecific antigen binding molecule that is capable of binding to either one of CD3 or CD137 but does not bind to both antigens simultaneously, and is capable of specifically binding to DLL3.

Antigen-binding domains

Herein, "antigen-binding domain" refers to a region that specifically binds and is complementary to the whole or a portion of an antigen. Herein, an antigen-binding molecule comprises an antigen-binding domain. When the molecular weight of an antigen is large, an antigen-binding domain can only bind to a particular portion of the antigen. The particular portion is called "epitope". In one embodiment, an antigen-binding domain comprises an antibody fragment which binds to a particular antigen. An antigen-binding domain can be provided from one or more antibody variable domains. In a non-limiting embodiment, the antigen-binding domains comprise both the antibody light chain variable region (VL) and antibody heavy chain variable region (VH). Examples of such antigen-binding domains include "single-chain Fv (scFv)", "single-chain antibody", "Fv", "single-chain Fv2 (scFv2)", "Fab", and "Fab'". In other embodiments, an antigen-binding domain comprises a non-antibody protein which binds to a particular antigen, or a fragment thereof. In a specific embodiment, an antigen-binding domain comprises one or two Fabs including a CH1 region. In a specific embodiment, an antigen-binding domain comprises a hinge region.

As used herein, the term "antigen binding domain" is able to direct the entity to which it is attached to a target site, for example to a specific type of tumor cell expressing the cancer antigen (DLL3). An antigen binding domain is able to activate signaling through its target antigen, for example a T cell receptor complex antigen (in particular CD3) and/or a costimulatory receptor (CD137).

As used herein, the terms "first", "second" and "third" with respect to antigen binding domains etc., are used for convenience of distinguishing when there is more than one of each type of moiety. Use of these terms is not intended to confer a specific order or orientation of the antigen binding molecule unless explicitly so stated.

Herein, "specifically binds" means binding in a state where one of the molecules involved in specific binding does not show any significant binding to molecules other than a single or a number of binding partner molecules. Furthermore, it is also used when an antigen-binding domain is specific to a particular epitope among multiple epitopes contained in an antigen. When an epitope bound by an antigen-binding domain is contained in multiple different antigens, antigen-binding molecules comprising the antigen-binding domain can bind to various antigens that have the epitope.

In the present disclosure, the recitation "binds to the same epitope" means that the epitopes to which two antigen-binding domains bind at least partially overlap each other. The degree of the overlap is, but not limited to, at least 10% or more, preferably 20% or more, 30% or more, 40% or more, 50% or more, 60% or more, 70% or more, and 80% or more, particularly preferably 90% or more, and most preferably 100%.

Antibodies

The term "antibody" herein is used in the broadest sense and encompasses various antibody structures, including but not limited to monoclonal antibodies, polyclonal antibodies, multispecific antibodies (e.g., bispecific antibodies or trispecific antibodies), and antibody fragments so long as they exhibit the desired antigen-binding activity.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called alpha (IgA), delta (IgD), epsilon (IgE), gamma (IgG), or mu (IgM), some of which may be further divided into subtypes, e.g. gamma 1 (IgG1), gamma 2 (IgG2), gamma 3 (IgG3), gamma 4 (IgG4), alpha 1 (IgA1) and alpha 2 (IgA2). The light chain of an immunoglobulin may be assigned to one of two types, called kappa and lambda , based on the amino acid sequence of its constant domain. An immunoglobulin essentially consists of two Fab molecules and an Fc domain, linked via the immunoglobulin hinge region.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The terms "full length antibody," "intact antibody," and "whole antibody" are used herein interchangeably to refer to an antibody having a structure substantially similar to a native antibody structure or having heavy chains that contain an Fc region as defined herein.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

HVR-H1, HVR-H2, HVR-H3, HVR-L1, HVR-L2, and HVR-L3 are also mentioned as "H-CDR1", "H-CDR2", "H-CDR3", "L-CDR1", "L-CDR2", and "L-CDR3", respectively.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

The term "hinge region" denotes an antibody heavy chain polypeptide portion in a wild-type antibody heavy chain that joins the CH1 domain and the CH2 domain, e.g., from about position 216 to about position 230 according to the EU numbering system, or from about position 226 to about position 243 according to the Kabat numbering system. It is known that in a native IgG antibody, cysteine residue at position 220 according to EU numbering in the hinge region forms a disulfide bond with cysteine residue at position 214 in the antibody light chain. It is also known that between the two antibody heavy chains, disulfide bonds are formed between cysteine residues at position 226 and between cysteine residues at position 229 according to EU numbering in the hinge region. In general, a "hinge region" is defined as extending from human IgG1 from 216 to 238 (EU numbering) or from 226 to 251 (Kabat numbering). This hinge can be further divided into three different regions, an upper hinge, a central hinge and a lower hinge. In human IgG1 antibodies, these regions are generally defined as follows:

Upper hinge: 216-225 (EU numbering) or 226-238 (Kabat numbering),
Central hinge: 226-230 (EU numbering) or 239-243 (Kabat numbering),

Lower hinge: 231-238 (EU numbering) or 244-251 (Kabat numbering).

The hinge region of other IgG isotypes can be aligned with the IgG1 sequence by placing the first and last cysteine residues that form an interheavy chain SS bond in the same position (e.g., Brekke et al., 1995, Immunol (See Table 1 of Today 16: 85-90). A hinge region herein includes wild-type hinge regions as well as variants in which amino acid residue(s) in a wild-type hinge region is altered by substitution, addition, or deletion.

The terms (amino acid residues are) "capable of being linked" and (a disulfide bond is) "capable of being formed" include cases where a disulfide bond has already been formed, and cases where a disulfide bond is not formed but may be formed later under suitable conditions.

The term "disulfide bond formed between amino acids which are not in a hinge region" (or "disulfide bond formed between amino acid residues in regions other than a hinge region") means disulfide bond formed, connected or linked through amino acids located in any antibody region which is outside of the "hinge region" defined above. For example, such disulfide bond is formed, connected or linked through amino acids in any position in an antibody other than in a hinge region (e.g., from about position 216 to about position 230 according to the EU numbering system, or from about position 226 to about position 243 according to the Kabat numbering system). In some embodiments, such disulfide bond is formed, connected or linked through amino acids located in a CH1 region, a CL region, a VL region, a VH region and/or a VHH region. In some embodiments, such disulfide bond is formed, connected or linked through amino acids located in positions 119 to 123, 131 to 140, 148 to 150, 155 to 167, 174 to 178, 188 to 197, 201 to 214, according to EU numbering, in the CH1 region. In some embodiments, such disulfide bond is formed, connected or linked through amino acids located in positions 119, 122, 123, 131, 132, 133, 134, 135, 136, 137, 138, 139, 140, 148, 150, 155, 156, 157, 159, 160, 161, 162, 163, 164, 165, 167, 174, 176, 177, 178, 188, 189, 190, 191, 192, 193, 194, 195, 196, 197, 201, 203, 205, 206, 207, 208, 211, 212, 213, 214 according to EU numbering, in the CH1 region. In some embodiments, such disulfide bond is formed, connected or linked through amino acids located in positions 188, 189, 190, 191, 192, 193, 194, 195, 196, and 197, according to EU numbering, in the CH1 region. In one preferred embodiment, such disulfide bond is formed, connected or linked through amino acids located in position 191, according to EU numbering, in the CH1 region.

The term "mis-disulfide bonded forms" means antigen-binding molecules in which disulfide bonds are formed between amino acid residues that are different from desired amino acid residues. When an antigen-binding molecule comprises disulfide bonds formed between desired amino acid residues and disulfide bonds formed between amino acid residues that are different from desired amino acid residues, such an antigen-binding molecule is included among the mis-disulfide bonded forms herein.

The term "non-disulfide bonded forms" refers to antigen-binding molecules in which cysteine residues remain in the free state, or in which the cysteine residues form disulfide bonds with molecules (such as impurity molecules) which are different from molecules that form antigen-binding molecules. "Non-disulfide bonded forms" described herein only need to have such characteristics, and for example, even if they contain disulfide bonds formed between amino acid residues in regions other than the hinge region (desired disulfide bonds), or disulfide bonds different from the desired disulfide bonds (mis-disulfide bonded parts), as long as they have the above-mentioned characteristics, they are included in the "non-disulfide bonded forms" herein.

Constant regions are preferably antibody constant regions, more preferably IgG1, IgG2, IgG3, and IgG4-type antibody constant regions, and even more preferably human IgG1, IgG2, IgG3, and IgG4-type antibody constant regions. Furthermore, constant regions are preferably heavy chain constant regions, more preferably IgG1, IgG2, IgG3, and IgG4-type heavy chain constant regions, and even more preferably human IgG1, IgG2, IgG3, and IgG4-type heavy chain constant regions. The amino acid sequences of the human IgG1 constant region, the human IgG2 constant region, the human IgG3 constant region, and the human IgG4 constant region are known. For the constant regions of human IgG1, human IgG2, human IgG3, and human IgG4, a plurality of allotype sequences with genetic polymorphism are described in Sequences of proteins of immunological interest, NIH Publication No.91-3242, and any of them can be used in the present invention. Amino acid-modified constant regions may contain other amino acid mutations or modifications, as long as they include an amino acid mutation of the present invention.

Herein, the term "Fc region" or "Fc domain" refers to a region comprising a fragment consisting of a hinge or a portion thereof and CH2 and CH3 domains in an antibody molecule. The Fc region of IgG class means, but is not limited to, a region from, for example, cysteine 226 (EU numbering (also referred to as EU index herein)) to the C terminus or proline 230 (EU numbering) to the C terminus. The Fc region can be preferably obtained by the partial digestion of, for example, an IgG1, IgG2, IgG3, or IgG4 monoclonal antibody with a proteolytic enzyme such as pepsin followed by the re-elution of a fraction adsorbed on a protein A column or a protein G column. Such a proteolytic enzyme is not particularly limited as long as the enzyme is capable of digesting a whole antibody to restrictively form Fab or F(ab')2 under appropriately set reaction conditions (e.g., pH) of the enzyme. Examples thereof can include pepsin and papain.

An Fc region derived from, for example, naturally occurring IgG can be used as the "Fc region". In this context, the naturally occurring IgG means a polypeptide that contains an amino acid sequence identical to that of IgG found in nature and belongs to a class of an antibody substantially encoded by an immunoglobulin gamma gene. The naturally occurring human IgG means, for example, naturally occurring human IgG1, naturally occurring human IgG2, naturally occurring human IgG3, or naturally occurring human IgG4. The naturally occurring IgG also includes variants or the like spontaneously derived therefrom. A plurality of allotype sequences based on gene polymorphism are described as the constant regions of human IgG1, human IgG2, human IgG3, and human IgG4 antibodies in Sequences of proteins of immunological interest, NIH Publication No. 91-3242, any of which can be used in the present invention. Particularly, the sequence of human IgG1 may have DEL or EEM as an amino acid sequence of EU numbering positions 356 to 358.

The Fc domain of the antigen binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment the antigen binding molecule described herein comprises not more than one Fc domain.

Herein, the Fc domain of the antigen binding molecule is an IgG Fc domain. In a particular embodiment the Fc domain is an IgG1 Fc domain. In another embodiment the Fc domain is an IgG1 Fc domain. In a further particular embodiment the Fc domain is a human IgG1 Fc region.

Chimeric antibody

The term "chimeric" antibody refers to an antibody in which a portion of the heavy and/or light chain is derived from a particular source or species, while the remainder of the heavy and/or light chain is derived from a different source or species. Similarly, the term "chimeric antibody variable domain" refers to an antibody variable region in which a portion of the heavy and/or light chain variable region is derived from a particular source or species, while the remainder of the heavy and/or light chain variable region is derived from a different source or species.

Humanized antibody

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization. A "humanized antibody variable region" refers to the variable region of a humanized antibody.

Antibody fragment

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Pluckthun, in The Pharmacology of Monoclonal Antibodies, vol. 113, Rosenburg and Moore eds., Springer-Verlag, New York, pp. 269-315 (1994); see also WO 93/16185 ; and U.S. Patent Nos. 5,571,894 and 5,587,458 . For discussion of Fab and F(ab')2 fragments comprising salvage receptor binding epitope residues and having increased in vivo half-life, see U.S. Patent No. 5,869,046 . Diabodies are antibody fragments with two antigen-binding sites that may be bivalent or bispecific. See, for example, EP 404,097 ; WO 1993/01161 ; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Fv (variable fragment)

Herein, the term "variable fragment (Fv)" refers to the minimum unit of an antibody-derived antigen-binding domain that is composed of a pair of the antibody light chain variable region (VL) and antibody heavy chain variable region (VH). In 1988, Skerra and Pluckthun found that homogeneous and active antibodies can be prepared from the E. coli periplasm fraction by inserting an antibody gene downstream of a bacterial signal sequence and inducing expression of the gene in E. coli (Science (1988) 240(4855), 1038-1041). In the Fv prepared from the periplasm fraction, VH associates with VL in a manner so as to bind to an antigen.

scFv, single-chain antibody, and sc(Fv)2

Herein, the terms "scFv", "single-chain antibody", and "sc(Fv)2" all refer to an antibody fragment of a single polypeptide chain that contains variable regions derived from the heavy and light chains, but not the constant region. In general, a single-chain antibody also contains a polypeptide linker between the VH and VL domains, which enables formation of a desired structure that is thought to allow antigen binding. The single-chain antibody is discussed in detail by Pluckthun in "The Pharmacology of Monoclonal Antibodies, Vol. 113, Rosenburg and Moore, eds., Springer-Verlag, New York, 269-315 (1994)". See also International Patent Publication WO 1988/001649; US Patent Nos. 4,946,778 and 5,260,203. In a particular embodiment, the single-chain antibody can be bispecific and/or humanized.

scFv is an antigen-binding domain in which VH and VL forming Fv are linked together by a peptide linker (Proc. Natl. Acad. Sci. U.S.A. (1988) 85(16), 5879-5883). VH and VL can be retained in close proximity by the peptide linker.

sc(Fv)2 is a single-chain antibody in which four variable regions of two VL and two VH are linked by linkers such as peptide linkers to form a single chain (J Immunol. Methods (1999) 231(1-2), 177-189). The two VH and two VL may be derived from different monoclonal antibodies. Such sc(Fv)2 preferably includes, for example, a bispecific sc(Fv)2 that recognizes two epitopes present in a single antigen as disclosed in the Journal of Immunology (1994) 152(11), 5368-5374. sc(Fv)2 can be produced by methods known to those skilled in the art. For example, sc(Fv)2 can be produced by linking scFv by a linker such as a peptide linker.

Herein, the forms of an antigen-binding domain forming an sc(Fv)2 include an antibody in which the two VH units and two VL units are arranged in the order of VH, VL, VH, and VL ([VH]-linker-[VL]-linker-[VH]-linker-[VL]) beginning from the N terminus of a single-chain polypeptide. The order of the two VH units and two VL units is not limited to the above form, and they may be arranged in any order. Example order of the form is listed below.

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

The molecular form of sc(Fv)$_2$ is also described in detail in WO 2006/132352. According to these descriptions, those skilled in the art can appropriately prepare desired sc(Fv)$_2$ to produce the polypeptide complexes disclosed herein.

Furthermore, the antigen-binding molecules or antibodies in the present disclosure may be conjugated with a carrier polymer such as PEG or an organic compound such as an anticancer agent. Alternatively, a sugar chain addition sequence is preferably inserted into the antigen-binding molecules or antibodies such that the sugar chain produces a desired effect.

The linkers to be used for linking the variable regions of an antibody comprise arbitrary peptide linkers that can be introduced by genetic engineering, synthetic linkers, and linkers disclosed in, for example, Protein Engineering, 9(3), 299-305, 1996. However, peptide linkers are preferred in the present disclosure. The length of the peptide linkers is not particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids. When sc(Fv)$_2$ contains three peptide linkers, their length may be all the same or different.

For example, such peptide linkers include:

Ser,
Gly-Ser,
Gly-Gly-Ser,
Ser-Gly-Gly,
Gly-Gly-Gly-Ser (SEQ ID NO: 5),
Ser-Gly-Gly-Gly (SEQ ID NO: 6),
Gly-Gly-Gly-Gly-Ser (SEQ ID NO: 7),
Ser-Gly-Gly-Gly-Gly (SEQ ID NO: 8),
Gly-Gly-Gly-Gly-Gly-Ser (SEQ ID NO: 9),
Ser-Gly-Gly-Gly-Gly-Gly (SEQ ID NO: 10),
Gly-Gly-Gly-Gly-Gly-Gly-Ser (SEQ ID NO: 11),
Ser-Gly-Gly-Gly-Gly-Gly-Gly (SEQ ID NO: 12),
(Gly-Gly-Gly-Gly-Ser (SEQ ID NO: 7))n, and

(Ser-Gly-Gly-Gly-Gly (SEQ ID NO: 8))n,

where n is an integer of 1 or larger. The length or sequences of peptide linkers can be selected accordingly by those skilled in the art depending on the purpose.

Synthetic linkers (chemical crosslinking agents) are routinely used to crosslink peptides, and examples include:

N-hydroxy succinimide (NHS),
disuccinimidyl suberate (DSS),
bis(sulfosuccinimidyl) suberate (BS3),
dithiobis(succinimidyl propionate) (DSP),
dithiobis(sulfosuccinimidyl propionate) (DTSSP),
ethylene glycol bis(succinimidyl succinate) (EGS),
ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES), and
bis[2-(sulfosuccinimidoxycarbonyloxy)ethyl] sulfone (sulfo-BSOCOES). These crosslinking agents are commercially available.

In general, three linkers are required to link four antibody variable regions together. The linkers to be used may be of the same type or different types.

Fab, F(ab')2, and Fab'

A "Fab molecule" refers to a protein consisting of the VH and CH1 domain of the heavy chain (the "Fab heavy chain") and the VL and CL domain of the light chain (the "Fab light chain") of an immunoglobulin. The heavy chain of a wild-type Fab molecule cannot form disulfide bonds with another heavy chain molecule. Herein, in addition to wild-type Fab molecules, Fab variants in which amino acid residue(s) in a wild-type Fab molecule is altered by substitution, addition, or deletion are also included. In a specific embodiment, mutated amino acid residue(s) comprised in Fab variants (e.g., cysteine residue(s) or lysine residue(s) after substitution, addition, or insertion) can form disulfide bond(s) with another heavy chain molecule or a portion thereof (e.g., Fab molecule).

scFab is an antigen-binding domain in which a single light chain, and a CH1 region and variable region from a single heavy chain which form Fab are linked together by a peptide linker. The light chain, and the CH1 region and variable region from the heavy chain can be retained in close proximity by the peptide linker.

"F(ab')2" or "Fab" is produced by treating an immunoglobulin (monoclonal antibody) with a protease such as pepsin and papain, and refers to an antibody fragment generated by digesting an immunoglobulin (monoclonal antibody) at near the disulfide bonds present between the hinge regions in each of the two H chains. For example, papain cleaves IgG upstream of the disulfide bonds present between the hinge regions in each of the two H chains to generate two homologous antibody fragments, in which an L chain comprising VL (L-chain variable region) and CL (L-chain constant region) is linked to an H-chain fragment comprising VH (H-chain variable region) and CH gamma 1 (gamma 1 region in an H-chain constant region) via a disulfide bond at their C-terminal regions. Each of these two homologous antibody fragments is called Fab'.

"F(ab')2" consists of two light chains and two heavy chains comprising the constant region of a CH1 domain and a portion of CH2 domains so that disulfide bonds are formed between the two heavy chains. The F(ab')2 disclosed herein can be preferably produced as follows. A whole monoclonal antibody or such comprising a desired antigen-binding domain is partially digested with a protease such as pepsin; and Fc fragments are removed by adsorption onto a Protein A column. The protease is not particularly limited, as long as it can cleave the whole antibody in a selective manner to produce F(ab')2 under an appropriate setup enzyme reaction condition such as pH. Such proteases include, for example, pepsin and ficin.

Fused

By "fused" is meant that the components (e.g. a Fab molecule and an Fc domain subunit) are linked by peptide bonds, either directly or via one or more peptide linkers.

"Crossover" Fab

By a "crossover" Fab molecule (also termed "Crossfab") is meant a Fab molecule wherein either the variable regions or the constant regions of the Fab heavy and light chain are exchanged, i.e. the crossover Fab molecule comprises a peptide chain composed of the light chain variable region and the heavy chain constant region, and a peptide chain composed of the heavy chain variable region and the light chain constant region. For clarity, in a crossover Fab molecule wherein the variable regions of the Fab light chain and the Fab heavy chain are exchanged, the peptide chain comprising the heavy chain constant region is referred to herein as the "heavy chain" of the crossover Fab molecule. Conversely, in a crossover Fab molecule wherein the constant regions of the Fab light chain and the Fab heavy chain are exchanged, the peptide chain comprising the heavy chain variable region is referred to herein as the "heavy chain" of the crossover Fab molecule.

"Conventional" Fab

In contrast thereto, by a "conventional" Fab molecule is meant a Fab molecule in its natural format, i.e. comprising a heavy chain composed of the heavy chain variable and constant regions (VH-CH1), and a light chain composed of the light chain variable and constant regions (VL-CL).

Single domain antibodies

Herein, those referred to by the term "single domain antibodies" are not particularly limited in their structure, as long as the domain can exert antigen-binding activity by itself. Ordinary antibodies exemplified by IgG antibodies exert antigen-binding activity in a state where a variable region is formed by the pairing of VH and VL. In contrast, a single domain antibody is known to be able to exert antigen-binding activity by its own domain structure alone without pairing with another domain. Single domain antibodies usually have a relatively low molecular weight and exist in the form of a monomer.

Examples of a single domain antibody include, but are not limited to, antigen binding molecules which naturally lack light chains, such as VHH of Camelidae animals and $V_{NAR}$ of sharks, and antibody fragments comprising the whole or a portion of an antibody VH domain or the whole or a portion of an antibody VL domain. Examples of a single domain antibody which is an antibody fragment comprising the whole or a portion of an antibody VH/VL domain include, but are not limited to, artificially prepared single domain antibodies originating from a human antibody VH or a human antibody VL as described, e.g., in US Patent No. 6,248,516 B1. One single domain antibody has three CDRs (CDR1, CDR2, and CDR3).

Single domain antibodies can be obtained from animals capable of producing single domain antibodies or by immunizing animals capable of producing single domain antibodies. Examples of animals capable of producing single domain antibodies include, but are not limited to, camelids and transgenic animals into which gene(s) for the capability of producing a single domain antibody has been introduced. Camelids include camel, llama, alpaca, dromedary, guanaco, and such. Examples of a transgenic animal into which gene(s) for the capability of producing a single domain antibody has been introduced include, but are not limited to, the transgenic animals described in International Publication No. WO2015/143414 or US Patent Publication No. US2011/0123527 A1. Humanized single chain antibodies can also be obtained, by replacing framework sequences of a single domain antibody obtained from an animal with human germline sequences or sequences similar thereto. A humanized single domain antibody (e.g., humanized VHH) is one embodiment of the single domain antibody.

Alternatively, single domain antibodies can be obtained from polypeptide libraries containing single domain antibodies by ELISA, panning, and such. Examples of polypeptide libraries containing single domain antibodies include, but are not limited to, naive antibody libraries obtained from various animals or humans (e.g., Methods in Molecular Biology 2012 911 (65-78) and Biochimica et Biophysica Acta - Proteins and Proteomics 2006 1764:8 (1307-1319)), antibody libraries obtained by immunizing various animals (e.g., Journal of Applied Microbiology 2014 117:2 (528-536)), and synthetic antibody libraries prepared from antibody genes of various animals or humans (e.g., Journal of Biomolecular Screening 2016 21:1 (35-43), Journal of Biological Chemistry 2016 291:24 (12641-12657), and AIDS 2016 30:11 (1691-1701)).

An "agonist" antigen-binding molecule or "agonist" antibody, as used herein, is an antigen-binding molecule or antibody which significantly potentiates a biological activity of the antigen it binds.

A "blocking" antigen-binding molecule or "blocking" antibody, or an "antagonist" antigen-binding molecule or "antagonist" antibody, as used herein, is an antigen-binding molecule or antibody which significantly inhibits (either partially or completely) a biological activity of the antigen it binds.

Antigen

As used herein, the term "antigen" refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. CD3, CD137, DLL3) can be any native form the proteins from any vertebrate source, including mammals such as primates (e.g. humans) and rodents (e.g. mice and rats), unless otherwise indicated. In a particular embodiment the antigen is a human CD3, human CD137 or human DLL3. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

Fc region with a reduced Fc gamma receptor-binding activity

Herein, "a reduced Fc gamma receptor-binding activity" means, for example, that based on the above-described analysis method the competitive activity of a test antigen-binding molecule or antibody is 50% or less, preferably 45% or less, 40% or less, 35% or less, 30% or less, 20% or less, or 15% or less, and particularly preferably 10% or less, 9% or less, 8% or less, 7% or less, 6% or less, 5% or less, 4% or less, 3% or less, 2% or less, or 1% or less than the competitive activity of a control antigen-binding molecule or antibody.

Antigen-binding molecules or antibodies comprising the Fc domain of a monoclonal IgG1, IgG2, IgG3, or IgG4 antibody can be appropriately used as control antigen-binding molecules or antibodies. The Fc domain structures are shown in SEQ ID NOs: 1 (A is added to the N terminus of RefSeq accession number AAC82527.1), 2 (A is added to the N terminus of RefSeq accession number AAB59393.1), 3 (A is added to the N terminus of RefSeq accession number CAA27268.1), and 4 (A is added to the N terminus of RefSeq accession number AAB59394.1). Furthermore, when an antigen-binding molecule or antibody comprising an Fc domain mutant of an antibody of a particular isotype is used as a test substance, the effect of the mutation of the mutant on the Fc gamma receptor-binding activity is assessed using as a control an antigen-binding molecule or antibody comprising an Fc domain of the same isotype. As described above, antigen-binding molecules or antibodies comprising an Fc domain mutant whose Fc gamma receptor-binding activity has been judged to be reduced are appropriately prepared.

Such known mutants include, for example, mutants having a deletion of amino acids 231A-238S (EU numbering) (WO 2009/011941), as well as mutants C226S, C229S, P238S, (C220S) (J. Rheumatol (2007) 34, 11); C226S and C229S (Hum. Antibod. Hybridomas (1990) 1(1), 47-54); C226S, C229S, E233P, L234V, and L235A (Blood (2007) 109, 1185-1192).

Specifically, the preferred antigen-binding molecules or antibodies include those comprising an Fc domain with a mutation (such as substitution) of at least one amino acid selected from the following amino acid positions: 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, 332, 439, 445, 449, or 451 (EU numbering), in the amino acids forming the Fc domain of an antibody of a particular isotype. The isotype of antibody from which the Fc domain originates is not particularly limited, and it is possible to use an appropriate Fc domain derived from a monoclonal IgG1, IgG2, IgG3, or IgG4 antibody. It is preferable to use Fc domains derived from IgG1 antibodies.

The preferred antigen-binding molecules or antibodies include, for example, those comprising an Fc domain which has any one of the substitutions shown below, whose positions are specified according to EU numbering (each number represents the position of an amino acid residue in the EU numbering; and the one-letter amino acid symbol before the number represents the amino acid residue before substitution, while the one-letter amino acid symbol after the number represents the amino acid residue after the substitution) in the amino acids forming the Fc domain of IgG1 antibody:

    (a) L234F, L235E, P331S;
    (b) C226S, C229S, P238S;
    (c) C226S, C229S; or
    (d) C226S, C229S, E233P, L234V, L235A;
    (e) L439M, A445N, R449Q, E451S
    (f) M428L, N434A, Q438R, S440E

as well as those having an Fc domain which has a deletion of the amino acid sequence at positions 231 to 238.

Furthermore, the preferred antigen-binding molecules or antibodies also include those comprising an Fc domain that has any one of the substitutions shown below, whose positions are specified according to EU numbering in the amino acids forming the Fc domain of an IgG2 antibody:

    (e) H268Q, V309L, A330S, and P331S;
    (f) V234A;
    (g) G237A;
    (h) V234A and G237A;
    (i) A235E and G237A; or
    (j) V234A, A235E, and G237A. Each number represents the position of an amino acid residue in EU numbering; and the one-letter amino acid symbol before the number represents the amino acid residue before substitution, while the one-letter amino acid symbol after the number represents the amino acid residue after the substitution.

Furthermore, the preferred antigen-binding molecules or antibodies also include those comprising an Fc domain that has any one of the substitutions shown below, whose positions are specified according to EU numbering in the amino acids forming the Fc domain of an IgG3 antibody:

(k) F241A;
(1) D265A; or
(m) V264A. Each number represents the position of an amino acid residue in EU numbering; and the one-letter amino acid symbol before the number represents the amino acid residue before substitution, while the one-letter amino acid symbol after the number represents the amino acid residue after the substitution.

Furthermore, the preferred antigen-binding molecules or antibodies also include those comprising an Fc domain that has any one of the substitutions shown below, whose positions are specified according to EU numbering in the amino acids forming the Fc domain of an IgG4 antibody:

(n) L235A, G237A, and E318A;
(o) L235E; or
(p) F234A and L235A. Each number represents the position of an amino acid residue in EU numbering; and the one-letter amino acid symbol before the number represents the amino acid residue before substitution, while the one-letter amino acid symbol after the number represents the amino acid residue after the substitution.

The other preferred antigen-binding molecules or antibodies include, for example, those comprising an Fc domain in which any amino acid at position 233, 234, 235, 236, 237, 327, 330, or 331 (EU numbering) in the amino acids forming the Fc domain of an IgG1 antibody is substituted with an amino acid of the corresponding position in EU numbering in the corresponding IgG2 or IgG4.
The preferred antigen-binding molecules or antibodies also include, for example, those comprising an Fc domain in which any one or more of the amino acids at positions 234, 235, and 297 (EU numbering) in the amino acids forming the Fc domain of an IgG1 antibody is substituted with other amino acids. The type of amino acid after substitution is not particularly limited; however, the antigen-binding molecules or antibodies comprising an Fc domain in which any one or more of the amino acids at positions 234, 235, and 297 are substituted with alanine are particularly preferred.
The preferred antigen-binding molecules or antibodies also include, for example, those comprising an Fc domain in which an amino acid at position 265 (EU numbering) in the amino acids forming the Fc domain of an IgG1 antibody is substituted with another amino acid. The type of amino acid after substitution is not particularly limited; however, antigen-binding molecules or antibodies comprising an Fc domain in which an amino acid at position 265 is substituted with alanine are particularly preferred.

Preferentially enriched (or increased)

The term "preferentially enriched (or increased)" means an increase in relative abundance of a desired form, or increase in relative proportion of a desired form, or increase in the population of a desired form (structural isoform). In some embodiments, the methods described herein increase relative abundance of an antibody structural isoform such as an antibody having at least one disulfide bond formed between amino acid residues outside of the hinge region. In one embodiment, said at least one disulfide bond is formed between the amino acid residues at position 191 according to EU numbering in the respective CH1 regions of the first antigen-binding domain and the second antigen-binding domain. In certain embodiment, said methods produce a homogenous antibody preparation having at least 50%, 60%, 70%, 80%, 90%, preferably at least 95% molar ratio of said antibody having at least one disulfide bond formed outside of the hinge region.

Homogeneous

A "homogeneous" population of an antibody means an antibody population that comprises largely a single form of the antibody, for example, at least 50%, 60%, 70%, 80% or more, preferably at least 90%, 95%, 96%, 97%, 99% or 100% of the antibody in the solution or composition is in the properly folded form. Similarly, a "homogeneous" population of an antibody having at least one disulfide bond formed outside of the hinge region means a population of said antibody which comprises largely a single, properly folded form, for example, at least 50%, 60%, 70%, 80% or more, preferably at least 90%, 95%, 96%, 97%, 99% or 100% molar ratio of said antibody having at least one disulfide bond formed outside of the hinge region. In one preferred embodiment, said "homogeneous" population of an antibody comprises at least one disulfide bond which is formed between the amino acid residues at position 191 according to EU numbering in the respective CH1 regions of the first antigen-binding domain and the second antigen-binding domain (i.e. "paired cysteines" at the position 191 according to EU numbering in the CH1 region).

Determining whether an antibody population is homogenous, and the relative abundance or proportions of a conformation of a protein/antibody in a mixture, can be done using any of a variety of analytical and/or qualitative techniques. If the two conformations resolve differently during separation techniques such as chromatography, electrophoresis, filtering or other purification technique, then the relative proportion of a conformation in the mixture can be determined using such purification techniques. For example, at least two different conformations of the recombinant IgG could be resolved by way of hydrophobic interaction chromatography. Further, since far UV Circular Dichroism has been used to estimate secondary structure composition of proteins (Perczel et al., 1 991, Protein Engrg. 4:669-679), such a technique can determine whether alternative conformations of a protein are present. Still another technique used to determine conformation is fluorescence spectroscopy which can be employed to ascertain complementary differences in tertiary structure assignable to tryptophan and tyrosine fluorescence. Other techniques that can be used to determine differences in conformation and, hence, the relative proportions of a conformation, are on-line SEC to measure aggregation status, differential scanning calorimetry to measure melting transitions (Tm's) and component enthalpies, and chaotrope unfolding. Yet another technique that can be used to determine differences in conformation and, hence, the relative proportions of a conformation is LC/MS detection to determine the heterogeneity of the protein.

Alternatively, if there is a difference in activity between the conformations of the antibody/protein, determining the relative proportion of a conformation in the mixture can be done by way of an activity assay (e.g., binding to a ligand, enzymatic activity, biological activity, etc.). Biological activity of the protein also could be used. Alternatively, the binding assays can be used in which the activity is expressed as activity units/mg of protein.

To determine the heterogeneity of the antibody/protein, IEC chromatography is used. In such a case, the antibody is purified or considered to be "homogenous", which means that no polypeptide peaks or fractions corresponding to other polypeptides are detectable upon analysis by IEC chromatography. In certain embodiments, the antibody is purified or considered to be "homogenous" such that no polypeptide bands corresponding to other polypeptides are detectable upon analysis by SDS-polyacrylamide gel electrophoresis (SDS-PAGE). It will be recognized by one skilled in the pertinent field that multiple bands corresponding to the polypeptide can be visualized by SDS-PAGE, due to differential glycosylation, differential post-translational processing, and the like. Most preferably, the polypeptide is purified to substantial homogeneity, as indicated by a single polypeptide band upon analysis by SDS-PAGE. The polypeptide band can be visualized by silver staining, Coomassie blue staining, and/or (if the polypeptide is radiolabeled) by auto radiography.

Herein, examples of conditions of SDS-PAGE analysis are as follows. Non-reducing SDS-PAGE is performed using 4-20% Mini-PROTEAN (registered trademark) TGX Stain-Free™ Precast Gels (Bio-Rad) with 1x Tris/Glycine/SDS running buffer (Bio-Rad). Monoclonal antibody samples are heated at 70°C for 10 min. 0.2 microgram is loaded and electrophoresis is conducted at 200 V for 90 min. Proteins are visualized with Chemidoc Imaging System (Bio-Rad). Percentage of individual band is analyzed by the Image Lab software version 6.0 (Bio-Rad), in which % intensity of the individual band (e.g. faster migration (Lower band) and slower migration (Upper band)) are calculated by intensity of the band divided by the sum of these two bands. Then, the gel may be stained with CBB, and the gel image may be captured, and the bands may be quantified using an imaging device. In the gel image, several, for example, two bands, i.e., "upper band" and "lower band", may be observed for an antibody variant sample. In this case, the molecular weight of the upper band may correspond to that of the parent antibody (before modification). Structural changes such as crosslinking via disulfide bonds of Fabs may be caused by cysteine substitution, which may result in the change in electrophoretic mobility. In this case, the lower band may be considered to correspond to the antibody having one or more engineered disulfide bond(s) formed between the CH1 regions. Antibody variant samples with additional cysteine substitutions may show a higher lower band to upper band ratio, compared to control samples. Additional cysteine substitutions may enhance/promote disulfide bond crosslinking of Fabs; and may increase the percentage or structural homogeneity of an antibody preparation having an engineered disulfide bond formed at a mutated position; and may decrease the percentage of an antibody preparation having no engineered disulfide bond formed at the mutated position. Herein, the term "lower band to upper band ratio" refers to a ratio between the quantities/intensities of the lower and upper bands that may be quantified during the above-mentioned SDS-PAGE experiments.

The terms "pharmaceutical formulation" and "pharmaceutical composition" refer to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

An "individual" or "subject" is a mammal. Mammals include, but are not limited to, domesticated animals (e.g., cows, sheep, cats, dogs, and horses), primates (e.g., humans and non-human primates such as monkeys), rabbits, and

## EP 4 624 489 A1

rodents (e.g., mice and rats). In certain embodiments, the individual or subject is a human.

II. Methods for producing preparations comprising antigen-binding molecules

The present disclosure relates to methods for producing preparations comprising antigen-binding molecules having at least one disulfide bond formed between amino acid residues in regions other than the hinge region. Methods in the present disclosure comprise a step of subjecting a mixture to chromatography in the presence of a reducing agent, wherein the mixture comprises antigen-binding molecules having at least one disulfide bond formed between amino acid residues in regions other than the hinge region and mis-disulfide bonded forms and/or non-disulfide bonded forms of the antigen-binding molecules (hereinafter, the mixture may be referred to as "mixture comprising antigen-binding molecules", "mixture of antigen-binding molecules", or such). In the methods of the present disclosure, the mixture comprising antigen-binding molecules can be subjected to one type of chromatography once or multiple times. Alternatively, the mixture comprising antigen-binding molecules can be subjected to multiple types (for example, 2, 3, or more types) of chromatography once or multiple times (for example, twice, three times, or more) each. When chromatographic treatment is performed multiple times, these treatments may or may not be performed sequentially.

In the methods of the present disclosure, chromatography well known to those skilled in the art may be used. Examples include but are not limited to column chromatography, membrane chromatography, and thin layer chromatography.

In the production methods of the present disclosure, the step of subjecting a mixture comprising antigen-binding molecules to chromatography is performed in the presence of a reducing agent. In the production methods of the present disclosure, the embodiment of the step of subjecting a mixture comprising antigen-binding molecules to chromatography is not limited as long as a reducing agent is present. For example, the antigen-binding molecules can be loaded onto a chromatography matrix in advance and then the antigen-binding molecules can be contacted with the reducing agent, or a material resulting from mixing the mixture comprising antigen-binding molecules and the reducing agent can be allowed to flow through chromatography.

In one aspect, when using column chromatography, methods of the present disclosure may comprise a step of contacting a chromatography matrix loaded in a column for column chromatography with a mixture comprising antigen-binding molecules having at least one disulfide bond formed between amino acid residues in regions other than the hinge region and mis-disulfide bonded forms and/or non-disulfide bonded forms of the antigen-binding molecules. In one aspect, when membrane chromatography is used, methods of the present disclosure may comprise a step of contacting a chromatography matrix applied onto a membrane for membrane chromatography with a mixture comprising antigen-binding molecules having at least one disulfide bond formed between amino acid residues in regions other than the hinge region and mis-disulfide bonded forms and/or non-disulfide bonded forms of the antigen-binding molecules. The mixture comprising antigen-binding molecules can be contacted in advance with a reducing agent.

In the present disclosure, the step of subjecting a mixture comprising antigen-binding molecules having at least one disulfide bond formed between amino acid residues in regions other than the hinge region and mis-disulfide bonded forms and/or non-disulfide bonded forms of the antigen-binding molecules to chromatography can comprise the following steps:

(a) contacting a mixture comprising antigen-binding molecules having at least one disulfide bond formed between amino acid residues in regions other than the hinge region and mis-disulfide bonded forms and/or non-disulfide bonded forms of the antigen-binding molecules with a solution comprising a reducing agent; and
(b) removing the reducing agent.

In one aspect, the methods of the present disclosure produce or purify a population of homogeneous antigen-binding molecules or a preparation or concentrate of homogenous antigen-binding molecules by the steps described herein.

In one aspect, the methods of the present disclosure can increase the proportion of antigen-binding molecules having at least one disulfide bond formed between amino acid residues in regions other than the hinge region, relative to antigen-binding molecules having amino acid residues that may form at least one disulfide bond between amino acid residues in regions other than the hinge region (such antigen-binding molecules comprise antigen-binding molecules in which at least one disulfide bond is appropriately formed between amino acid residues in regions other than the hinge region, and antigen-binding molecules in which at least one disulfide bond is not appropriately formed between amino acid residues in regions other than the hinge region, namely, mis-disulfide bonded forms and/or non-disulfide bonded forms of the antigen-binding molecule), that are loaded on a matrix.

In one aspect, the methods of the present disclosure produce antigen-binding molecules having at least one disulfide bond between amino acid residues in regions other than the hinge region at a molar ratio of at least 50%, at least 60%, at least 70%, at least 80%, at least 90%, at least 95%, at least 96%, at least 97%, at least 98%, or at least 99% relative to antigen-binding molecules having amino acid residues that may form at least one disulfide bond between amino acid residues in regions other than the hinge region (such antigen-binding molecules comprise antigen-binding molecules in which at least one disulfide bond is appropriately formed between amino acid residues in regions other than the hinge region, and antigen-binding molecules in which at least one disulfide bond is not appropriately formed between amino acid residues in regions other than the hinge region, or in other words, mis-disulfide bonded forms and/or non-disulfide bonded forms of the antigen-binding molecules), that are loaded on a matrix.

Therefore, in one embodiment, the present disclosure relates to methods for producing a preparation comprising antigen-binding molecules having at least one disulfide bond formed between amino acid residues in regions other than the hinge region at increased concentration. In one embodiment, the preparation produced by a method of the present disclosure may be a concentrate of antigen-binding molecules having at least one disulfide bond formed between amino acid residues in regions other than the hinge region. Furthermore, in one embodiment, the present disclosure relates to methods for increasing the concentration of antigen-binding molecules having at least one disulfide bond formed between amino acid residues in regions other than the hinge region in a mixture of antigen-binding molecules. In a further embodiment, the present disclosure relates to methods for purifying a preparation comprising antigen-binding molecules having at least one disulfide bond formed between amino acid residues in regions other than the hinge region from a mixture comprising antigen-binding molecules having at least one disulfide bond formed between amino acid residues in regions other than the hinge region and mis-disulfide bonded forms and/or non-disulfide bonded forms of the antigen-binding molecules. In the present disclosure, antigen-binding molecules that do not have a disulfide bond formed between amino acid residues in regions other than the hinge region include those in which cysteine residues in regions other than the hinge region are capped, or exist simply as cysteines.

In methods of the present disclosure, a mixture of antigen-binding molecules is contacted with a solution comprising a reducing agent. In a specific embodiment, a mixture of antigen-binding molecules loaded on a matrix is contacted with a solution comprising a reducing agent in chromatography. In one aspect, examples of the chromatography matrix of the present disclosure include but are not limited to affinity chromatography matrix, ion exchange chromatography matrix, hydrophobic interaction chromatography matrix, matrix for multimodal chromatography comprising both ion exchange chromatography and hydrophobic interaction chromatography, and hydroxyapatite matrix. In the present disclosure, "matrix", "resin", "ligand", and "substrate" can be used interchangeably.

In the present disclosure, the affinity chromatography matrix is not limited as long as it shows affinity chromatographic action. In one aspect, without being limited to the following, protein A matrix, protein G matrix, protein L matrix, sequence-selective peptide matrix, a matrix that binds selectively to antigen-binding molecules, and such can be presented as examples of the affinity chromatography matrix.

In the methods of the present disclosure, commercially available products can be used as the affinity chromatography matrix. In a specific aspect, without being limited to the following,

MabSelect SuRe (registered trademark) (Cytiva Corporation),
MabSelect Xtra (registered trademark) (Cytiva Corporation),
MabSelect SuRe (registered trademark) LX (Cytiva Corporation),
MabSelect SuRe (registered trademark) pcc (Cytiva Corporation),
MabSelect PrismA (registered trademark) (Cytiva Corporation),
MabSpeed (registered trademark) rP202 (Cytiva Corporation),
TOYOPEARL (registered trademark) rProtein A HC-650F (TOSOH Corporation),
Praesto (registered trademark) Jetted A50 (Purolite Corporation),
Amsphere (registered trademark) A3 (JSR Corporation),
Amsphere (registered trademark) Protein A JWT203 (JSR Corporation),
Eshmuno A (registered trademark) (Merck Millipore Corporation),
Capto L (registered trademark) (Cytiva Corporation), and
MabSelect (registered trademark) VL (Cytiva Corporation)
can be presented as examples of the affinity chromatography matrix.

In one aspect, the ion exchange chromatography matrix in the present disclosure includes anion exchange resins (anion exchange ligands) or cation exchange resins (cation exchange ligands). The anion exchange resins and cation exchange resins in the present disclosure are not limited as long as they show anion exchange actions and cation exchange actions, respectively.

In the methods of the present disclosure, commercially available products can be used as the anion exchange chromatography matrix. In a specific aspect, without being limited to the following,

YMC-BioPro (YMC Corporation),
Q Sepharose (registered trademark) High Performance (Cytiva Corporation),

Q Sepharose (registered trademark) Fast Flow (Cytiva Corporation),
Q Sepharose (registered trademark) XL (Cytiva Corporation),
Q Sepharose (registered trademark) Big Beads (Cytiva Corporation),
Capto (registered trademark) Q ImpRes (Cytiva Corporation),
Capto (registered trademark) Q (Cytiva Corporation),
Capto (registered trademark) Q XP (Cytiva Corporation),
Capto (registered trademark) DEAE (Cytiva Corporation),
SOURCE (registered trademark) 30Q (Cytiva Corporation),
SOURCE (registered trademark) 15Q (Cytiva Corporation),
DEAE Sepharose (registered trademark) Fast Flow (Cytiva Corporation),
ANX Sepharose (registered trademark) 4 Fast Flow (Cytiva Corporation),
POROS (registered trademark) 50 PI (Thermo Fisher Corporation),
POROS (registered trademark) 50 HQ (Thermo Fisher Corporation),
POROS (registered trademark) HQ (Thermo Fisher Corporation),
POROS (registered trademark) D (Thermo Fisher Corporation),
POROS (registered trademark) PI (Thermo Fisher Corporation),
Eshumuno (registered trademark) Q (Merck Millipore Corporation),
Fractogel (registered trademark) TMAE (Merck Millipore Corporation),
Fractogel (registered trademark) DEAE (Merck Millipore Corporation),
Macro-Prep (registered trademark) Q (Bio-Rad Laboratories Corporation),
Macro-Prep (registered trademark) DEAE (Bio-Rad Laboratories Corporation),
Giga Cap (registered trademark) Q-650M (TOSOH Corporation),
Giga Cap (registered trademark) DEAE-650M (TOSOH Corporation), and
Q HyperCel (registered trademark) (PALL Corporation)
can be presented as examples of the anion exchange chromatography matrix.

Commercially available products can also be used for cation exchange chromatography matrix. In a specific aspect, without being limited to the following,

Capto (registered trademark) S (Cytiva Corporation),
Capto (registered trademark) SP ImpRes (Cytiva Corporation),
Capto (registered trademark) S ImpaAct (Cytiva Corporation),
SP Sepharose (registered trademark) Fast Flow (Cytiva Corporation),
CM Sepharose (registered trademark) Fast Flow (Cytiva Corporation),
SP Sepharose (registered trademark) High Performance (Cytiva Corporation),
CM Sepharose (registered trademark) High Performance (Cytiva Corporation),
SP Sepharose (registered trademark) XL (Cytiva Corporation),
SP Sepharose (registered trademark) Big Beads (Cytiva Corporation),
Eshumuno (registered trademark) CPX (Merck Millipore Corporation),
Eshumuno (registered trademark) CP-FT (Merck Millipore Corporation),
POROS (registered trademark) 50HS (Thermo Fisher Corporation), and
POROS (registered trademark) XS (Thermo Fisher Corporation)
can be presented as examples of the cation exchange chromatography matrix.

In the present disclosure, the matrix for hydrophobic interaction chromatography is not limited as long as it shows hydrophobic interaction chromatographic action. In one aspect, hydrophobic interaction chromatography matrix includes hydrophobic ligands.

In the methods of the present disclosure, commercially available products can be used as the hydrophobic interaction chromatography matrix. In a specific aspect, without being limited to the following,

Phenyl Sepharose (registered trademark) High Performance (Cytiva Corporation),
Butyl Sepharose (registered trademark) High Performance (Cytiva Corporation),
Phenyl Sepharose (registered trademark) 6 Fast Flow (Cytiva Corporation),
Butyl-S Sepharose (registered trademark) 6 Fast Flow (Cytiva Corporation),
Butyl Sepharose (registered trademark) 4 Fast Flow (Cytiva Corporation),
Octyl Sepharose (registered trademark) 4 Fast Flow (Cytiva Corporation),
Capto (registered trademark) Phenyl ImpRes (Cytiva Corporation),
Capto (registered trademark) Phenyl (Cytiva Corporation),

Capto (registered trademark) Phenyl (High Sub) (Cytiva Corporation),
Capto (registered trademark) Butyl (Cytiva Corporation),
Capto (registered trademark) Butyl ImpRes (Cytiva Corporation),
Capto (registered trademark) Octyl (Cytiva Corporation),
Phenyl Sepharose (registered trademark) 6 Fast Flow (Low Sub) (Cytiva Corporation),
Phenyl Sepharose (registered trademark) 6 Fast Flow (High Sub) (Cytiva Corporation),
POROS (registered trademark) Ethyl (Thermo Fisher Corporation),
Fractogel (registered trademark) Phenyl (Merck Millipore Corporation),
Fractogel (registered trademark) Propyl (Merck Millipore Corporation),
TOYOPEARL (registered trademark) Butyl (TOSOH Corporation),
TOYOPEARL (registered trademark) Ether (TOSOH Corporation),
TOYOPEARL (registered trademark) Hexyl (TOSOH Corporation),
TOYOPEARL (registered trademark) Phenyl (TOSOH Corporation),
TOYOPEARL (registered trademark) PPG (TOSOH Corporation),
TOYOPEARL (registered trademark) Super Butyl (TOSOH Corporation),
TOYOPEARL (registered trademark) Butyl-600 (TOSOH Corporation),
TOYOPEARL (registered trademark) Phenyl-650C (TOSOH Corporation),
TOYOPEARL (registered trademark) Phenyl-650M (TOSOH Corporation),
TOYOPEARL (registered trademark) Phenyl-650S (TOSOH Corporation),
TOYOPEARL (registered trademark) Phenyl-600M (TOSOH Corporation), and
Macro-Prep (registered trademark) HIC (Bio-Rad Laboratories Corporation)
can be presented as examples of the hydrophobic interaction chromatography matrix.

In one aspect, multimodal chromatography matrix of the present disclosure can include a matrix that combines cation exchange ligands and hydrophobic ligands, or a matrix that combines anion exchange ligands and hydrophobic ligands. In the methods of the present disclosure, commercially available products can be used for the multimodal chromatography matrix. For example, without being limited to the following,

Capto (registered trademark) adhere (Cytiva Corporation),
Capto (registered trademark) adhere ImpRes (Cytiva Corporation),
Capto (registered trademark) MMC (Cytiva Corporation),
Capto (registered trademark) MMC ImpRes (Cytiva Corporation), and
Eshmuno (registered trademark) CMX (Merck Millipore Corporation)
can be presented as examples of the matrix for multimodal chromatography.

In one aspect, the hydroxyapatite chromatography matrix can include hydroxyapatite or its derivative (such as fluoroapatite). In the methods of the present disclosure, commercially available products can also be used for the hydroxyapatite chromatography matrix. In a specific embodiment, without being limited to the following,

Ceramic Hydroxyapatite (registered trademark) Type I (Bio-Rad Laboratories Corporation),
Ceramic Hydroxyapatite (registered trademark) Type II (Bio-Rad Laboratories Corporation),
Ceramic Fluoroapatite (registered trademark) (Bio-Rad Laboratories Corporation),
MPC (registered trademark) Ceramic Hydroxyfluoroapatite (Bio-Rad Laboratories Corporation),
HA Ultrogel (registered trademark) (PALL Corporation), and
Ca++Pure-HA (registered trademark) (TOSOH Corporation)
can be presented as examples of the matrix for hydroxyapatite chromatography.

In the methods of the present disclosure, commercially available products can also be used for membrane chromatography. In a specific embodiment, without being limited to the following,

Mustang (registered trademark) Q (PALL Corporation),
Mustang (registered trademark) S (PALL Corporation),
Sartobind (registered trademark) Q (Sartorius Corporation),
Sartobind (registered trademark) S (Sartorius Corporation),
Sartobind (registered trademark) STIC (Sartorius Corporation),
Sartobind (registered trademark) protein A (Sartorius Corporation),
Fibro (Cytiva Corporation),
Protein capture (GORE Corporation),

Natrix (registered trademark) Q (Merck Millipore Corporation),
Purexa (registered trademark) -MQ (Purilogics Corporation),
Purexa (registered trademark) -A (Purilogics Corporation), and
Purexa (registered trademark) -DMAE (Purilogics Corporation)
can be presented as examples of products for membrane chromatography.

In the present disclosure, the terms "reduction reagent", "reducing agent", and "solution comprising a reducing agent" are used interchangeably. In some embodiments, the reducing agents described above are free thiols. Furthermore, in one aspect, the reducing agent in the present disclosure can be a monothiol, a dithiol, a phosphine, or an inorganic reagent. Examples of the monothiol in the present disclosure include glutathione, cysteine, lipoic acid, 2-mercaptoethanol, and 2-MEA, but are not limited thereto. Examples of the dithiol in the present disclosure include dithiothreitol (DTT), DTE, and DTBA, but are not limited thereto. Examples of the phosphine in the present disclosure include tris(2-carboxyethyl) phosphine (TCEP) and THPP, but are not limited thereto. Examples of the inorganic reagent in the present disclosure include sodium sulfite and sodium pyrosulfite, but are not limited thereto.

The reduction reagents are preferably composed of compounds selected from the group consisting of glutathione (GSH), dithiothreitol (DTT), 2-mercaptoethanol, 2-aminoethanethiol (2-MEA), tris(2-carboxyethyl)phosphine (TCEP), dithionitrobenzoate, cysteine, sodium sulfite, sodium pyrosulfite, and $Na_2SO_3$. In some embodiments, TCEP, 2-MEA, DTT, cysteine, GSH, or $Na_2SO_3$ can be used. In some preferred embodiments, cysteine can be used. In some preferred embodiments, TCEP can be used. In the methods of the present disclosure, one or more types (for example, 2, 3, or more types) of reducing agents can be used.

In one embodiment, the reducing agent can be a solution, or preferably in the form of a buffer. Examples of the buffer include buffers used in chromatography techniques such as phosphate buffer, Tris buffer, acetate buffer, citrate buffer, tartrate buffer, and borate buffer, but are not limited thereto. Furthermore, examples of a base include sodium hydroxide, potassium hydroxide, lithium hydroxide, and Tris.

In the present disclosure, "contacting" means subjecting or exposing a mixture of antigen-binding molecules to a solution comprising a reducing agent, or mixing a mixture of antigen-binding molecules with a solution comprising a reducing agent. In the methods of the present disclosure, a mixture of antigen-binding molecules can be contacted with a solution comprising a reducing agent while the molecules are in a state bound to a solid matrix (for example, a column chromatography column or a membrane chromatography membrane).

While not being bound by the following theory, it is considered that the presence of unLINC formats (i.e., divalent or trivalent antigen-binding molecules that do not have engineered disulfide bonds or "paired cysteines") could be due to the unpaired Cys residues often forming disulfide bonds with molecules that contain free thiol groups, such as cysteinylation and glutathionylation which prevent LINC formation (formation of engineered disulfide bonds in regions other than the hinge region) by "capping" the unpaired Cys residues. For removal of molecules with unpaired capped cysteines, reducing agents can help de-capping of surface cysteines, and further re-oxidation of the de-capped antigen-binding molecules (for example, by removal of reduction reagents) can promote disulfide bond formation between the de-capped cysteines for LINC formation. Therefore, removal of cysteinylation from the unpaired sulfhydryl in the unLINC format by reduction and re-oxidation can remove the unLINC formats and improve homogeneity of the antibodies.

In one aspect, in the methods of the present disclosure, contacting a mixture of antigen-binding molecules with a solution comprising a reducing agent causes cleavage of disulfide bonds formed between amino acid residues that may form disulfide bonds in the antigen-binding molecules. In one aspect, contacting a mixture of antigen-binding molecules with a solution comprising a reducing agent causes de-capping of capped cysteine residues in the antigen-binding molecules.

In one aspect, in the methods of the present disclosure, removal of the reducing agent causes formation of disulfide bonds between amino acid residues that may form disulfide bonds in the antigen-binding molecules.

Contacting the mixture of antigen-binding molecules with a solution comprising a reducing agent is performed for a time sufficient to increase the relative proportion of the desired conformations. Any relative increase in proportion is desirable, including for example, conversion of at least 10%, 20%, 30%, 40%, 50%, 60%, 70%, 80%, 90%, 95%, 96%, 97%, 98%, or 99% of the antigen-binding molecules having undesirable conformation to antigen-binding molecules having the desired conformation.

In one aspect, in the methods of the present disclosure, a solution comprising a reducing agent is allowed to flow through a chromatographic system containing a matrix carrying the antigen-binding molecules. In a specific embodiment, the antigen-binding molecules constitute the stationary phase immobilized to the chromatography matrix, and the solution comprising a reducing agent is a part of the mobile phase. In this case, the contacting may be performed as a part of a chromatographic purification procedure.

The reducing agent is present at a concentration that is sufficient to increase the relative proportion of the desired conformation (for example, an antigen-binding molecule in the "paired cysteines" form which has one or more engineered disulfide bonds between the two Fabs of the antibody, for example, between regions other than the hinge region). The optimal absolute concentration and molar ratio of the reducing agent depends on the total concentration of antigen-binding molecules and in some circumstances the concentration of specific antigen-binding molecules. Furthermore, the reducing agent concentration also depends on the number and accessibility of the unpaired cysteines in the antigen-binding molecules.

In one aspect, examples of the concentration of the reducing agent include ranges that can be specified by any combination of a lower limit selected from the group consisting of about 0.0001 mM, about 0.0005 mM, about 0.001 mM, about 0.005 mM, about 0.01 mM, about 0.05 mM, about 0.1 mM, about 0.5 mM, and about 1.0 mM, and an upper limit selected from the group consisting of about 100.0 mM, about 75.0 mM, about 50.0 mM, about 25.0 mM, about 10.0 mM, about 5.0 mM, about 1.0 mM, about 0.5 mM, about 0.1 mM, and about 0.01 mM. In some embodiments, as the concentration of the reducing agent, a range of about 0.00001 mM to about 10.0 mM, about 0.00005 mM to about 5.0 mM, about 0.0001 mM to about 1.0 mM, about 0.0005 mM to about 0.5 mM, about 0.001 mM to about 0.1 mM, or about 0.001 mM to about 0.01 mM can be presented as an example. In further embodiments, as the concentration of the reducing agent, a range of about 0.001 mM to about 100.0 mM, about 0.005 mM to about 75.0 mM, about 0.01 mM to about 50.0 mM, about 0.05 mM to about 25.0 mM, or about 0.1 mM to about 10 mM can be presented as an example. In further embodiments, as the concentration of the reducing agent, a range of about 0.0001 mM to about 100.0 mM, about 0.0005 mM to about 50.0 mM, about 0.001 mM to about 10.0mM, about 0.005 mM to about 5.0 mM, about 0.01 mM to about 1.0 mM can be presented as an example. In a specific embodiment, the concentration of the reducing agent can be about 0.001 mM, about 0.01 mM, about 0.1 mM, about 0.15 mM, about 1.0 mM, or about 10.0 mM.

In some preferred embodiments, when the reducing agent is cysteine, the concentration of the reducing agent can be about 0.01 mM to about 100.0 mM, about 0.05 mM to about 50.0 mM, about 0.1 mM to about 10.0 mM, or about 0.5 mM to about 5.0 mM. In further preferred embodiments, when the reducing agent is cysteine, the concentration of the reducing agent can be about 0.001 mM to about 100.0 mM, about 0.005 mM to about 75.0 mM, about 0.01 mM to about 50.0 mM, about 0.05 mM to about 25.0 mM, or about 0.1 mM to about 10 mM. In further preferred embodiments, when the reducing agent is cysteine, the concentration of the reducing agent can be about 0.0001 mM to about 100.0 mM, about 0.001 mM to about 75.0 mM, about 0.01 mM to about 50.0 mM, or about 0.1 mM to about 10.0 mM. In some preferred embodiments, when the reducing agent is a dithiol, the concentration of the reducing agent can be about 0.001 mM to about 10 mM. In some preferred embodiments, when the reducing agent is a phosphine, the concentration of the reducing agent can be about 0.0001 mM to about 1 mM or about 0.001 mM to about 0.01 mM. In some preferred embodiments, when the reducing agent is TCEP, the concentration of the reducing agent can be about 0.00001 mM to about 10.0 mM, about 0.00005 mM to about 5.0 mM, about 0.0001 mM to about 1 mM, about 0.0005 mM to about 0.5 mM, about 0.001 mM to about 0.1 mM, or about 0.001 mM to about 0.01 mM. In some preferred embodiments, when the reducing agent is an inorganic reagent, the concentration of the reducing agent can be about 0.0001 mM to about 10 mM or about 0.001 mM to about 0.1 mM.

In a specific embodiment, when the reducing agent is cysteine, the concentration of the reducing agent can be about 0.1 mM, about 0.15 mM, about 1.0 mM, about 10.0 mM, or about 100 mM. In a specific embodiment, when the reducing agent is TCEP, the concentration of the reducing agent can be about 0.001 mM, about 0.01 mM, about 0.1 mM, or about 1.0 mM.

In order to maximize the yield of an antigen-binding molecule with a desired conformation, the pH of a solution comprising a reducing agent is selected so as to protect the stability of the antigen-binding molecule and to be optimal for disulfide exchange. In one aspect, the pH of the solution comprising the reducing agent of the present disclosure can be about 4.5 to about 10.0, about 5.0 to about 9.0, about 6.5 to about 8.5, or about 7.0 to about 8.0. In a nonlimiting embodiment of the present invention, the optimal pH was found to be about 7.0, about 7.5, or about 8.0. However, the optimal pH in a particular embodiment of the present invention can be easily determined experimentally by those skilled in the art.

In the methods of the present disclosure, contacting of the chromatography matrix, the antigen-binding molecules, and the reducing agent takes place in chromatography. In one embodiment, in the methods of the present disclosure, contacting between the antigen-binding molecules loaded on a matrix and the reducing agent takes place in chromatography. In other words, in the methods of the present disclosure, contacting between the reducing agent and the antigen-binding molecules can be performed by allowing the solution comprising the reducing agent to flow through a column chromatography column or a membrane chromatography device that contains the antigen-binding molecules loaded onto a matrix.

In some embodiments, the solution comprising the reducing agent is allowed to flow through the column chromatography column or the membrane chromatography device with a residence time of about 2 seconds to about 80 minutes or about 3 seconds to about 24 minutes. In a specific embodiment, the solution comprising the reducing agent is allowed to flow through the column chromatography column or the membrane chromatography device with a residence time of about 12 minutes or about 30 seconds. In some embodiments, the flow can be stopped temporarily while in a state where the column interior or the device interior is filled with the solution comprising the reducing agent.

In one aspect, when column chromatography is used, the solution comprising the reducing agent is allowed to flow through the column with a residence time of about 2 minutes to about 80 minutes or about 4 minutes to about 24 minutes. In a particular embodiment, when column chromatography is used, the solution comprising the reducing agent is allowed to flow through the column with a residence time of about 12 minutes. In one aspect, the flow can be stopped temporarily while in a state where the column interior is filled with the solution comprising the reducing agent. In one aspect, when column chromatography is used, the solution comprising the reducing agent of the present disclosure can be allowed to flow through the column at a rate of about 25 cm/hour to about 500 cm/hour, about 50 cm/hour to about 400 cm/hour, about 75 cm/hour to about 350 cm/hour, or about 100 cm/hour to about 300 cm/hour. In some embodiments, the solution comprising the reducing agent of the present disclosure can be allowed to flow through the column at a rate of about 100 cm/hour. In one aspect, the flow can be stopped temporarily while in a state where the column interior is filled with the solution comprising the reducing agent.

In one aspect, when membrane chromatography is used, the solution comprising the reducing agent is allowed to flow through the membrane device with a residence time of about 2 seconds to about 60 minutes or about 3 seconds to about 6 minutes, for example, about 30 seconds. In some embodiments, the flow can be stopped temporarily while in a state where the membrane device interior is filled with the solution comprising the reducing agent. In one aspect, when membrane chromatography is used, the solution comprising the reducing agent in the present disclosure can be allowed to flow through a membrane device at a rate of about 0.017 MV/minute to about 30 MV/minute, about 0.17 MV/minute to about 20 MV/minute, about 1 MV/minute to about 10 MV/ minute, or about 2 MV/minute to about 5 MV/minute. In a specific embodiment, the solution comprising the reducing agent in the present disclosure can be allowed to flow through a membrane device at a rate of about 2 MV/minute. The flow can be stopped temporarily while in a state where the membrane device interior is filled with the solution.

The amount of the solution comprising the reducing agent allowed to flow through the column is not particularly limited, but for example, when column chromatography is used, the flow volume of the solution comprising the reducing agent can be about 0.1-times (about 0.1 column volume (CV)) to about 100-times (about 100 CV), about 1-time (about 1 CV) to about 20-times (about 20 CV), or about 3-times (about 3 CV) to about 10-times (about 10 CV) the column volume. In some embodiments, the amount of the solution comprising the reducing agent allowed to flow through the column can be about 10-times the column volume (about 10 CV). Furthermore, when membrane chromatography is used, the flow volume of the solution comprising the reducing agent can be about 1-time (about 1 membrane volume (MV)) to about 500-times (about 500 MV), or about 2-times (about 2 MV) to about 100-times (about 100 MV) the membrane device volume. In some embodiments, the amount of the solution comprising the reducing agent allowed to flow through the column can be about 15 times the column volume (about 15 MV).

The reaction time (contacting time) between the mixture of antigen-binding molecules and the solution comprising the reducing agent is not particularly limited, but examples include about 6 seconds to about 1440 minutes, about 18 seconds to about 300 minutes, about 5 minutes to about 180 minutes, about 10 minutes to about 150 minutes, or about 12 minutes to about 120 minutes. In a particular embodiment, the contacting time between the mixture of antigen-binding molecules and the solution comprising the reducing agent can be about 120 minutes or about 7.5 minutes.

In one aspect, when column chromatography is used, the contacting time between the mixture of antigen-binding molecules and the solution comprising the reducing agent can be about 4 minutes to about 1440 minutes, about 8 minutes to about 300 minutes, about 30 minutes to about 240 minutes, about 60 minutes to about 180 minutes, or about 120 minutes. In a specific embodiment, the contacting time between the mixture of antigen-binding molecules and the solution comprising the reducing agent can be about 120 minutes.

In one aspect, when membrane chromatography is used, the contacting time between the mixture of antigen-binding molecules and the solution comprising the reducing agent can be about 6 seconds to about 600 minutes or about 18 seconds to about 120 minutes, for example, about 7.5 minutes.

In the methods of the present disclosure, the antigen-binding molecules can be contacted with the reducing agent in various volumes as appropriate, such as at the analytical experimental scale (1 mL to 50 mL), preparative scale (50 mL to 10 L), and manufacturing scale (10 L or more). In one aspect, the quantity of antigen-binding molecules loaded onto a substrate can be about 5 to 100 g, about 7 to 70 g, about 10 to 40 g, for example, about 10 g or about 25g, per 1 L of the chromatography matrix containing the substrate.

In one aspect, when column chromatography is used, the quantity of antigen-binding molecules loaded onto a substrate can be about 5 g to about 80 g, about 7 g to about 70 g, or about 10 g to about 40 g, for example, about 10 g, per 1 L of the chromatography matrix containing the substrate.

In one aspect, when membrane chromatography is used, the quantity of antigen-binding molecules loaded onto a membrane can be about 5 g to about 100 g, about 7 g to about 50 g, or about 10 g to about 40 g, for example, about 25 g, per 1 L of membrane device volume.

The methods of the present disclosure comprise a step of removing the reducing agent. In the present disclosure, removing the reducing agent includes completely removing the reducing agent, decreasing the concentration of the reducing agent, and chemically inactivating the reducing agent. Removal of the reducing agent promotes reoxidation of disulfide bonds in regions other than the hinge region between two Fabs constituting the antigen-binding molecule.

In one aspect, the reducing agent can be removed by contacting the antigen-binding molecule with a solution not comprising a reducing agent. In one aspect, the reducing agent can be removed by allowing a solution not comprising a reducing agent to flow through a column chromatography column or through a membrane chromatography device.

In some embodiments, the solution not comprising a reducing agent is allowed to flow through a column chromatography column or through a membrane chromatography device with a residence time of about 2 seconds to about 80 minutes or about 3 seconds to about 24 minutes. In a specific embodiment, the solution not comprising a reducing agent is allowed to flow through a column chromatography column or through a membrane chromatography device with a residence time of about 4 minutes. In some embodiments, the flow can be stopped temporarily while in a state where the column interior or the device interior is filled with the solution.

In some embodiments, when column chromatography is used, the solution not comprising a reducing agent is allowed to flow through the column with a residence time of about 2 minutes to about 80 minutes or about 4 minutes to about 24 minutes. In a particular embodiment, when column chromatography is used, the solution not comprising a reducing agent is allowed to flow through the column with a residence time of about 4 minutes. In some embodiments, the flow can be stopped temporarily while in a state where the column interior is filled with the solution.

In some embodiments, when column chromatography is used, the solution not comprising a reducing agent can be allowed to flow through the column at a rate of about 25 cm/hour to about 500 cm/hour, about 50 cm/hour to about 450 cm/hour, about 200 cm/hour to about 400 cm/hour, or about 250 cm/hour to about 350 cm/hour. Furthermore, in a specific embodiment, the solution not comprising a reducing agent of the present disclosure can be allowed to flow through the column at a rate of about 300 cm/hour. The flow can be stopped temporarily while in a state where the column interior is filled with the solution.

In some embodiments, when membrane chromatography is used, the solution not comprising a reducing agent is allowed to flow through the membrane device with a residence time of about 2 seconds to about 60 minutes or about 3 seconds to about 6 minutes, for example, about 30 seconds. In some embodiments, the flow can be stopped temporarily while in a state where the device interior is filled with the solution.

In some embodiments, when membrane chromatography is used, the solution not comprising a reducing agent can be allowed to flow through the membrane device at a rate of about 0.017 MV/minute to about 30 MV/minute, about 0.17 MV/minute to about 20 MV/minute, about 1 MV/minute to about 10 MV/minute, or about 2 MV/minute to about 5 MV/minute. Furthermore, in a specific embodiment, the solution not comprising a reducing agent of the present disclosure can be allowed to flow through the membrane device at a rate of about 2 MV/minute. The flow can be stopped temporarily while in a state where the membrane device interior is filled with the solution.

The solution not comprising a reducing agent is not particularly limited, but in one example, buffers may be used, and more specifically, buffers ordinarily used in chromatography techniques such as phosphate buffer, Tris buffer, acetate buffer, citrate buffer, tartrate buffer, and borate buffer may be used. Furthermore, examples of a base include sodium hydroxide, potassium hydroxide, lithium hydroxide, and Tris. In one aspect, in the methods of the present disclosure, for the solution not comprising a reducing agent, the same type of solution as the solution comprising the reducing agent used in the contacting step may be used, except that the reducing agent is absent.

In one aspect, when column chromatography is used, the flow volume of the solution not comprising a reducing agent can be about 0.1-times (about 0.1 CV) to about 100-times (about 100 CV), about 1-time (about 1 CV) to about 20-times (about 20 CV), or about 3-times (about 3 CV) to about 10-times (about 10 CV) the column volume. In some embodiments, the flow volume of the solution not comprising a reducing agent can be about 5.0-times the column volume (about 5.0 CV).

In one aspect, when membrane chromatography is used, the flow volume of the solution not comprising a reducing agent can be about 1-time (1 MV) to about 500-times (500 MV), or about 2-times (2 MV) to about 100-times (100 MV) the membrane device volume, for example, about 15 times the membrane device volume (15 MV).

In one aspect, the reaction time (contacting time) between the mixture of antigen-binding molecules and the solution not comprising a reducing agent can be about 6 seconds to about 1440 minutes, about 18 seconds to about 300 minutes, about 5 minutes to about 60 minutes, about 10 minutes to about 50 minutes, or about 12 minutes to about 40 minutes. In a particular embodiment, the reaction time (contacting time) between the mixture of antigen-binding molecules and the solution not comprising a reducing agent can be about 20 minutes.

In one aspect, when column chromatography is used, the reaction time (contacting time) between the mixture of antigen-binding molecules and the solution not comprising a reducing agent can be about 4 minutes to about 1440 minutes or about 8 minutes to about 300 minutes. In a particular embodiment, when column chromatography is used, the reaction time (contacting time) between the mixture of antigen-binding molecules and the solution not comprising a reducing agent can be about 20 minutes.

In one aspect, when membrane chromatography is used, the reaction time (contacting time) between the mixture of antigen-binding molecules and the solution not comprising a reducing agent can be about 6 seconds to about 600 minutes or about 18 seconds to about 120 minutes, for example, about 7.5 minutes.

The methods of the present invention can be performed over a wide temperature range. For example, the methods of the present invention can be carried out at about 4°C to about 37°C or about 15°C to about 37°C. A typical temperature for contacting a partially or fully purified preparation of antigen-binding molecules is about 4°C to about 25°C (ambient temperature), or preferably at 23°C, but this contacting can also be performed at lower temperatures and at higher temperatures. In some embodiments, the methods of the present disclosure can be performed at temperatures from about 20°C to 37°C, preferably at 23°C, 25°C, or 37°C, and more preferably at 23°C.

Also, for the solution not comprising a reducing agent, the optimal pH can be easily determined experimentally by those skilled in the art. The examples can be about 4.5 to about 10.0, about 5.0 to about 9.0, about 6.5 to about 8.5, or about 7.0 to about 8.0. Furthermore, in a specific embodiment, the optimal pH can be about 7.1, about 7.2, about 7.3, about 7.4, about 7.5, about 7.6, about 7.7, about 7.8, or about 7.9.

In one aspect, in the methods of the present disclosure, prior to the step of contacting the mixture of antigen-binding molecules with the solution comprising the reducing agent, a step of contacting the mixture with a chromatography matrix to fill the column interior with the antigen-binding molecules or to immobilize the antigen-binding molecules onto the membrane can be included. In a specific embodiment, when the mixture is a cell culture solution comprising the antigen-binding molecules (harvested cell culture fluid: HCCF), a step of contacting the cell culture solution with a chromatography matrix to fill the column interior with the antigen-binding molecules or to immobilize the antigen-binding molecules onto the membrane can be included prior to the step of contacting the cell culture solution with the solution comprising the reducing agent. In one aspect, the mixture of antigen-binding molecules comprises antigen-binding molecules having at least one disulfide bond in regions other than the hinge region (i.e., LINC format) and antigen-binding molecules not having a disulfide bond in regions other than the hinge region (i.e., unLINC format).

In certain aspects, the antigen-binding molecule may be at least partially purified before the step of contacting with the solution comprising the reducing agent. In some embodiments, the production methods in the present disclosure can comprise before the contacting step, a step of subjecting the cell culture solution comprising the antigen-binding molecules to affinity chromatography (preferably protein A chromatography).

In one aspect, the methods of the present disclosure can comprise, prior to the contacting step, a step of removing impurities in the chromatography. As described in the Examples as one example, impurities can be removed by methods well known to those skilled in the art, such as allowing a buffer to flow through a column carrying a substrate to which the antigen-binding molecules are adsorbed.

In one aspect, the methods of the present disclosure can further comprise before or after the step of removing the reducing agent, a step of collecting or eluting antigen-binding molecules having at least one disulfide bond in a region other than the hinge region, and/or a step of purifying the antigen-binding molecules. In one embodiment, collection and elution of antigen-binding molecules can be performed by eluting the antigen-binding molecules from a chromatography column by methods well known to those skilled in the art, such as using an elution buffer. Those skilled in the art can adjust the pH and composition of the elution buffer appropriately according to the properties of the antigen-binding molecules to be purified.

In one embodiment, preparations of antigen-binding molecules produced as described herein can be further purified by techniques known in this technical field such as high-performance liquid chromatography, ion exchange chromatography, gel electrophoresis, affinity chromatography, and size exclusion chromatography. The actual conditions used to purify a particular antigen-binding molecule partially depend on factors such as net charge, hydrophobicity, and hydrophilicity, which are apparent to those skilled in the art. For affinity chromatography purification, an antibody, ligand, receptor, or antigen can be used to which the antigen-binding molecule binds. For example, for affinity chromatography purification of antigen-binding molecules, a matrix with protein A or protein G may be used. Sequential Protein A or G affinity chromatography and size exclusion chromatography can be used to isolate antigen-binding molecules. The purity of the antigen-binding molecules can be determined by any of a variety of well known analytical methods including gel electrophoresis and highpressure liquid chromatography.

In certain embodiments, the antigen-binding molecule having at least one disulfide bond in regions other than a hinge region as produced or purified by the method described herein are further processed in a separate processing step which employs chaotropic denaturants such as, for example, sodium dodecyl sulfate (SDS), urea or guanidium hydrochloride (GuHCl). Significant amounts of chaotropic agents are needed to observe perceptible unfolding. In some embodiments the processing step uses between 0.1M and 2 M chaotrope that produces an effect equivalent to the use of 0.1 M to 2M guanidine hydrochloride. In a specific embodiment, the oxidative refolding is achieved in the presence of approximately 1.0 M guanidine hydrochloride or an amount of other chaotropic agent that produces the same or similar amount of refolding as 1M guanidine hydrochloride. In some embodiments, the methods use between about 1.5 M and 0.5 M chaotrope. The amount of chaotropic agent used is based on the structural stability of the antigen-binding molecule in the presence of the said chaotrope. One needs to have enough chaotrope present to perturb the local tertiary structure and/or quaternary structure of domain interactions of the antigen-binding molecule, but less than that required to fully unfold secondary structure of the molecule and/or individual domains. To determine the point at which an antigen-binding molecule will start to unfold by equilibrium denaturation, one practiced in the art would titrate a chaotrope into a solution containing the antigen-binding molecule and monitor structure by a technique such as circular dichroism or fluorescence.

There are other parameters that could be used to unfold or slightly perturb the structure of an antigen-binding molecule that may be used instead of a chaotrope. Temperature and pressure are two fundamental parameters that have been previously used to alter the structure of an antigen-binding molecule and may be used in place of a chaotropic agent while contacting with an oxidizing agent and/or a reducing agent. The inventors contemplate that any parameter that has been shown to denature or perturb the structure of an antigen-binding molecule may be used by a person practiced in the art in place of a chaotropic agent.

The methods of the present disclosure increase the "LINC ratio", that is, the proportion of antigen-binding molecules in the LINC format relative to the antigen-binding molecules loaded on a matrix (i.e., the total of antigen-binding molecules having the LINC format, which have at least one disulfide bond in regions other than the hinge region, and antigen-binding molecules having the unLINC format). For example, as described in WO2021/157679, the LINC ratio can be calculated by separating the crosslinked LINC format from the non-crosslinked unLINC format (open-type) by non-reducing SDS-PAGE and comparing the respective abundance ratios.

In one embodiment, a preparation of antigen-binding molecules produced as described herein can be additionally subjected to techniques such as electrophoresis and chromatography to determine the LINC ratio. More specifically, following the step of purification by chromatography, the methods of the present disclosure may comprise a step of quantifying the LINC ratio in the preparation obtained from the purification step, that is, the proportion of antigen-binding molecules having at least one disulfide bond in regions other than the hinge region (LINC form) relative to the total of (i) antigen-binding molecules having at least one disulfide bond formed between amino acid residues in regions other than the hinge region (LINC form) and (ii) mis-disulfide bonded forms and/or non-disulfide bonded forms of the antigen-binding molecules (unLINC form). In a certain embodiment, the methods of the present disclosure may comprise a step of quantifying the proportion of antigen-binding molecules having at least one disulfide bond in regions other than the hinge region (LINC form) relative to the total of (i) antigen-binding molecules having at least one disulfide bond formed between amino acid residues in regions other than the hinge region (LINC form) and (ii) non-disulfide bonded forms of the antigen-binding molecules.

In one embodiment, the step of quantifying the LINC ratio includes electrophoresis, chromatography, or such. In one embodiment, non-reducing SDS-polyacrylamide gel electrophoresis (SDS-PAGE), non-reducing capillary SDS gel electrophoresis (CE-SDS), and such can be presented as examples of electrophoresis. Furthermore, in one embodiment, hydrophobic interaction chromatography (HIC) and such can be presented as examples of chromatography.

In one embodiment, prior to the step of quantifying the LINC ratio, methods of the present disclosure may comprise a step of adding a protease to a preparation of antigen-binding molecules produced as described herein. In one embodiment, the protease of the present disclosure may be, for example, IgdE, or preferably IgdE derived from *Streptococcus agalactiae.* IgdE is a protease that recognizes the upper part of the hinge region of the human IgG1 antibody (KSCDKT/HTCPPCP), and is an enzyme that digests the antibody between T and H. Such an enzyme can degrade the antibody to generate Fab and Fc regions. *Streptococcus agalactiae*-derived IgdE is an enzyme disclosed in the literature such as Spoerry C et al., (2016) PLoS ONE 11(10): e0164809. doi:10.1371/journal.pone.0164809, and can be easily obtained by those skilled in the art using well-known techniques. Furthermore, *Streptococcus agalactiae*-derived IgdE is commercially available, for example, from Genovis Inc. as "FabALACTICA™(IgdE)". The present inventors added *Streptococcus agalactiae*-derived IgdE to a preparation of antigen-binding molecules produced as described herein, and discovered that the LINC ratio of the preparation can be determined by subjecting the preparation containing IgdE to electrophoresis, chromatography, or such.

The concentration of the protease added to the preparation is not particularly limited, but in one embodiment, examples include 0.6 to 1.8 Units-enzyme/$\mu$g-protein.

In certain embodiments, when the method of the present invention comprises a step of adding a protease to a preparation of antigen-binding molecules produced as described herein, non-reducing capillary SDS gel electrophoresis (CE-SDS) or hydrophobic interaction chromatography (HIC) can be used in the step of quantifying the LINC ratio. Furthermore, in certain embodiments, chromatography for obtaining a preparation of antigen-binding molecules may be membrane chromatography.

In one embodiment, in the method of the present disclosure, LINC ratio can be calculated as follows. For example, when non-reducing capillary SDS gel electrophoresis (CE-SDS) is used in the quantification step, electropherograms are prepared for a preparation with protease addition, a preparation without protease addition, and a sample containing the protease alone. In this case, the LINC ratio is determined as the ratio of the LINC form-derived peak area to the value resulting from subtracting "the peak area derived from the preparation without protease addition which does not include the unLINC form-derived peak" and "the peak area derived from the sample containing the protease alone" from "the peak area derived from the preparation with protease addition which includes the unLINC form-derived and protease-derived peaks" and then adding the "LINC form-derived peak area". The sample containing the protease alone is preferably a sample containing the same components as the preparation with protease addition, except that the sample does not contain the preparation of antigen-binding molecules obtained by the method of the present disclosure.

In a different embodiment, in the methods of the present disclosure, the LINC ratio can be calculated as follows.

For example, when hydrophobic interaction chromatography (HIC) is used in the quantification step, the LINC ratio is determined from the proportion of the peak derived from the LINC form relative to the sum of the peak derived from the LINC form and the peaks derived from all of the unLINC forms. Mobile phase A and Mobile phase B in the hydrophobic interaction chromatography (HIC) can be prepared appropriately based on common technical knowledge by those skilled in the art. Furthermore, a chromatographic matrix can be appropriately selected by those skilled in the art, and for example, those described herein may be used.

In one embodiment, the methods of the present disclosure may further comprise a step of culturing the preparation after protease addition. Culturing conditions are not particularly limited, but incubation can be carried out, for example, in a phosphate buffer at pH 6 to 8, for example at 37°C for 16 to 20 hours, such as 18 hours.

In addition, in the present disclosure, the antigen-binding molecules subjected to chromatography for purification may comprise a signal sequence. In particular, when antigen-binding molecules having amino acid residues that may form at least one disulfide bond between amino acid residues in regions other than the hinge region are recombinantly expressed in cells, in some cases, to facilitate expression and/or secretion, the antigen-binding molecules may have a signal sequence. In one embodiment, when a translation product from the nucleic acid sequence for recombinant expression of the antigen-binding molecule comprises a signal sequence, the method of the present disclosure can comprise a step of cleaving the signal sequence from the antigen-binding molecules. The signal sequence can be cleaved before or after the step of subjecting the antigen-binding molecules to chromatography for purification, or before or after the step of quantifying the LINC ratio of the substance purified by chromatography. Cleavage of the signal sequence can be carried out by methods well-known to those skilled in the art.

In one aspect, the amino acid residues that may form disulfide bonds are mutated, substituted, introduced, or engineered cysteine residues.

In one aspect, the at least one disulfide bond in regions other than the hinge region is formed between polypeptides constituting an antigen-binding molecule. More specifically, in a specific embodiment, the at least one disulfide bond in regions other than the hinge region in the present disclosure is an interchain disulfide bond. In some embodiments, the at least one disulfide bond in regions other than the hinge region is 1, 2, 3, 4, 5, or more interchain disulfide bonds.

In one aspect, the at least one disulfide bond in regions other than the hinge region is an engineered disulfide bond not present in wild-type IgG.

III. Antigen-binding molecule

In one aspect, an antigen-binding molecule in the present disclosure comprises a first antigen-binding domain and a second antigen-binding domain which can be linked to each other via at least one disulfide bond. In one aspect, in an antigen-binding molecule produced or purified by a method of the present disclosure, the first antigen-binding domain and the second antigen-binding domain are linked via at least one disulfide bond. In an embodiment of the above aspects, the first antigen-binding domain and the second antigen-binding domain are linked via two, three, four, or more disulfide bonds.

In an embodiment, at least one of the first and second antigen-binding domains has, by itself, activity of binding to an antigen (i.e., a single antigen-binding domain independently has antigen-binding activity). In certain embodiments, each of the first and second antigen-binding domains has, by itself, activity of binding to an antigen.

In one aspect, each of the first antigen-binding domain and the second antigen-binding domain in the present disclosure may have a Fab, Fab', scFab, Fv, scFv, or VHH structure.

In a certain aspect, each of the first antigen-binding domain and the second antigen-binding domain is a Fab molecule, and the antigen-binding molecule comprises at least one disulfide bond formed between the first antigen-binding domain and the second antigen-binding domain. Preferably, the at least one disulfide bond is formed between amino acid residues (cysteine) that are not present within the hinge region, or preferably between amino acid residues (cysteine) within the CH1 region of each antigen-binding domain.

In an embodiment of the above aspects, both the first and second antigen-binding domains comprise a Fab and a hinge region.

In certain embodiments, at least one of the amino acid residues that form a disulfide bond between the antigen-binding domains is a mutated amino acid residue which is not present in a wild-type Fab or hinge region, and for example, it is a cysteine residue which is not present in a wild-type Fab or hinge region. Such a mutated amino acid residue can be introduced into a wild-type Fab or hinge region by, for example, a method of amino acid substitution.

Alternatively, in another embodiment, in an antigen-binding molecule in the present disclosure, an amino acid residue that is present in a wild-type Fab or hinge region and may be involved in a disulfide bond between the antigen-binding domains (for example, a cysteine residue) may be substituted with another amino acid residue or deleted. Examples of such cysteine residues include the cysteine residues at positions 220, 226, and 229 according to EU numbering in the hinge region, and the cysteine residue at position 214 in the CL region.

In one embodiment of the above aspects, at least one of the first and second antigen-binding domains comprise an antibody fragment that binds to a specific antigen. In certain embodiments, the antibody fragment is Fab, Fab', scFab, Fv, scFv, or a single-domain antibody. In certain embodiments, the first and/or second antigen-binding domains comprise a hinge region. Amino acid residues that form a disulfide bond are each present in the first and second antigen-binding domains, and a bond between the antigen-binding domains is formed by linkage between those amino acid residues. In certain embodiments, at least one of the amino acid residues that form a disulfide bond between the antigen-binding domains is present within the antibody fragment.

In one aspect, each of the first antigen-binding domain and the second antigen-binding domain in the present disclosure may or may not comprise a hinge region. In certain embodiments, the first and the second antigen-binding domains in the present disclosure may each comprise a Fab and a hinge region which form a F(ab')2 structure.

In an embodiment of the above aspects, both the first and second antigen-binding domains bind to the same antigen. In certain embodiments, both the first and second antigen-binding domains bind to the same epitope on the same antigen. In certain other embodiments, each of the first and second antigen-binding domains binds to a different epitope on the same antigen. In certain embodiments, the antigen-binding molecule in the present disclosure is a biparatopic antigen-binding molecule (for example, a biparatopic antibody) that targets one specific antigen.

In another embodiment of the above aspects, each of the first and second antigen-binding domains binds to a different antigen.

In another embodiment of the above aspects, the antigen-binding molecule in the present disclosure is a clamping antigen-binding molecule (for example, a clamping antibody). A clamping antigen-binding molecule herein means an antigen-binding molecule which specifically binds to an antigen/antigen-binding molecule complex formed between a given antigen A and an antigen-binding molecule which binds to antigen A, and which thereby increases the binding activity toward antigen A of the antigen-binding molecule that binds to antigen A (or alternatively, stabilizes the antigen/antigen-binding molecule complex formed by antigen A and the antigen-binding molecule that binds to antigen A). For example, a CD3 clamping antibody specifically binds to the antigen-antibody complex formed between CD3 and an antibody with reduced binding ability toward CD3 (binding-attenuated CD3 antibody) and can thereby increase the binding activity of the binding-attenuated CD3 antibody toward CD3 (or alternatively, stabilize the antigen-antibody complex formed by CD3 and the binding-attenuated CD3 antibody). In certain embodiments, the first and/or second antigen-binding domains in the antigen-binding molecule in the present disclosure can be antigen-binding domains derived from clamping antigen-binding molecules (clamping antigen-binding domains).

In an embodiment of the above aspects, both the first and second antigen-binding domains have the same amino acid sequence. In another embodiment, each of the first and second antigen-binding domains has a different amino acid sequence.

In another embodiment of the above aspects, the antigen-binding molecule in the present disclosure has activity of regulating interaction between two antigen molecules. Without being bound by a particular theory, the activity of regulating interaction is thought to be resulted from the antigen-binding molecule in the present disclosure holding two antigen molecules at spatially closer positions and reducing their mobility. In certain embodiments, the antigen-binding molecule in the present disclosure can enhance or diminish interaction between two antigen molecules as compared to a control antigen-binding molecule. The control antigen-binding molecule differs from the antigen-binding molecule in the present disclosure only in that the control antigen-binding molecule has one less disulfide bond between the two antigen-binding domains. In a further embodiment, the one less disulfide bond can be selected from bonds derived from mutated amino acid residues that are not present in a wild-type Fab or hinge region (for example, cysteine residues that are not present in the wild-type Fab or hinge region). The mutated amino acid residues are, for example, artificially mutated, substituted, introduced, or engineered cysteine residues.

In one embodiment, antigen molecules are selected from the group consisting of receptors belonging to cytokine receptor superfamilies, G protein-coupled receptors, ion channel receptors, tyrosine kinase receptors, immune checkpoint receptors, antigen receptors, CD antigens, costimulatory molecules, and cell adhesion molecules.

In certain embodiments, the two antigen molecules bound by the antigen-binding molecule in the present disclosure can be a ligand and a receptor thereof, respectively. The antigen-binding molecule in the present disclosure has activity of promoting activation of the receptor by the ligand. In certain other embodiments, the two antigen molecules bound by the antigen-binding molecule in the present disclosure can be an enzyme and a substrate thereof, respectively. The antigen-binding molecule in the present disclosure has activity of promoting catalytic reaction of the enzyme with the substrate.

Further, in certain other embodiments, both of the two antigen molecules bound by the antigen-binding molecule in the present disclosure can be antigens (for example, proteins) present on cellular surfaces. The antigen-binding molecule in the present disclosure has activity of promoting interaction between a cell expressing the first antigen and a cell expressing the second antigen. For example, the cell expressing the first antigen and the cell expressing the second antigen can be a cell with cytotoxic activity and a target cell thereof, respectively. The antigen-binding molecule in the present disclosure promotes damage of the target cell by the cell with cytotoxic activity. The cell with cytotoxic activity is, for example, a T cell, NK cell, monocyte, or macrophage.

In an embodiment of the above aspects, the antigen-binding molecule in the present disclosure has resistance to protease cleavage. In certain embodiments, the antigen-binding molecule in the present disclosure has increased resistance to protease cleavage as compared to a control antigen-binding molecule. In certain embodiments, in the antigen-binding molecule in the present disclosure, the proportion of the full-length molecule (for example, full-length IgG molecule) remaining after protease treatment is increased as compared to the control antigen-binding molecule. In certain embodiments, in the antigen-binding molecule in the present disclosure, the proportion of a particular fragment (for example, Fab monomer) produced after protease treatment is reduced as compared to the control antigen-binding molecule.

In an embodiment of the above aspects, when the antigen-binding molecule in the present disclosure is treated with a protease, a dimer of the antigen-binding domains or fragments thereof (for example, crosslinked Fab dimer) is excised. In one embodiment, the protease can cleave the hinge region of the antigen-binding molecule.

In one aspect, at least one of the first and second antigen-binding domains in the present disclosure can bind to a soluble protein. In another embodiment, at least one of the first and second antigen-binding domains in the present disclosure can bind to a membrane protein.

The first antigen-binding domain and the second antigen-binding domain in the present disclosure can bind to a first antigen and a second antigen, respectively. In some embodiments, the first antigen and the second antigen are derived from humans, mice, rats, monkeys, rabbits, or dogs. In some embodiments, examples of the first antigen and the second antigen include, but are not limited to, for example, an immunocyte surface molecule (e.g., a T cell surface molecule, an NK cell surface molecule, a dendritic cell surface molecule, a B cell surface molecule, an NKT cell surface molecule, an MDSC cell surface molecule, and a macrophage surface molecule), or an antigen expressed not only on tumor cells, tumor vessels, stromal cells, and the like but on normal tissues (integrin, tissue factor, VEGFR, PDGFR, EGFR, IGFR, MET chemokine receptor, heparan sulfate proteoglycan, CD44, fibronectin, DR5, TNFRSF, etc.), a receptor belonging to cytokine receptor superfamilies, a G protein-coupled receptor, an ion channel receptor, a tyrosine kinase receptor, an immune checkpoint receptor, an antigen receptor, a CD antigen, a costimulatory molecule, and a cell adhesion molecule.

In some embodiments, any one of the first antigen and the second antigen can be, for example, a molecule specifically expressed on a T cell, and the other antigen can be a molecule expressed on the surface of a T cell or any other immunocyte. In another embodiment of the combination of the first antigen and the second antigen, preferably, any one of the first antigen and the second antigen is, for example, a molecule specifically expressed on a T cell, and the other antigen is a molecule that is expressed on an immunocyte and is different from the preliminarily selected antigen.

Specific examples of the molecule specifically expressed on a T cell include CD3 and T cell receptors. Particularly, CD3 is preferred. In the case of, for example, human CD3, a site in the CD3 to which the antigen-binding molecule in the present disclosure binds may be any epitope present in a gamma chain, delta chain, or epsilon chain sequence constituting the human CD3. Particularly, an epitope present in the extracellular region of an epsilon chain in a human CD3 complex is preferred. The polynucleotide sequences of the gamma chain, delta chain, and epsilon chain structures constituting CD3 are NM_000073.2, NM_000732.4, and NM_000733.3, and the polypeptide sequences thereof are NP_000064.1, NP_000723.1, and NP _000724.1 (RefSeq registration numbers). Examples of the other antigen include Fc gamma receptors, TLR, lectin, IgA, immune checkpoint molecules, TNF superfamily molecules, TNFR superfamily molecules, and NK receptor molecules.

In one embodiment, the first antigen can be a molecule specifically expressed on a T cell, preferably a T cell receptor complex molecule such as CD3, more preferably human CD3. In another embodiment, the second antigen can be a molecule expressed on a T cell or any other immune cell, preferably a cell surface modulator on an immune cell, more preferably a costimulatory molecule expressed on a T cell, and even more preferably a protein of "TNF superfamily" or the "TNF receptor superfamily" including not limited to human CD137 (4-1BB), CD137L, CD40, CD40L, OX40, OX40L, CD27, CD70, HVEM, LIGHT, RANK, RANKL, CD30, CD153, GITR, and GITRL. In one preferred embodiment, the first antigen can be CD3 and the second antigen can be CD137. Herein, the first antigen and the second antigen are defined interchangeably.

In certain embodiments, the antigen binding molecule in the present disclosure specifically binds to the whole or a portion of a partial peptide of CD3. In a particular embodiment CD3 is human CD3 or cynomolgus CD3, most particularly human CD3. In a particular embodiment the antigen binding molecule is cross-reactive for (i.e. specifically binds to) human and cynomolgus CD3. In some embodiments, the antigen binding molecule is capable of specific binding to the epsilon subunit of CD3, in particular the human CD3 epsilon subunit of CD3 which is shown in SEQ ID NO: 13 (NP_000724.1) (RefSeq registration numbers are shown within the parentheses). In some embodiments, the antigen binding molecule is capable of specific binding to the CD3 epsilon chain expressed on the surface of eukaryotic cells. In some embodiments, the antigen binding molecule binds to the CD3 epsilon chain expressed on the surface of T cells.

In certain embodiments, the antigen-binding molecule in the present disclosure specifically binds to the whole or a portion of a partial peptide of CD137. The term "CD137" herein, also called 4-1BB, is a member of the tumor necrosis factor (TNF) receptor family. Examples of factors belonging to the TNF superfamily or the TNF receptor superfamily include CD137, CD137L, CD40, CD40L, OX40, OX40L, CD27, CD70, HVEM, LIGHT, RANK, RANKL, CD30, CD153, GITR, and GITRL.

In certain embodiments, the CD137 is human CD137. In some embodiments, favorable examples of an antigen-binding molecule in the present disclosure include antigen-binding molecules that bind to the same epitope as the human CD137 epitope bound by the antibody selected from the group consisting of:

antibody that recognize a region comprising the SPCPPNSFSSAGGQRTCDICRQCKGVFRTRKECSSTSNAECD CTPGFHCLGAGCSMCEQD CKQGQELTKKGC sequence (SEQ ID NO: 14),

antibody that recognize a region comprising the DCTPGFHCLGAGCSMCEQDCKQGQELTKKGC sequence (SEQ ID NO: 15),

antibody that recognize a region comprising the LQDPCSNCPAGTFCDNNRNQICSPCPPNSFSSAGGQRTCDIC RQCKGVFRTRKECSSTSNA EC sequence (SEQ ID NO: 16), and

antibody that recognize a region comprising the LQDPCSNCPAGTFCDNNRNQIC sequence (SEQ ID NO: 17) in the human CD137 protein.

In an embodiment of the above aspects, at least one of the first and second antigen-binding domains comprises a non-antibody protein binding to a particular antigen, or a fragment thereof. In certain embodiments, the non-antibody protein is either of a pair of a ligand and a receptor which specifically bind to each other. Such receptors include, for example, receptors belonging to cytokine receptor superfamilies, G protein-coupled receptors, ion channel receptors, tyrosine kinase receptors, immune checkpoint receptors, antigen receptors, CD antigens, costimulatory molecules, and cell adhesion molecules.

In one aspect, the antigen-binding molecule of the present disclosure may further comprise a third antigen-binding domain. In one aspect, the third antigen-binding domain can be fused to either one of the first antigen-binding domain or the second antigen-binding domain.

In one aspect, the third antigen-binding domain may be a Fab or scFv; in which case, the third antigen-binding domain may be fused at its C-terminus to the N-terminus of the Fab heavy chain (VH region) of either one of the first antigen-binding domain or the second antigen-binding domain, optionally via a peptide linker.

In certain embodiments, each of the first, second, and third antigen-binding domains of the present disclosure can be a Fab molecule; in which case, the third antigen-binding domain may be fused at the C-terminus of its Fab heavy chain (CH1 region) to the N-terminus of the Fab heavy chain (VH region) of either one of the first antigen-binding domain or the second antigen-binding domain, optionally via a peptide linker.

In one aspect, an example of the peptide linker of the present disclosure may be a sequence selected from the group consisting of the amino acid sequences of SEQ ID NO: 18, SEQ ID NO: 19, and SEQ ID NO: 20, but is not limited thereto.

In one aspect, the third antigen-binding domain of the present disclosure can be a crossover Fab molecule in which the variable regions of the Fab light chain and the Fab heavy chain are exchanged, and each of the first antigen-binding domain and the second antigen-binding domain can be a conventional Fab molecule.

In one aspect, the third antigen-binding domain of the present disclosure can bind to a third antigen that is different from the first antigen and the second antigen described above. The third antigen-binding domain binding to a third antigen may be an antigen-binding domain that recognizes an arbitrary antigen. The third antigen-binding domain binding to a third antigen in the present disclosure may be an antigen-binding domain that recognizes a molecule specifically expressed in a cancer cell or a cancer tissue.

In some embodiments, the third antigen is derived from humans, mice, rats, monkeys, rabbits, or dogs. In some embodiments, the third antigen is a molecule specifically expressed on the cell or the organ derived from humans, mice, rats, monkeys, rabbits, or dogs. The third antigen is preferably, a molecule not systemically expressed on the cell or the organ. The third antigen is preferably, for example, a tumor cell-specific antigen and also includes an antigen expressed in association with the malignant alteration of cells as well as an abnormal sugar chain that appears on cell surface or a protein molecule during the malignant transformation of cells. Specific examples thereof include ALK receptor (pleiotrophin receptor), pleiotrophin, KS 1/4 pancreatic cancer antigen, ovary cancer antigen (CA125), prostatic acid phosphate, prostate-specific antigen (PSA), melanoma-associated antigen p97, melanoma antigen gp75, high-molecular-weight melanoma antigen (HMW-MAA), prostate-specific membrane antigen, carcinoembryonic antigen (CEA), polymorphic epithelial mucin antigen, human milk fat globule antigen, colorectal tumor-associated antigen (e.g., CEA, TAG-72, CO17-1A, GICA 19-9, CTA-1, and LEA), Burkitt's lymphoma antigen 38.13, CD19, human B lymphoma antigen CD20, CD33, melanoma-specific antigen (e.g., ganglioside GD2, ganglioside GD3, ganglioside GM2, and ganglioside GM3), tumor-specific transplantation antigen (TSTA), T antigen, virus-induced tumor antigen (e.g., envelope antigens of DNA tumor virus and RNA tumor virus), colon CEA, oncofetal antigen alpha-fetoprotein (e.g., oncofetal trophoblastic glycoprotein 5T4 and oncofetal bladder tumor antigen), differentiation antigen (e.g., human lung cancer antigens L6 and L20), fibrosarcoma antigen, human T cell leukemia-associated antigen Gp37, newborn glycoprotein, sphingolipid, breast cancer antigen (e.g., EGFR (epithelial growth factor receptor)), NY-BR-16, NY-BR-16 and HER2 antigen (p185HER2), polymorphic epithelial mucin (PEM), malignant human lymphocyte antigen APO-1, differentiation antigen such as I antigen found in fetal erythrocytes, primary endoderm I antigen found in adult erythrocytes, I (Ma) found in embryos before transplantation or gastric cancer, M18 found in mammary gland epithelium, M39, SSEA-1 found in bone marrow cells, VEP8, VEP9, Myl, VIM-D5, D156-22 found in colorectal cancer, TRA-1-85 (blood group H), SCP-1 found in testis and ovary cancers, C14 found in colon cancer, F3 found in lung cancer, AH6 found in gastric cancer, Y hapten, Ley found in embryonic cancer cells, TL5 (blood group A), EGF receptor found in A431 cells, E1 series (blood group B) found in pancreatic cancer, FC10.2 found in embryonic cancer cells, gastric cancer antigen, CO-514 (blood group Lea) found in adenocarcinoma, NS-10 found in adenocarcinoma, CO-43 (blood group Leb), G49 found in A431 cell EGF receptor, MH2 (blood group ALeb/Ley) found in colon cancer, 19.9 found in colon cancer, gastric cancer mucin, T5A7 found in bone marrow cells, R24 found in melanoma, 4.2, GD3, D1.1, OFA-1, GM2, OFA-2, GD2, and M1:22:25:8 found in embryonic cancer cells, SSEA-3 and SSEA-4 found in 4-cell to 8-cell embryos, cutaneous T cell lymphoma-associated antigen, MART-1 antigen, sialyl Tn (STn) antigen, colon cancer antigen NY-CO-45, lung cancer antigen NY-LU-12 variant A, adenocarcinoma antigen ART1, paraneoplastic associated brain-testis-cancer antigen (onconeuronal antigen MA2 and paraneoplastic neuronal antigen), neuro-oncological ventral antigen 2 (NOVA2), blood cell cancer antigen gene 520, tumor-associated antigen CO-029, tumor-associated antigen MAGE-C1 (cancer/testis antigen CT7), MAGE-B1 (MAGE-XP antigen), MAGE-B2 (DAM6), MAGE-2, MAGE-4a, MAGE-4b MAGE-X2, cancer-testis antigen (NY-EOS-1), YKL-40, and any fragment of these polypeptides, and modified structures thereof (aforementioned modified phosphate groups, sugar chains, etc.), EpCAM, EREG, CA19-9, CA15-3, sialyl SSEA-1 (SLX), HER2, PSMA, CEA, and CLEC12A.

In one preferred embodiment, the third antigen is Glypican-3 (GPC3). In yet another embodiment, the third antigen is DLL3 (Delta-like 3). In certain embodiments, the third antigen-binding domain can be a "DLL3 antigen-binding domain" which binds to DLL3.

The term "DLL3", as used herein, refers to any native DLL3 (Delta-like 3) from any vertebrate source, including mammals such as primates (e.g. humans) and rodents (e.g., mice and rats), unless otherwise indicated. The term encompasses "full-length" unprocessed DLL3 as well as any form of DLL3 that results from processing in the cell. The term also encompasses naturally occurring variants of DLL3, e.g., splice variants or allelic variants. The amino acid sequence of an exemplary human DLL3 is known as NCBI Reference Sequence (RefSeq) NM_016941.3, and the amino acid sequence of an exemplary cynomolgus DLL3 is known as NCBI Reference Sequence XP_005589253.1, and the amino acid sequence of an exemplary mouse DLL3 is known as NCBI Reference Sequence NM_007866.2.

The human DLL3 protein comprises a transmembrane (TM) region and an intracellular domain on the C-terminal side, and a DSL (Notch) domain on the N-terminal side. In addition, DLL3 has an EGF domain comprising six regions, EGF1 to EGF6 from the N-terminal side to the C-terminal side.

In some embodiments, the DLL3 antigen binding domain binds specifically to the extracellular domain of DLL3. In some embodiments, the DLL3 antigen binding domain binds specifically to an epitope within the extracellular domain of DLL3. In some embodiments, the DLL3 antigen binding domain binds to the DLL3 protein expressed on the surface of eukaryotic cells. In some embodiments, the DLL3 antigen binding domain binds to the DLL3 protein expressed on the surface of cancer cells.

In some embodiments, the DLL3 antigen binding domain in the present disclosure binds to an epitope within the extracellular domain (ECD), i.e., the domain from the N-terminus to immediately before the TM region, but not to the TM region or the C-terminal intracellular domain. The DLL3 antigen binding domain in the present disclosure may bind to an epitope within any of the above-mentioned domains/regions within the ECD. In preferred embodiments, the DLL3 antigen binding domain in the present disclosure binds to an epitope within the region from EGF6 to immediately before the TM region. More specifically, the DLL3 antigen binding domain in the present disclosure may bind to an epitope within the regions defined in SEQ ID NO: 21 in human DLL3. In some embodiments, the DLL3 antigen binding domain in the present disclosure binds to the EGF1, EGF2, EGF3, EGF4, EGF5, or EGF6 region or a region from EGF6 to immediately before the TM region of human DLL3, or an epitope within the EGF1, EGF2, EGF3, EGF4, EGF5, or EGF6 region or a region from EGF6 to immediately before the TM region of human DLL3. In some embodiments, the antigen-binding molecules or the DLL3 antigen binding domain can be derived from previously reported anti-DLL3 antibodies in which the DLL3 epitopes bound have been characterized (e.g. WO2019131988 and WO2011093097).

In human DLL3, the above-mentioned domains/regions have the following amino acid residues (see, e.g., http://www.uniprot.org/uniprot/Q9NYJ7 or WO2013/126746):

Extracellular domain (ECD): amino acid residues at positions 1 to 492;
DSL domain: amino acid residues at positions 176 to 215;
EGF domain: amino acid residues at positions 216 to 465;
EGF1 region: amino acid residues at positions 216 to 249;
EGF2 region: amino acid residues at positions 274 to 310;

EGF3 region: amino acid residues at positions 312 to 351;

EGF4 region: amino acid residues at positions 353 to 389;

EGF5 region: amino acid residues at positions 391 to 427;

EGF6 region: amino acid residues at positions 429 to 465;

The region from EGF6 to immediately before the TM region: amino acid residues at positions 429 to 492;

TM region: amino acid residues at positions 493 to 513; and

C-terminal intracellular domain: amino acid residues at positions 516 to 618 (or 516 to 587 in some isoforms). The amino acid positions mentioned above also refers to the amino acid positions in the amino acid sequence shown in SEQ ID NO: 22.

Thus, the antigen-binding molecules in the present disclosure may bind to an above-mentioned region/domain having the amino acid residues at the above-mentioned positions in human DLL3. That is, the antigen-binding molecules in the present disclosure may bind to an epitope within the above-mentioned region/domain having the amino acid residues at the above-mentioned positions in human DLL3.

The DLL3 protein used in the present disclosure is not limited by its origin and is preferably a human or cynomolgus DLL3 protein.

In some embodiments, for the DLL3 protein, DLL3 ECD fragment proteins (or ECD variants) may be used. Depending on the site of truncation, the fragments/variants may comprise, from the N-terminal side to the C-terminal side, the DSL domain to EGF6, EGF1 to EGF6, EGF2 to EGF6, EGF3 to EGF6, EGF4 to EGF6, EGF5 and EGF6, or EGF6. The fragments/variants may further comprise a region spanning from immediately after the EGF6 region to immediately before the TM region. A Flag tag may be attached to the C terminus of the fragments/variants using a technique well-known in the art.

The CD3, CD137, or DLL3 protein in the present disclosure may be a protein having the sequence described above or may be a modified protein having a sequence derived from the sequence described above by the modification of one or more amino acids. Examples of the modified protein having a sequence derived from the sequence described above by the modification of one or more amino acids can include polypeptides having 70% or more, preferably 80% or more, more preferably 90% or more, even more preferably 95% or more identity with to the amino acid sequence described above. Alternatively, partial peptides of these CD3, CD137, or DLL3 proteins may be used.

In certain embodiments the antigen binding molecule described herein binds to an epitope of CD3, CD137 or DLL3 that is conserved among the CD3, CD137 or DLL3 from different species. In certain embodiments the antigen binding molecule in the present disclosure is a trispecific antigen binding molecule, i.e. it is capable of specifically binding to three different antigens - capable of binding to either one of CD3 or CD137 but does not bind to both antigens simultaneously, and is capable of specifically binding to DLL3.

In an aspect, the third antigen-binding domain is a conventional Fab, and

(a) the first polypeptide comprises (starting from N-terminus to C-terminus) the VH of the third antigen-binding domain, a heavy chain constant region (CH1); and the VH of the first antigen-binding domain, a heavy chain constant region (CH1); and optionally a hinge region and/or a Fc region (CH2 and CH3);

(b) the second polypeptide comprises (starting from N-terminus to C-terminus) the VL of the third antigen-binding domain, and a light chain constant region (CL);

(c) the third polypeptide comprises (starting from N-terminus to C-terminus) the VH of the second antigen-binding domain, a heavy chain constant region (CH1); and optionally a hinge region and/or a Fc region (CH2 and CH3);

(d) the fourth polypeptide comprises (starting from N-terminus to C-terminus) the VL of the second antigen-binding domain, and a light chain constant region (CL); and

(e) the fifth polypeptide comprises (starting from N-terminus to C-terminus) the VL of the first antigen-binding domain, and a light chain constant region (CL).

In an aspect, the third antigen-binding domain is a VH/VL crossover Fab (in which the variable regions of the Fab light chain and the Fab heavy chain are exchanged), and

(a) the first polypeptide comprises (starting from N-terminus to C-terminus) the VL of the third antigen-binding domain, a heavy chain constant region (CH1); and the VH of the first antigen-binding domain, a heavy chain constant region (CH1); and optionally a hinge region and/or a Fc region (CH2 and CH3);

(b) the second polypeptide comprises (starting from N-terminus to C-terminus) the VH of the third antigen-binding domain, and a light chain constant region (CL);

(c) the third polypeptide comprises (starting from N-terminus to C-terminus) the VH of the second antigen-binding domain, a heavy chain constant region (CH1); and optionally a hinge region and/or a Fc region (CH2 and CH3);

(d) the fourth polypeptide comprises (starting from N-terminus to C-terminus) the VL of the second antigen-binding domain, and a light chain constant region (CL); and

(e) the fifth polypeptide comprises (starting from N-terminus to C-terminus) the VL of the first antigen-binding domain, and a light chain constant region (CL).

In one aspect, an antigen-binding molecule in the present disclosure can further comprise an Fc region. In an aspect, the Fc region is composed of a first and a second Fc region subunit capable of stable association.

In one aspect, in an antigen-binding molecule in the present disclosure, each of the first and second antigen-binding domains is a Fab, wherein the first antigen-binding domain is fused at the C-terminus of the Fab heavy chain to the N-terminus of the first or second subunit of the Fc region, and the second antigen-binding domain is fused at the C-terminus of the Fab heavy chain to the N-terminus of the remaining subunit of the Fc region.

In an aspect, an Fc region in the present disclosure can be derived from humans. In some embodiments, an Fc region in the present disclosure can be an IgG Fc region, preferably a human IgG Fc region, more preferably a human IgG1 Fc region.

In certain embodiments, the Fc region of the antigen-binding molecule is composed of a first and a second Fc region subunit capable of stable association, and wherein the Fc region exhibits reduced binding affinity to human Fc gamma receptor, as compared to a native human IgG1 Fc region.

In particular embodiments, the Fc region of the antigen binding molecule described herein comprises a modification promoting the association of the first and the second subunits of the Fc region. Said modification is a so-called "knob-into-hole" modification, comprising a "knob" modification in one of the two subunits of the Fc region and a "hole" modification in the other one of the two subunits of the Fc region, as described more specifically below.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001).

In a specific embodiment, in the Fc domain composed of a first and a second Fc region subunit capable of stable association and exhibiting reduced binding affinity to human Fc-gamma receptor as compared to a native human IgG1 Fc domain, the first Fc-region subunit is selected from the group consisting of:

(a1) a Fc region polypeptide comprising the mutations L234A, L235A;
(a2) a Fc region polypeptide comprising the mutations L234A, L235A, N297A; and
(a3) a Fc region polypeptide comprising the mutations L234A, L235A, N297A, S354C, T366W; and

the second Fc-region polypeptide is selected from the group consisting of:

(a4) a Fc region polypeptide comprising the mutations L234A, L235A;
(a5) a Fc region polypeptide comprising the mutations L234A, L235A, N297A; and

(a6) a Fc region polypeptide comprising the mutations L234A, L235A, N297A, Y349C, T366S, L368A, Y407V (the amino acid positions are numbered using EU index numbering).

In certain embodiments, the Fc region of the antigen-binding molecule described herein exhibits enhanced FcRn-binding activity under an acidic pH condition (e.g., pH 5.8) as compared to that of the Fc region of a native IgG. Such an Fc domain comprises Ala at position 434; Glu, Arg, Ser, or Lys at position 438; and Glu, Asp, or Gln at position 440, according to EU numbering.

In some embodiments, the Fc domain comprises Ala at position 434; Arg or Lys at position 438; and Glu or Asp at position 440, according to EU numbering.

In some embodiments, the Fc domain further comprises Ile or Leu at position 428; and/or Ile, Leu, Val, Thr, or Phe at position 436, according to EU numbering.

In an embodiment, the Fc domain comprises a combination of amino acid substitutions selected from the group consisting of:

(a) N434A/Q438R/S440E;
(b) N434A/Q438R/S440D;
(c) N434A/Q438K/S440E;
(d) N434A/Q438K/S440D;
(e) N434A/Y436T/Q438R/S440E;
(f) N434A/Y436T/Q438R/S440D;
(g) N434A/Y436T/Q438K/S440E;
(h) N434A/Y436T/Q438K/S440D;
(i) N434A/Y436V/Q438R/S440E;
(j) N434A/Y436V/Q438R/S440D;
(k) N434A/Y436V/Q438K/S440E;
(1) N434A/Y436V/Q438K/S440D;
(m) N434A/R435H/F436T/Q438R/S440E;
(n) N434A/R435H/F436T/Q438R/S440D;
(o) N434A/R435H/F436T/Q438K/S440E;
(p) N434A/R435H/F436T/Q438K/S440D;
(q) N434A/R435H/F436V/Q438R/S440E;
(r) N434A/R435H/F436V/Q438R/S440D;
(s) N434A/R435H/F436V/Q438K/S440E;
(t) N434A/R435H/F436V/Q438K/S440D;
(u) M428L/N434A/Q438R/S440E;
(v) M428L/N434A/Q438R/S440D;
(w) M428L/N434A/Q438K/S440E;
(x) M428L/N434A/Q438K/S440D;
(y) M428L/N434A/Y436T/Q438R/S440E;
(z) M428L/N434A/Y436T/Q438R/S440D;
(aa) M428L/N434A/Y436T/Q438K/S440E;
(ab) M428L/N434A/Y436T/Q438K/S440D;
(ac) M428L/N434A/Y436V/Q438R/S440E;
(ad) M428L/N434A/Y436V/Q438R/S440D;
(ae) M428L/N434AJY436V/Q438K/S440E;
(af) M428L/N434A/Y436V/Q438K/S440D;
(ag) L235R/G236R/S239K/M428L/N434A/Y436T/Q438R/S440E; and
(ah) L235R/G236R/A327G/A330S/P331S/M428L/N434A/Y436T/Q438R/S440E, according to EU numbering.

In several embodiments, the Fc region of an antigen-binding molecule comprises a combination of amino acid substitutions of M428L/N434A/Q438R/S440E.

In one aspect, the Fc region of the present disclosure may comprise a combination of one or more amino acid substitutions that promote multimerization of Fc regions. Examples of the amino acid substitution that promotes multimerization include amino acid substitutions at at least one site selected from the group consisting of positions 247, 248, 253, 254, 310, 311, 338, 345, 356, 359, 382, 385, 386, 430, 433, 434, 436, 437, 438, 439, 440, and 447 according to EU numbering (for example, see WO2016/164480). In a specific embodiment, examples of a multimer include, but are not limited to, dimer, trimer, and tetramer.

In one aspect, the antigen-binding molecule of the present disclosure may have in its hinge region, amino acid residue(s) resulting from substitution of at least one of the cysteine residues. In some embodiments, the cysteine residues can be present at positions 226 and/or 229 according to EU numbering of the hinge region.

In an embodiment, the Fc region comprises any of the following:

(a) a first Fc subunit that comprises the amino acid sequence shown in SEQ ID NO: 23 and a second Fc subunit that comprises the amino acid sequence shown in SEQ ID NO: 24;
(b) a first Fc subunit that comprises the amino acid sequence shown in SEQ ID NO: 25 and a second Fc subunit that comprises the amino acid sequence shown in SEQ ID NO: 26; or
(c) a first Fc region subunit comprising the amino acid sequence shown in SEQ ID NO: 58 and a second Fc region subunit comprising the amino acid sequence shown in SEQ ID NO: 59;

In one aspect, the antigen-binding molecule of the present disclosure is a multi-specific antigen-binding molecule. In some embodiments, the multispecific antigen-binding molecule is a bispecific antigen-binding molecule or a tri-specific antigen-binding molecule.

In one aspect, the antigen-binding molecule of the present disclosure is an antibody. In a specific embodiment, the antibody of the present disclosure is an IgG antibody, preferably an IgG1, IgG2, IgG3, or IgG4 antibody.

IV. Disulfide bond

In the present disclosure, the at least one disulfide bond in regions other than a hinge region may be described with the abbreviated term "LINC". Using this abbreviation, in some embodiments, the antigen-binding molecule in the present disclosure may be indicated as, e.g., "Dual/LINC", "DLL3-Dual/LINC", "paired cysteines form" or the like. Antigen-binding molecules of which the first antigen-binding domain and the second antigen-binding domain that are not linked/yet to be linked with each other via at least one disulfide bond may be described with the abbreviated term "unLINC" or "Dual-LINC-Ig with unpaired cysteines" or the like.

In one aspect of the present invention, each of the first antigen-binding domain and the second antigen-binding domain comprises at least one cysteine residue in a region other than a hinge region (through mutation, substitution, or insertion). In the "LINC" antigen-binding molecule in the present disclosure, the at least one cysteine residue forms at least one disulfide bond between the first antigen-binding domain and the second antigen-binding domain.

In an embodiment of the above aspects, at least one bond linking the first antigen-binding domain and the second antigen-binding domain can hold the two antigen binding domains (i.e., the first antigen-binding domain and the second antigen-binding domain as described above) at spatially close positions. By virtue of the linkage between the first antigen-binding domain and the second antigen-binding domain via the disulfide bond(s), the antigen-binding molecule in the present disclosure is capable of holding two antigen-binding domains at closer positions than a control antigen-binding molecule, which differs from the antigen-binding molecule in the present disclosure only in that the control antigen-binding molecule does not have the additional bond(s) introduced between the two antigen-binding domains. In some embodiments, the term "spatially close positions" or "closer positions" includes the meaning that the first antigen-binding domain and the second antigen-binding domain as described above are held in shortened distance and/or reduced flexibility.

As the results, the two antigen binding domains (i.e., the first antigen-binding domain and the second antigen-binding domain as described above) of the antigen-binding molecule in the present disclosure can bind to the antigens expressed on the same single cell. In other words, the respective two antigen-binding domains (i.e., the first antigen-binding domain and the second antigen-binding domain as described above) of the antigen-binding molecule in the present disclosure do not bind to antigens expressed on different cells so as to cause a cross-linking the different cells. Such antigen-binding manner of the antigen-binding molecule in the present disclosure can be called "cis-binding", whereas the antigen-binding manner of an antigen-binding molecule in which the respective two antigen-binding domains of the antigen-binding molecule bind to antigens expressed on different cells so as to cause a cross-linking the different cells can be called "trans-binding". In some embodiments, the antigen-binding molecule in the present disclosure predominantly binds to the antigens expressed on the same single cell in "cis-binding" manner.

In an embodiment of the above aspects, by virtue of the disulfide linkage between the first antigen-binding domain and the second antigen-binding domain via the disulfide bond(s) as described above, the antigen-binding molecule in the present disclosure is capable of reducing and/or preventing unwanted cross-linking and activation of immune cells (e.g., T-cells, NK cells, DC cells, or the like). That is, in some embodiments, the first antigen-binding domain of the antigen-binding molecule in the present disclosure binds to any signaling molecule expressed on an immune cell such as T-cell (e.g., the first antigen), and the second antigen-binding domain of the antigen-binding molecule in the present disclosure also binds to any signaling molecule expressed on an immune cell such as T-cell (e.g., the first antigen or the second antigen which is different from the first antigen). Thus, the first antigen-binding domain and the second antigen-binding domain of the antigen binding-molecule in the present disclosure can bind to either of the first or second signaling molecule expressed on the same single immune cell such as T cell (i.e., cis-binding manner) or on different immune cell such as T cells (i.e., trans-binding manner). When the first antigen-binding domain and the second antigen-binding domain bind to the signaling molecule expressed on different immune cells such as T-cells in trans-binding manner, those different immune cells such as T-cells are cross-linked, and, in certain situation, such crosslinking of immune cells such as T-cells may cause unwanted activation of the immune cells such as T-cells.

On the other hand, in the case of another embodiment of the antigen-binding molecule in the present disclosure, both the first antigen-binding domain and the second antigen-binding domain can bind to the signaling molecules expressed on the same single immune cell such as a T cell in "cis-binding" manner, so that the crosslinking of different immune cells such as T-cells via the antigen-binding molecule can be reduced to avoid unwanted activation of immune cells.

In an embodiment of the above aspects, the at least one bond which links the first antigen-binding domain and the second antigen-binding domain may be formed by linking amino acid residues present at the same position in the first antigen-binding domain and in the second antigen-binding domain with each other, or it may be formed by linking amino acid residues present at a respectively different position with each other.

Positions of amino acid residues in the antigen-binding domain can be shown according to the Kabat numbering or EU numbering system (also called the EU index) described in Kabat et al., Sequences of Proteins of Immunological Interest, 5th Ed. Public Health Service, National Institutes of Health, Bethesda, MD, 1991. For example, if amino acid residues involved in a bond between the first and second antigen-binding domains are present at an identical position corresponding in the antigen-binding domains, the position of these amino acid residues can be indicated as the same number according to the Kabat numbering or EU numbering system. Alternatively, if amino acid residues involved in a bond between the first and second antigen-binding domains are present at different positions which are not corresponding in the antigen-binding domains, the positions of these amino acid residues can be indicated as different numbers according to the Kabat numbering or EU numbering system.

Amino acid residues from which the disulfide bonds between the antigen-binding domains originate are respectively present in the first and second antigen-binding domains, and the bonds between the antigen-binding domains are formed by linking these amino acid residues. In an embodiment of the above aspects, at least one of the amino acid residues from which the disulfide bond between the antigen-binding domains originates is a mutated amino acid residue that is artificially mutated, substituted, introduced, or engineered and, for example, it is an artificially introduced cysteine residue. Such a mutated amino acid residue can be introduced into a wild-type antigen-binding domain by, for example, a method of amino acid substitution. In some embodiments, the at least one disulfide bond is formed between an amino acid residue in the heavy chain of the first antigen-binding domain and an amino acid residue in the heavy chain of the second antigen-binding domain, or between an amino acid residue in the light chain of the first antigen-binding domain and an amino acid residue in the light chain of the second antigen-binding domain. In a further embodiment, it is formed between amino acid residues in any combination of the CH1 region, CL region, VH region, VL region, and VHH region of the first antigen-binding domain and the CH1 region, CL region, VH region, VL region, and VHH region of the second antigen-binding domain.

In an aspect, the at least one disulfide bond is formed between an amino acid residue in the CH1 region of the first antigen-binding domain and an amino acid residue in the CH1 region of the second antigen-binding domain. In certain embodiments, the amino acid residues in the first antigen-binding domain and the second antigen-binding domain are each present at any of positions 119 to 123, 131 to 140, 148 to 150, 155 to 167, 174 to 178, 188 to 197, 201 to 214, and 218 to 219 according to EU numbering in the CH1 region. In certain embodiments, the amino acid residue is present at a position selected from the group consisting of positions 119, 122, 123, 131, 132, 133, 134, 135, 136, 137, 138, 139, 140, 148, 150, 155, 156, 157, 159, 160, 161, 162, 163, 164, 165, 167, 174, 176, 177, 178, 188, 189, 190, 191, 192, 193, 194, 195, 196, 197, 201, 203, 205, 206, 207, 208, 211, 212, 213, 214, 218, and 219 according to EU numbering in the CH1 region. In certain embodiments, the amino acid residue is present at position 134, 135, 136, 137, 191, 192, 193, 194, 195, or 196 according to EU numbering in the CH1 region. In certain embodiments, the amino acid residue is present at position 135, 136, or 191 according to EU numbering in the CH1 region.

In an embodiment of the above aspects, the at least one disulfide bond is formed between an amino acid residue in the CH1 region of the first antigen-binding domain and an amino acid residue in the CH1 region of the second antigen-binding domain. In certain embodiments, the amino acid residues in the first antigen-binding domain and the second antigen-binding domain are each independently selected from the group consisting of positions 119, 120, 121, 122, and 123 according to EU numbering. In certain embodiments, the amino acid residues in the first antigen-binding domain and the second antigen-binding domain are each independently selected from the group consisting of positions 131, 132, 133, 134, 135, 136, 137, 138, 139, and 140 according to EU numbering. In certain embodiments, the amino acid residues in the first antigen-binding domain and the second antigen-binding domain are each independently selected from the group consisting of positions 148, 149, and 150 according to EU numbering. In certain embodiments, the amino acid residues in the first antigen-binding domain and the second antigen-binding domain are each independently selected from the group consisting of positions 155, 156, 157, 158, 159, 160, 161, 162, 163, 164, 165, 166, and 167 according to EU numbering. In certain embodiments, the amino acid residues in the first antigen-binding domain and the second antigen-binding domain are each independently selected from the group consisting of positions 174, 175, 176, 177, and 178 according to EU numbering. In certain embodiments, the amino acid residues in the first antigen-binding domain and the second antigen-binding domain are each independently selected from the group consisting of positions 188, 189, 190, 191, 192, 193, 194, 195, 196, and 197 according to EU numbering. In certain embodiments, the amino acid residues in the first antigen-binding domain and the second antigen-binding domain are each independently selected from the group consisting of positions 201, 202, 203, 204, 205, 206, 207, 208, 209, 210, 211, 212, 213, and 214 according to EU numbering. In certain embodiments, the amino acid residues in the first antigen-binding domain and the second antigen-binding domain are each independently selected from the group consisting of positions 218 and 219 according to EU numbering.

In an embodiment of the above aspects, the difference in the positions of respective amino acid residues in the first antigen-binding domain and the second antigen-binding domain that form a disulfide bond (i.e. the distance between them) is three amino acids or less (i.e., three amino acids, two amino acids, or one amino acid). This means that when the position of the amino acid residue forming a disulfide bond in the CH1 region of the first antigen-binding domain and the position of the amino acid residue forming the disulfide bond in the CH1 region of the second antigen-binding domain are compared according to EU numbering, the difference (i.e., distance) is three amino acids or less. In certain embodiments, the at least one disulfide bond between the first antigen-binding domain and the second antigen-binding domain is formed between the amino acid residue at position 135 according to EU numbering in the CH1 region of the first antigen-binding domain and an amino acid residue at any of positions 132 to 138 according to EU numbering in the CH1 region of the second antigen-binding domain. In certain embodiments, the at least one disulfide bond between the first antigen-binding domain and the second antigen-binding domain is formed between the amino acid residue at position 136 according to EU numbering in the CH1 region of the first antigen-binding domain and an amino acid residue at any of positions 133 to 139 according to EU numbering in the CH1 region of the second antigen-binding domain.

In certain embodiments, the at least one disulfide bond between the first antigen-binding domain and the second antigen-binding domain is formed between the amino acid residue at position 191 according to EU numbering in the CH1 region of the first antigen-binding domain and an amino acid residue at any of positions 188 to 194 according to EU numbering in the CH1 region of the second antigen-binding domain. In an exemplary embodiment, the at least one disulfide bond between the first antigen-binding domain and the second antigen-binding domain is formed between the amino acid residues at position 135 according to EU numbering in the CH1 regions of the two antigen-binding domains. In an exemplary embodiment, the at least one disulfide bond between the first antigen-binding domain and the second antigen-binding domain is formed between the amino acid residues at position 136 according to EU numbering in the CH1 regions of the two antigen-binding domains. In an exemplary embodiment, the at least one disulfide bond between the first antigen-binding domain and the second antigen-binding domain is formed between the amino acid residues at position 191 according to EU numbering in the CH1 regions of the two antigen-binding domains.

In some embodiments, an antigen-binding molecule in the present disclosure comprises one, two, or more additional disulfide bonds between the first antigen-binding domain and the second antigen-binding domain. The additional one, two or more disulfide bond(s) is/are formed between the first antigen-binding domain and the second antigen-binding domain via the amino acid residues at the following positions according to EU numbering in each of the respective CH1 regions of the first antigen-binding domain and the second antigen-binding domain:

(a) between amino acid residues at any position of 131 to 138, 194 and 195 in each of the two antigen-binding domains;
(b) between the amino acid residues at position 131 in each of the two antigen-binding domains, and between the amino acid residues at position 194 in each of the two antigen-binding domains;
(c) between the amino acid residues at position 132 in each of the two antigen-binding domains, and between the amino acid residues at position 194 in each of the two antigen-binding domains;
(d) between the amino acid residues at position 133 in each of the two antigen-binding domains, and between the amino acid residues at position 194 in each of the two antigen-binding domains;
(e) between the amino acid residues at position 134 in each of the two antigen-binding domains, and between the amino acid residues at position 194 in each of the two antigen-binding domains;
(f) between the amino acid residues at position 135 in each of the two antigen-binding domains, and between the amino acid residues at position 194 in each of the two antigen-binding domains;
(g) between the amino acid residues at position 136 in each of the two antigen-binding domains, and between the amino acid residues at position 194 in each of the two antigen-binding domains;
(h) between the amino acid residues at position 137 in each of the two antigen-binding domains, and between the amino acid residues at position 194 in each of the two antigen-binding domains;
(i) between the amino acid residues at position 138 in each of the two antigen-binding domains, and between the amino acid residues at position 194 in each of the two antigen-binding domains;
(j) between the amino acid residues at position 131 in each of the two antigen-binding domains, and between the amino acid residues at position 195 in each of the two antigen-binding domains;

(k) between the amino acid residues at position 132 in each of the two antigen-binding domains, and between the amino acid residues at position 195 in each of the two antigen-binding domains;

(l) between the amino acid residues at position 133 in each of the two antigen-binding domains, and between the amino acid residues at position 195 in each of the two antigen-binding domains;

(m) between the amino acid residues at position 134 in each of the two antigen-binding domains, and between the amino acid residues at position 195 in each of the two antigen-binding domains;

(n) between the amino acid residues at position 135 in each of the two antigen-binding domains, and between the amino acid residues at position 195 in each of the two antigen-binding domains;

(o) between the amino acid residues at position 136 in each of the two antigen-binding domains, and between the amino acid residues at position 195 in each of the two antigen-binding domains;

(p) between the amino acid residues at position 137 in each of the two antigen-binding domains, and between the amino acid residues at position 195 in each of the two antigen-binding domains; and

(q) between the amino acid residues at position 138 in each of the two antigen-binding domains, and between the amino acid residues at position 195 in each of the two antigen-binding domains.

In some embodiments of the above aspects, any one of the first and second antigen-binding domains comprises one, two or more charged amino acid residues at position 136-138 (according to EU numbering) in the respective CH1 region; and the other antigen-binding domain out of the first and second antigen-binding domains comprises one, two or more oppositely charged amino acid residues at position 193-195 (according to EU numbering) in the respective CH1 region.

In some embodiments of the above aspects, any one of the first and second antigen-binding domains comprises one, two or more positively charged amino acid residues at position 136-138 (according to EU numbering) in the respective CH1 region; and the other antigen-binding domain out of the first and second antigen-binding domains comprises one, two or more negatively charged amino acid residues at position 193-195 (according to EU numbering) in the respective CH1 region.

In some embodiments of the above aspects, any one of the first and second antigen-binding domains comprises one, two or more negatively charged amino acid residues at position 136-138 (according to EU numbering) in the respective CH1 region; and the other antigen-binding domain out of the first and second antigen-binding domains comprises one, two or more positively charged amino acid residues at position 193-195 (according to EU numbering) in the respective CH1 region.

In some embodiments of the above aspects, any one of the first and second antigen-binding domains comprises one, two or more of the following amino acid residues in the respective CH1 region (according to EU numbering):

(a) the amino acid residue at position 136 which is glutamic acid (E) or aspartic acid (D);

(b) the amino acid residue at position 137 which is glutamic acid (E) or aspartic acid (D);

(c) the amino acid residue at position 138 which is glutamic acid (E) or aspartic acid (D); and the other antigen-binding domain of the first and second antigen-binding domains comprises one, two or more of the following amino acid residues in the respective CH1 region (according to EU numbering):

(d) the amino acid residue at position 193 which is lysine (K), arginine (R), or histidine (H);

(e) the amino acid residue at position 194 which is lysine (K), arginine (R), or histidine (H); and

(f) the amino acid residue at position 195 which is lysine (K), arginine (R), or histidine (H).

In some embodiments of the above as aspects, any one of the first and second antigen-binding domains comprises one or more of the following amino acid residues in the respective CH1 region (according to EU numbering):

(a) the amino acid residue at position 136 which is lysine (K), arginine (R), or histidine (H);

(b) the amino acid residue at position 137 which is lysine (K), arginine (R), or histidine (H);

(c) the amino acid residue at position 138 which is lysine (K), arginine (R), or histidine (H); and the other antigen-binding domain of the first and second antigen-binding domains comprises one or more of the following amino acid residues in the respective CH1 region (according to EU numbering):

(d) the amino acid residue at position 193 which is glutamic acid (E) or aspartic acid (D);

(e) the amino acid residue at position 194 which is glutamic acid (E) or aspartic acid (D); and

(f) the amino acid residue at position 195 which is glutamic acid (E) or aspartic acid (D).

In certain embodiments, each of the first and second antigen-binding domains comprises any of the combinations of specific charge mutations in the respective CH1 region (according to EU numbering) listed in Table 1, Table 2, or Table 3.

[Table 1]

| Charge mutations (EU numbering) |
|---|
| S136K/G137K/G138K/L193D/G194D/T195D |
| S136K/G137K/G138K/L193E/G194E/T195E |
| S136R/G137R/G138R/L193D/G194D/T195D |
| S136R/G137R/G138R/L193E/G194E/T195E |
| G137K/G138K/L193D/G194D/T195D |
| S136K/G138K/L193D/G194D/T195D |
| S136K/G137K/L193D/G194D/T195D |
| S136K/G137K/G138K/G194D/T195D |
| S136K/G137K/G138K/L193D/T195D |
| S136K/G137K/G138K/L193D/G194D |
| G137K/G138K/L193E/G194E/T195E |
| S136K/G138K/L193E/G194E/T195E |
| S136K/G137K/L193E/G194E/T195E |
| S136K/G137K/G138K/G194E/T195E |
| S136K/G137K/G138K/L193E/T195E |
| S136K/G137K/G138K/L193E/G194E |
| G137R/G138R/L193D/G194D/T195D |
| S136R/G138R/L193D/G194D/T195D |
| S136R/G137R/L193D/G194D/T195D |
| S136R/G137R/G138R/G194D/T195D |
| S136R/G137R/G138R/L193D/T195D |
| S136R/G137R/G138R/L193D/G194D |
| S136K/L193D/G194D/T195D |
| G137K/L193D/G194D/T195D |
| G138K/L193D/G194D/T195D |
| S136K/L193E/G194E/T195E |
| G137K/L193E/G194E/T195E |
| G138K/L193E/G194E/T195E |
| S136R/L193D/G194D/T195D |
| G137R/L193D/G194D/T195D |
| G138R/L193D/G194D/T195D |
| S136R/L193E/G194E/T195E |
| G137R/L193E/G194E/T195E |
| G138R/L193E/G194E/T195E |
| G138K/T195D |
| G138K/T195E |
| G138R/T195D |
| G138R/T195E |
| G138D/T195K |

(continued)

| Charge mutations (EU numbering) |
|---|
| G138D/T195R |
| G138E/T195K |
| G138E/T195R |
| G138K/G194D |
| G138K/G194E |
| G138R/G194D |
| G138R/G194E |
| G138D/G194K |
| G138D/G194R |
| G138E/G194K |
| G138E/G194R |
| G137K/T195D |
| G137K/T195E |
| G137R/T195D |
| G137R/T195E |
| G137D/T195K |
| G137D/T195R |
| G137E/T195K |
| G137E/T195R |
| G137K/G194D |
| G137K/G194E |
| G137R/G194D |
| G137R/G194E |
| G137D/G194K |
| G137D/G194R |
| G137E/G194K |
| G137E/G194R |
| G137K/G138K/G194E/T195E |
| G137K/G138K/L193E/T195E |
| S136K/G138K/G194E/T195E |
| S136K/G138K/L193E/T195E |
| S136K/G137K/G194E/T195E |
| S136K/G137K/L193E/T195E |
| S136K/G194E/T195E |
| S136K/L193E/T195E |
| G137K/G194E/T195E |
| G137K/L193E/T195E |
| G138K/G194E/T195E |
| G138K/L193E/T195E |

(continued)

| Charge mutations (EU numbering) |
|---|
| G137R/G138R/G194D/T195D |
| G137R/G138R/L193D/T195D |
| S136R/G138R/G194D/T195D |
| S136R/G138R/L193D/T195D |
| S136R/G137R/G194D/T195D |
| S136R/G137R/L193D/T195D |
| S136R/G194D/T195D |
| S136R/L193D/T195D |
| G137R/G194D/T195D |
| G137R/L193D/T195D |
| G138R/G194D/T195D |
| G138R/L193D/T195D |
| S136K/T195D |
| S136K/T195E |
| S136R/T195D |
| S136R/T195E |
| S136D/T195K |
| S136D/T195R |
| S136E/T195K |
| S136E/T195R |
| S136K/G194D |
| S136K/G194E |
| S136R/G194D |
| S136R/G194E |
| S136D/G194K |
| S136D/G194R |
| S136E/G194K |
| S136E/G194R |

[Table 2]

| Charge mutations (EU numbering) |
|---|
| G137K/G138K/L193E |
| G137K/G138K/G194E |
| G137K/G138K/T195E |
| S136K/G138K/L193E |
| S136K/G138K/G194E |
| S136K/G138K/T195E |
| S136K/G137K/L193E |
| S136K/G137K/G194E |

(continued)

| Charge mutations (EU numbering) |
|---|
| S136K/G137K/T195E |
| G137R/G138R/L193D |
| G137R/G138R/G194D |
| G137R/G138R/T195D |
| S136R/G138R/L193D |
| S136R/G138R/G194D |
| S136R/G138R/T195D |
| S136R/G137R/L193D |
| S136R/G137R/G194D |
| S136R/G137R/T195D |
| G137K/G138K/L193D |
| G137K/G138K/G194D |
| G137K/G138K/T195D |
| S136K/G138K/L193D |
| S136K/G138K/G194D |
| S136K/G138K/T195D |
| S136K/G137K/L193D |
| S136K/G137K/G194D |
| S136K/G137K/T195D |
| G137R/G138R/L193E |
| G137R/G138R/G194E |
| G137R/G138R/T195E |
| S136R/G138R/L193E |
| S136R/G138R/G194E |
| S136R/G138R/T195E |
| S136R/G137R/L193E |
| S136R/G137R/G194E |
| S136R/G137R/T195E |
| S136K/L193D |
| S136K/L193E |
| S136R/L193D |
| S136R/L193E |
| G137K/L193D |
| G137K/L193E |
| G137R/L193D |
| G137R/L193E |
| G138K/L193D |
| G138K/L193E |
| G138R/L193D |

(continued)

| Charge mutations (EU numbering) |
| --- |
| G138R/L193E |

[Table 3]

| Charge mutations (EU numbering) |
| --- |
| G137R/G138R/L193D |
| G137R/G138R/T195D |
| S136R/G138R/L193D |
| S136R/G138R/T195D |
| S136R/G137R/L193D |
| S136R/G137R/T195D |
| G137R/G138R/L193E |
| G137R/G138R/T195E |
| S136R/G138R/L193E |
| S136R/G138R/T195E |
| S136R/G137R/L193E |
| S136R/G137R/T195E |

In some embodiments of the above aspects, any one of the first and second antigen-binding domains comprises one, two or more hydrophobic amino acid residues at position 136-138 (according to EU numbering) in the respective CH1 region; and the other antigen-binding domain out of the first and second antigen-binding domains comprises one, two or more hydrophobic amino acid residues at position 193-195 (according to EU numbering) in the respective CH1 region.

In some embodiments of the above aspects, the hydrophobic amino acid residue(s) is/are alanine (Ala), valine (Val), leucine (Leu), isoleucine (Ile), phenylalanine (Phe), and/or tryptophan (Trp).

In certain embodiments, each of the first and second antigen-binding domains comprise any of the combinations of specific hydrophobic amino acid mutations in the respective CH1 region (according to EU numbering) listed in Table 4.

[Table 4]

| Hydrophobic mutations (EU numbering) |
| --- |
| S136W/G137W/G138W/L193W/G194W/T195W |
| S136L/G137L/G138L/G194L/T195L |
| S136V/G137V/G138V/L193V/G194V/T195V |
| S136A/G137A/G138A/L193A/G94A/T195A |
| S136V/G137V/G138V/L193W/G194W/T195W |
| S136W/G137W/G138W/L193V/G194V/T195V |
| S136V/G137V/G138V/G194L/T195L |
| S136L/G137L/G138L/L193V/G194V/T195V |
| G137V/G138V/L193V/G194V/T195V |
| S136V/G138V/L193V/G194V/T195V |
| S136V/G137V/L193V/G194V/T195V |
| S136V/G137V/G138V/G194V/T195V |
| S136V/G137V/G138V/L193V/T195V |
| S136V/G137V/G138V/L193V/G194V |

(continued)

| Hydrophobic mutations (EU numbering) |
| --- |
| S136A/G137A/G138A/L193W/G194W/T195W |
| S136A/G137A/G138A/G194L/T195L |
| S136A/G137A/G138A/L193V/G194V/T195V |
| S136W/G137W/G138W/L193A/G194A/T195A |
| G137W/G138W/L193A/G194A/T195A |
| S136W/G138W/L193A/G194A/T195A |
| S136W/G137W/L193A/G194A/T195A |
| S136W/L193A/G194A/T195A |
| G137W/L193A/G194A/T195A |
| G138W/L193A/G194A/T195A |
| S136L/G137L/G138L/L193A/G194A/T195A |
| S136V/G137V/G138V/L193A/G194A/T195A |
| S136V/L193V/G194V/T195V |
| G137V/L193V/G194V/T195V |
| G138V/L193V/G194V/T195V |
| S136W/L193S/G194V/T195A |
| G137W/L193S/G194V/T195A |
| G138W/L193S/G194V/T195A |
| G137V/G138A/L193W |
| G137V/G138A/G194W |
| G137V/G138A/T195W |

In some embodiments of the above aspects, any one of the first and second antigen-binding domains comprises one "knob" amino acid residues at position 136-138 (according to EU numbering) in the respective CH1 region; and the other antigen-binding domain out of the first and second antigen-binding domains comprises one, two or more "hole" amino acid residues at position 193-195 (according to EU numbering) in the respective CH1 region.

In some embodiments of the above aspects, any one of the first and second antigen-binding domains comprises one, two or more "hole" amino acid residues at position 136-138 (according to EU numbering) in the respective CH1 region; and the other antigen-binding domain out of the first and second antigen-binding domains comprises one "knob" amino acid residues at position 193-195 (according to EU numbering) in the respective CH1 region.

In some embodiments, said "knob" amino acid residue(s) is/are selected from the group consisting of tryptophan (Trp) and phenylalanine (Phe); and said "hole" amino acid residue(s) is/are selected from the group consisting of alanine (Ala), valine (Val), threonine (Thr), and serine (Ser).

In some embodiments of the above aspects, any one of the first and second antigen-binding domains comprises one, two or more aromatic amino acid residues at position 136-138 (according to EU numbering) in the respective CH1 region; and the other antigen-binding domain out of the first and second antigen-binding domains comprises one, two or more positively charged amino acid residues at position 193-195 (according to EU numbering) in the respective CH1 region.

In some embodiments of the above aspects, any one of the first and second antigen-binding domains comprises one, two or more positively charged amino acid residues at position 136-138 (according to EU numbering) in the respective CH1 region; and the other antigen-binding domain out of the first and second antigen-binding domains comprises one, two or more aromatic amino acid residues at position 193-195 (according to EU numbering) in the respective CH1 region.

In some embodiments, said aromatic amino acid residue(s) is/are selected from the group consisting of tryptophan (Trp), tyrosine (Tyr), histidine (His), and phenylalanine (Phe); and said positively charged amino acid residue(s) is/are selected from a group consisting of lysine (Lys), arginine (Arg), and histidine (His).

In one aspect, the at least one disulfide bond is formed between an amino acid residue in the CL region of the first antigen-binding domain and an amino acid residue in the CL region of the second antigen-binding domain. The amino acid residue is present at, for example, any of positions 108 to 112, 121 to 128, 151 to 156, 184 to 190, 195 to 196, 200 to 203, and 208 to 213 according to Kabat numbering in the CL region. In certain embodiments, the amino acid residue is present at a position selected from the group consisting of positions 108, 109, 112, 121, 123, 126, 128, 151, 152, 153, 156, 184, 186, 188, 189, 190, 195, 196, 200, 201, 202, 203, 208, 210, 211, 212, and 213 according to Kabat numbering in the CL region. In certain embodiments, the amino acid residue is present at position 126 according to Kabat numbering in the CL region.

In one embodiment of the above aspects, the at least one disulfide bond is formed between an amino acid residue in the CL region of the first antigen-binding domain and an amino acid residue in the CL region of the second antigen-binding domain. In certain embodiments, the amino acid residues in the first antigen-binding domain and the second antigen-binding domain are each independently selected from the group consisting of positions 108, 109, 110, 111, and 112 according to Kabat numbering. In certain embodiments, the amino acid residues in the first antigen-binding domain and the second antigen-binding domain are each independently selected from the group consisting of positions 121, 122, 123, 124, 125, 126, 127, and 128 according to Kabat numbering. In certain embodiments, the amino acid residues in the first antigen-binding domain and the second antigen-binding domain are each independently selected from the group consisting of positions 151, 152, 153, 154, 155, and 156 according to Kabat numbering. In certain embodiments, the amino acid residues in the first antigen-binding domain and the second antigen-binding domain are each independently selected from the group consisting of positions 184, 185, 186, 187, 188, 189, and 190 according to Kabat numbering. In certain embodiments, the amino acid residues in the first antigen-binding domain and the second antigen-binding domain are each independently selected from the group consisting of positions 195 and 196 according to Kabat numbering. In certain embodiments, the amino acid residues in the first antigen-binding domain and the second antigen-binding domain are each independently selected from the group consisting of positions 200, 201, 202, and 203 according to Kabat numbering. In certain embodiments, the amino acid residues in the first antigen-binding domain and the second antigen-binding domain are each independently selected from the group consisting of positions 208, 209, 210, 211, 212, and 213 according to Kabat numbering.

In one embodiment of the above-described aspects, the difference in (i.e., distance between) the positions of the amino acid residues forming each of the disulfide bonds in the first antigen-binding domain and the second antigen-binding domain is within three amino acids (that is, three amino acids, two amino acids, or one amino acid). This means that when the position of the amino acid residue forming the disulfide bond in the CL region of the first antigen-binding domain and the position of the amino acid residue forming the disulfide bond in the CL region of the second antigen-binding domain are compared according to EU numbering, the difference (i.e., distance) is within three amino acids. In an exemplary embodiment, the at least one disulfide bond between the first antigen-binding domain and the second antigen-binding domain is formed by linking the amino acid residues at position 126 according to Kabat numbering in the CL regions of the two antigen-binding domains with each other.

In an embodiment of the above aspects, the at least one disulfide bond is formed between an amino acid residue in the CH1 region of the first antigen-binding domain and an amino acid residue in the CL region of the second antigen-binding domain. In certain embodiments, the amino acid residue in the CH1 region of the first antigen-binding domain is selected from the group consisting of positions 188, 189, 190, 191, 192, 193, 194, 195, 196, and 197 according to EU numbering, and the amino acid residue in the CL region of the second antigen-binding domain is selected from the group consisting of positions 121, 122, 123, 124, 125, 126, 127, and 128 according to Kabat numbering. In an exemplary embodiment, the at least one disulfide bond is formed between the amino acid residue at position 191 according to EU numbering in the CH1 region of the first antigen-binding domain and the amino acid residue at position 126 according to Kabat numbering in the CL region of the second antigen-binding domain.

In an aspect, the constant region is derived from human. In certain embodiments, the subclass of the heavy chain constant region is any of IgG1, IgG2, IgG3, IgG4, IgA1, IgA2, IgM, IgD, and IgE. In certain embodiments, the subclass of the CH1 region is any of gamma 1, gamma 2, gamma 3, gamma 4, alpha 1, alpha 2, mu, delta, and epsilon.

In an aspect, in the constant domain CL of the light chain of each of the first and second antigen-binding moieties of the antigen-binding molecule of the present disclosure, the amino acid at position 123 and/or the amino acid at position 124 may be substituted independently by lysine (K), arginine (R), or histidine (H) (numbering according to Kabat). In other aspects, in the constant domain CH1 of the heavy chain of each of the first and second antigen-binding moieties of the antigen-binding molecule of the present disclosure, the amino acid at position 147 and/or the amino acid at position 213 may be substituted independently by glutamic acid (E) or aspartic acid (D) (numbering according to EU numbering).

In an aspect, in the constant domain CL of the light chain of each of the first and second antigen-binding moieties of the antigen-binding molecule of the present disclosure, the amino acids at positions 123 and 124 can each be arginine (R) and lysine (K) (numbering according to Kabat). In other embodiments, in the constant domain CH1 of the heavy chain of each of the first and second antigen-binding moieties of the antigen-binding molecule of the present disclosure, the amino acids at positions 147 and 213 may be glutamic acid (E) (numbering according to EU numbering).

In one embodiment of the above-described aspects, the constant region is derived from human. In certain embodiments, the subclass of the CL region is κ or λ.

In one embodiment of the above-described aspects, the at least one disulfide bond is formed between an amino acid residue in the variable region of the first antigen-binding domain and an amino acid residue in the variable region of the second antigen-binding domain. In certain embodiments, the at least one disulfide bond is formed between an amino acid residue in the VH region of the first antigen-binding domain and an amino acid residue in the VH region of the second antigen-binding domain. In certain embodiments, the amino acid residues are present, for example, at any position selected from the group consisting of positions 6, 8, 16, 20, 25, 26, 28, 74, and 82b according to EU numbering of the VH region. In certain embodiments, the at least one disulfide bond is formed between an amino acid residue in the VL region of the first antigen-binding domain and an amino acid residue in the VL region of the second antigen-binding domain. In certain embodiments, the aforementioned amino acid residue is present, for example, at any position selected from the group consisting of positions 21, 27, 58, 77, 100, 105, and 107 according to Kabat numbering in the VL region (subclass κ) and positions 6, 19, 33, and 34 according to Kabat numbering in the VL region (subclass λ).

In one embodiment of the above-described aspects, the at least one disulfide bond is formed between an amino acid residue in the VHH region of the first antigen-binding domain and an amino acid residue in the VHH region of the second antigen-binding domain. In certain embodiments, the amino acid residue is present, for example, at any position selected from the group consisting of positions 4, 6, 7, 8, 9, 10, 11, 12, 14, 15, 17, 20, 24, 27, 29, 38, 39, 40, 41, 43, 44, 45, 46, 47, 48, 49, 67, 69, 71, 78, 80, 82, 82c, 85, 88, 91, 93, 94, and 107 according to Kabat numbering in the VHH region.

In other embodiments, other techniques for promoting the association among H chains and between L and H chains having the desired combinations can be applied to the antigen-binding molecules in the present disclosure.

For example, techniques for suppressing undesired H-chain association by introducing electrostatic repulsion at the interface of the second constant region or the third constant region of the antibody H chain (CH2 or CH3) can be applied to antigen-binding molecule association (WO2006/106905).

In the technique of suppressing unintended H-chain association by introducing electrostatic repulsion at the interface of CH2 or CH3, examples of amino acid residues in contact at the interface of the other constant region of the H chain include regions corresponding to the residues at EU numbering positions 356, 439, 357, 370, 399, and 409 in the CH3 region.

More specifically, examples include an antibody comprising two types of H-chain CH3 regions, in which one to three pairs of amino acid residues in the first H-chain CH3 region, selected from the pairs of amino acid residues indicated in (1) to (3) below, carry the same type of charge: (1) amino acid residues comprised in the H chain CH3 region at EU numbering positions 356 and 439; (2) amino acid residues comprised in the H-chain CH3 region at EU numbering positions 357 and 370; and (3) amino acid residues comprised in the H-chain CH3 region at EU numbering positions 399 and 409.

Furthermore, the antibody may be an antibody in which pairs of the amino acid residues in the second H-chain CH3 region which is different from the first H-chain CH3 region mentioned above, are selected from the aforementioned pairs of amino acid residues of (1) to (3), wherein the one to three pairs of amino acid residues that correspond to the aforementioned pairs of amino acid residues of (1) to (3) carrying the same type of charges in the first H-chain CH3 region mentioned above carry opposite charges from the corresponding amino acid residues in the first H-chain CH3 region mentioned above.

Each of the amino acid residues indicated in (1) to (3) above come close to each other during association. Those skilled in the art can find out positions that correspond to the above-mentioned amino acid residues of (1) to (3) in a desired H-chain CH3 region or H-chain constant region by homology modeling and such using commercially available software, and amino acid residues of these positions can be appropriately subjected to modification.

In the antigen-binding molecules mentioned above, "charged amino acid residues" are preferably selected, for example, from amino acid residues included in either one of the following groups:

(a) glutamic acid (E) and aspartic acid (D); and
(b) lysine (K), arginine (R), and histidine (H).

In the above-mentioned antigen-binding molecules, the phrase "carrying the same type of charge" means, for example, that all of the two or more amino acid residues are selected from the amino acid residues included in either one of groups (a) and (b) mentioned above. The phrase "carrying opposite charges" means, for example, that when at least one of the amino acid residues among two or more amino acid residues is selected from the amino acid residues included in either one of groups (a) and (b) mentioned above, the remaining amino acid residues are selected from the amino acid residues included in the other group.

In a preferred embodiment, the antigen-binding molecules mentioned above may have their first H-chain CH3 region and second H-chain CH3 region crosslinked by disulfide bonds.

In the present disclosure, amino acid residues subjected to modification are not limited to the above-mentioned amino acid residues of the antibody variable regions or the antibody constant regions. Those skilled in the art can identify the amino acid residues that form an interface in mutant polypeptides or heteromultimers by homology modeling and such using commercially available software; and amino acid residues of these positions can then be subjected to modification so as to regulate the association.

In addition, other known techniques can also be used for formation of antigen-binding molecules in the present disclosure. Association of polypeptides having different sequences can be induced efficiently by complementary association of CH3 using a strand-exchange engineered domain CH3 produced by changing part of one of the H-chain CH3s of an antigen-binding molecule to a corresponding IgA-derived sequence and introducing a corresponding IgA-derived sequence into the complementary portion of the other H-chain CH3 (Protein Engineering Design & Selection, 23; 195-202, 2010). This known technique can also be used to efficiently form antigen-binding molecules of interest.

In addition, technologies for antibody production using association of antibody CH1 and CL and association of VH and VL as described in WO 2011/028952, WO2014/018572, and Nat Biotechnol. 2014 Feb; 32(2):191-8; technologies for producing bispecific antibodies using separately prepared monoclonal antibodies in combination (Fab Arm Exchange) as described in WO2008/119353 and WO2011/131746; technologies for regulating association between antibody heavy-chain CH3s as described in WO2012/058768 and WO2013/063702; technologies for producing antigen-binding molecules composed of two types of light chains and one type of heavy chain as described in WO2012/023053; technologies for producing antigen-binding molecules using two bacterial cell strains that individually express one of the chains of an antibody comprising a single H chain and a single L chain as described by Christoph et al. (Nature Biotechnology Vol. 31, p 753-758 (2013)); and such may be used for the formation of antigen-binding molecules.

Alternatively, even when an antigen-binding molecule of interest cannot be formed efficiently, an antigen-binding molecule in the present disclosure can be obtained by separating and purifying the antigen-binding molecule of interest from the produced antibodies. For example, a method for enabling purification of two types of homomeric forms and the heteromeric antibody of interest by ion-exchange chromatography by imparting a difference in isoelectric points by introducing amino acid substitutions into the variable regions of the two types of H chains has been reported (WO2007114325). To date, as a method for purifying heteromeric antibodies, methods using Protein A to purify a heterodimeric antibody comprising a mouse IgG2a H chain that binds to Protein A and a rat IgG2b H chain that does not bind to Protein A have been reported (WO98050431 and WO95033844). Furthermore, a heterodimeric antibody can be purified efficiently on its own by using H chains comprising substitution of amino acid residues at EU numbering positions 435 and 436, which is the IgG-Protein A binding site, with Tyr, His, or such which are amino acids that yield a different Protein A affinity, or using H chains with a different protein A affinity, to change the interaction of each of the H chains with Protein A, and then using a Protein A column.

<u>V. Antigen-binding molecules that comprise a first antigen-binding domain and a second antigen-binding domain, each of which is capable of binding to CD3 and CD137, but does not bind to CD3 and CD137 simultaneously, and a third antigen-binding domain that is capable of binding to DLL3</u>

In one aspect, the antigen-binding domains of the present disclosure can be a first antigen-binding domain and a second antigen domain, each of which is capable of binding to CD3 and CD137, but does not bind to CD3 and CD137 at the same time, and a third antigen-binding domain that is capable of binding to a third antigen which is preferably an antigen expressed on a cancer cell/tissue. More specifically, in one aspect, the antigen-binding molecule of the present disclosure can be an antigen-binding molecule comprising a first antigen-binding domain and a second antigen domain, each of which is capable of binding to CD3 and CD137, but does not bind to CD3 and CD137 at the same time, and a third antigen-binding domain that is capable of binding to a third antigen which is preferably an antigen expressed on a cancer cell/tissue.

In an aspect, each of the first antigen-binding domain and the second antigen-binding domain may be a "Dual antigen-binding domain" that is capable of binding to CD3 and CD137 but does not bind simultaneously to both CD3 and CD137. The third antigen-binding domain may be a "DLL3 antigen-binding domain".

In some embodiments, each of the first antigen-binding domain and the second antigen-binding domain is a Fab molecule, and the antigen-binding molecule comprises at least one disulfide bond formed between the CH1 region of the first antigen-binding domain and the CH1 region of the second antigen-binding domain. In a preferred embodiment, the disulfide bond may be formed between the amino acid residues (cysteine residues) at position 191 according to EU numbering in the respective CH1 region of the first antigen-binding domain and the second antigen-binding domain.

In some embodiments, the third antigen-binding domain may be Fab or scFv, and is fused to one of either the first antigen-binding domain or the second antigen-binding domain. When each of the first, second, and third antigen-binding domains is a Fab molecule that comprises a heavy chain comprising the VH region and the CH1 region and a light chain comprising the VL region and the CL region, the third antigen-binding domain may be fused at the C-terminus of the Fab heavy chain (CH1) to the N-terminus of the Fab heavy chain of one of either the first antigen-binding domain or the second antigen-binding domain, directly or via a peptide linker. Representative peptide linkers include those consisting of the amino acid sequence of SEQ ID NO: 18, 19, or 20. In a specific embodiment, the first antigen-binding domain is the same as the second antigen-binding domain.

In some embodiments, the third antigen-binding domain is a crossover Fab molecule in which the variable regions of the Fab light chain and the Fab heavy chain are exchanged, and each of the first and second antigen-binding domains is a conventional Fab molecule.

In certain embodiments, the Dual antigen-binding domain ("first antigen-binding domain" or "second antigen-binding domain" or "Dual-Fab") specifically binds to the whole CD3 or to a portion of a partial peptide of CD3.

In certain embodiments, the Dual antigen-binding domain ("first antigen-binding domain" or "second antigen-binding domain" or "Dual-Fab") specifically binds to the whole CD137 or to a portion of a partial peptide of CD137.

In certain embodiments, the first and second antigen-binding domains of the antigen-binding molecule of the present disclosure each comprises an antibody variable region that can be the same or different and is independently selected from the group consisting of the following (a1) to (a4):

(a1) an antibody variable region that comprises

a heavy chain variable region comprising
a heavy chain complementarity determining region (CDR) 1 comprising the amino acid sequence of SEQ ID NO: 27,
a heavy chain CDR 2 comprising the amino acid sequence of SEQ ID NO: 28, and
a heavy chain CDR 3 comprising the amino acid sequence of SEQ ID NO: 29, and
a light chain variable region comprising
a light chain CDR 1 comprising the amino acid sequence of SEQ ID NO: 30,
a light chain CDR 2 comprising the amino acid sequence of SEQ ID NO: 31, and
a light chain CDR 3 comprising the amino acid sequence of SEQ ID NO: 32;

(a2) an antibody variable region that comprises

a heavy chain variable region comprising
a heavy chain complementarity determining region (CDR) 1 comprising the amino acid sequence of SEQ ID NO: 33,
a heavy chain CDR 2 comprising the amino acid sequence of SEQ ID NO: 34, and
a heavy chain CDR 3 comprising the amino acid sequence of SEQ ID NO: 35, and
a light chain variable region comprising
a light chain CDR 1 comprising the amino acid sequence of SEQ ID NO: 30,
a light chain CDR 2 comprising the amino acid sequence of SEQ ID NO: 31, and
a light chain CDR 3 comprising the amino acid sequence of SEQ ID NO: 32;

(a3) an antibody variable region that binds to the same epitope as the epitope bound by the antibody variable region of (a1) or (a2); and
(a4) an antibody variable region that competes for binding to an antigen with the antibody variable region of (a1) or (a2).

In certain embodiments, the first and second antigen-binding domains of the antigen-binding molecule of the present disclosure each comprises an antibody variable region that can be the same or different and is independently selected from the group consisting of the following (a1) to (a4):

(a1) an antibody variable region that comprises

a heavy chain variable region comprising the amino acid sequence of SEQ ID NO: 36, and
a light chain variable region comprising the amino acid sequence of SEQ ID NO: 37;

(a2) an antibody variable region that comprises

a heavy chain variable region comprising the amino acid sequence of SEQ ID NO: 38, and
a light chain variable region comprising the amino acid sequence of SEQ ID NO: 37;

(a3) an antibody variable region that binds to the same epitope as the epitope bound by the antibody variable region of (a1) or (a2); and
(a4) an antibody variable region that competes for binding to an antigen with the antibody variable region of (a1) or (a2).

In one embodiment the Dual antigen-binding domain ("first antigen-binding domain" or "second antigen-binding domain" or "Dual-Fab") comprises a heavy chain variable region sequence comprising an amino acid sequence that is at least about 95%, about 96%, about 97%, about 98%, about 99%, or about 100% identical to the amino acid sequence of SEQ ID NO: 36 and a light chain variable region sequence comprising an amino acid sequence that is at least about 95%, about 96%, about 97%, about 98%, about 99%, or about 100% identical to the amino acid sequence of SEQ ID NO: 37. In one embodiment the Dual antigen-binding domain ("first antigen-binding domain" or "second antigen-binding domain" or "Dual-Fab") comprises a heavy chain variable region comprising the amino acid sequence of SEQ ID NO: 36 and a light chain variable region comprising the amino acid sequence of SEQ ID NO: 37.

In one embodiment, the Dual antigen-binding domain ("first antigen-binding domain" or "second antigen-binding domain" or "Dual-Fab") comprises a heavy chain variable region sequence comprising an amino acid sequence that is at least about 95%, about 96%, about 97%, about 98%, about 99%, or about 100% identical to the amino acid sequence of SEQ ID NO: 38 and a light chain variable region sequence comprising an amino acid sequence that is at least about 95%, about 96%, about 97%, about 98%, about 99%, or about 100% identical to the amino acid sequence of SEQ ID NO: 37. In one embodiment the Dual antigen-binding domain ("first antigen-binding domain" or "second antigen-binding domain" or "Dual-Fab") comprises a heavy chain variable region comprising the amino acid sequence of SEQ ID NO: 38 and a light chain variable region comprising the amino acid sequence of SEQ ID NO: 37.

The antigen-binding molecule described herein comprises at least one antigen-binding domain capable of binding to Delta-like 3 (DLL3) (also referred to herein as "DLL3 antigen-binding domain", "third antigen-binding domain", or "antigen-binding domain capable of binding to DLL3").

In a specific embodiment, the antigen-binding molecule comprises one antigen-binding domain capable of binding to DLL3. In a specific embodiment, the antigen-binding molecule comprises two antigen-binding domains capable of binding to DLL3. In certain such embodiments, each of these antigen-binding domains specifically binds to the same epitope of DLL3. In an even more specific embodiment, all of these "DLL3 antigen-binding domains" are identical. In one embodiment, the antigen-binding molecule comprises an immunoglobulin molecule capable of specifically binding to DLL3. In one embodiment, the antigen-binding molecule comprises not more than two antigen-binding domains capable of binding to DLL3.

In a specific embodiment, the DLL3 antigen-binding domain is a crossover Fab molecule, more specifically a DLL3 molecule in which either the variable regions or the constant regions of the Fab light chain and the Fab heavy chain are exchanged. In a specific embodiment, the DLL3 antigen-binding domain is a crossover Fab molecule in which the variable regions of the Fab light chain and the Fab heavy chain are exchanged.

In certain embodiments, as the third antigen-binding domain, the antigen-binding molecule of the present disclosure may comprise an antibody variable region independently selected from the group consisting of any one of the following (a1) to (a4):

(a1) an antibody variable region that comprises

a heavy chain variable region comprising
a heavy chain complementarity determining region (CDR) 1 comprising the amino acid sequence of SEQ ID NO: 46,
a heavy chain CDR 2 comprising the amino acid sequence of SEQ ID NO: 47, and
a heavy chain CDR 3 comprising the amino acid sequence of SEQ ID NO: 48, and
a light chain variable region comprising
a light chain CDR 1 comprising the amino acid sequence of SEQ ID NO: 49,
a light chain CDR 2 comprising the amino acid sequence of SEQ ID NO: 50, and
a light chain CDR 3 comprising the amino acid sequence of SEQ ID NO: 51;

(a2) an antibody variable region that comprises

a heavy chain variable region comprising the amino acid sequence of SEQ ID NO: 52, and
a light chain variable region comprising the amino acid sequence of SEQ ID NO: 53;

(a3) an antibody variable region that binds to the same epitope as the epitope bound by the antibody variable region of (a1) or (a2); and
(a4) an antibody variable region that competes for binding to an antigen with the antibody variable region of (a1) or (a2).

In one embodiment the DLL3 antigen-binding domain comprises a heavy chain variable region sequence comprising an amino acid sequence that is at least about 95%, about 96%, about 97%, about 98%, about 99%, or about 100% identical to the amino acid sequence of SEQ ID NO: 52 and a light chain variable region sequence comprising an amino acid sequence that is at least about 95%, about 96%, about 97%, about 98%, about 99%, or about 100% identical to the amino acid sequence of SEQ ID NO: 53. In one embodiment the DLL3 antigen-binding domain comprises a heavy chain variable region comprising the amino acid sequence of SEQ ID NO: 52 and a light chain variable region comprising the amino acid sequence of SEQ ID NO: 53.

The antigen-binding molecule of the present disclosure can further comprise an Fc region. When each of the first and second antigen-binding domain is a Fab, the first antigen-binding domain may be fused at the C-terminus of the Fab heavy chain to the N-terminus of the first or second subunit of the Fc region, and the second antigen-binding domain may be fused at the C-terminus of the Fab heavy chain to the N-terminus of the remaining subunit of the Fc domain. In some embodiments, the third antigen-binding domain is fused at the C-terminus to the N-terminus of the Fab heavy chain of one of either the first antigen-binding domain or the second antigen-binding domain, optionally via a peptide linker.

According to any of the above embodiments, components of the antigen binding molecules (e.g. antigen binding domain, Fc region) may be fused directly or through various linkers, particularly peptide linkers comprising one or more amino acids, typically about 2-20 amino acids, that are described herein or are known in the art. Suitable, non-immunogenic peptide linkers include, for example, (G4S)n, (SG4)n, G4(G4S)n or G4(SG4)n peptide linkers, wherein n is generally a number between 1 and 10, typically between 2 and 4.

In certain embodiments, the Fc region may be an Fc region which comprises a first Fc region subunit and a second Fc region subunit, wherein the first Fc region subunit is selected from the group consisting of:

an Fc region polypeptide comprising alanine at each of positions 234 and 235;
an Fc region polypeptide comprising alanine at each of positions 234, 235, and 297; and
an Fc region polypeptide comprising alanine at each of positions 234, 235, and 297, cysteine at position 354, and tryptophan at position 366; and

wherein the second Fc region subunit is selected from the group consisting of:

an Fc region polypeptide comprising alanine at each of positions 234 and 235;
an Fc region polypeptide comprising alanine at each of positions 234, 235, and 297; and
an Fc region polypeptide comprising alanine at each of positions 234, 235, and 297, cysteine at position 349, serine at position 366, alanine at position 368, and valine at position 407.

In a specific embodiment, the Fc region preferably comprises either one of the following:

(a) a first Fc subunit comprising the amino acid sequence shown in SEQ ID NO: 23 and a second Fc subunit comprising the amino acid sequence shown in SEQ ID NO: 24;
(b) a first Fc subunit comprising the amino acid sequence shown in SEQ ID NO: 25 and a second Fc subunit comprising the amino acid sequence shown in SEQ ID NO: 26; and
(c) a first Fc region subunit comprising the amino acid sequence shown in SEQ ID NO: 58 and a second Fc region subunit comprising the amino acid sequence shown in SEQ ID NO: 59.

In one embodiment, the antigen-binding molecule of the present disclosure may comprise a polypeptide chain (chain 1) comprising an amino acid sequence that is at least about 95%, about 96%, about 97%, about 98%, about 99%, or about 100% identical to the amino acid sequence of SEQ ID NO: 39, a polypeptide chain (chain 2) comprising an amino acid sequence that is at least about 95%, about 96%, about 97%, about 98%, about 99%, or about 100% identical to the amino acid sequence of SEQ ID NO: 40, a polypeptide chain (chain 3) comprising an amino acid sequence that is at least about 95%, about 96%, about 97%, about 98%, about 99%, or about 100% identical to the amino acid sequence of SEQ ID NO: 41, and two polypeptide chains (chain 4 and chain 5) comprising an amino acid sequence that is at least about 95%, about 96%, about 97%, about 98%, about 99%, or about 100% identical to the amino acid sequence of SEQ ID NO: 42.

In one embodiment, the antigen-binding molecule of the present disclosure may comprise a polypeptide chain (chain 1) comprising an amino acid sequence that is at least about 95%, about 96%, about 97%, about 98%, about 99%, or about 100% identical to the amino acid sequence of SEQ ID NO: 43, a polypeptide chain (chain 2) comprising an amino acid sequence that is at least about 95%, about 96%, about 97%, about 98%, about 99%, or about 100% identical to the amino acid sequence of SEQ ID NO: 40, a polypeptide chain (chain 3) comprising an amino acid sequence that is at least about 95%, about 96%, about 97%, about 98%, about 99%, or about 100% identical to the amino acid sequence of SEQ ID NO: 44, and two polypeptide chains (chain 4 and chain 5) comprising an amino acid sequence that is at least about 95%, about 96%, about 97%, about 98%, about 99%, or about 100% identical to the amino acid sequence of SEQ ID NO: 42.

In one embodiment, the antigen-binding molecule of the present disclosure may comprise a polypeptide chain (chain 1) comprising an amino acid sequence that is at least about 95%, about 96%, about 97%, about 98%, about 99%, or about 100% identical to the amino acid sequence of SEQ ID NO: 45, a polypeptide chain (chain 2) comprising an amino acid sequence that is at least about 95%, about 96%, about 97%, about 98%, about 99%, or about 100% identical to the amino acid sequence of SEQ ID NO: 40, a polypeptide chain (chain 3) comprising an amino acid sequence that is at least about 95%, about 96%, about 97%, about 98%, about 99%, or about 100% identical to the amino acid sequence of SEQ ID NO: 44, and two polypeptide chains (chain 4 and chain 5) comprising an amino acid sequence that is at least about 95%, about 96%, about 97%, about 98%, about 99%, or about 100% identical to the amino acid sequence of SEQ ID NO: 42.

In an aspect, the fourth polypeptide (chain 4) and the fifth polypeptide (chain 5) are identical.

In certain embodiments, the antigen-binding molecule of the present disclosure may comprise five polypeptide chains in any one of the combinations selected from the group consisting of the following:

(a1) a polypeptide chain comprising the amino acid sequence of SEQ ID NO: 39 (chain 1),

a polypeptide chain comprising the amino acid sequence of SEQ ID NO: 40 (chain 2),
a polypeptide chain comprising the amino acid sequence of SEQ ID NO: 41 (chain 3), and
two polypeptide chains each comprising the amino acid sequence of SEQ ID NO: 42 (chain 4 and chain 5);

(a2) a polypeptide chain comprising the amino acid sequence of SEQ ID NO: 43 (chain 1),

a polypeptide chain comprising the amino acid sequence of SEQ ID NO: 40 (chain 2),
a polypeptide chain comprising the amino acid sequence of SEQ ID NO: 44 (chain 3), and
two polypeptide chains each comprising the amino acid sequence of SEQ ID NO: 42 (chain 4 and chain 5); and

(a3) a polypeptide chain comprising the amino acid sequence of SEQ ID NO: 45 (chain 1),

a polypeptide chain comprising the amino acid sequence of SEQ ID NO: 40 (chain 2),
a polypeptide chain comprising the amino acid sequence of SEQ ID NO: 44 (chain 3), and
two polypeptide chains each comprising the amino acid sequence of SEQ ID NO: 42 (chain 4 and chain 5);

and preferably, the five polypeptide chains (chain 1 to chain 5) connect and/or associate with each other according to the orientation shown in Fig. 3.

In certain embodiments, the antigen-binding molecule of the present disclosure may comprise the following five polypeptide chains:

a polypeptide chain comprising the amino acid sequence of SEQ ID NO: 54 (chain 1),
a polypeptide chain comprising the amino acid sequence of SEQ ID NO: 55 (chain 2),
a polypeptide chain comprising the amino acid sequence of SEQ ID NO: 56 (chain 3), and
two polypeptide chains each comprising the amino acid sequence of SEQ ID NO: 57 (chain 4 and chain 5);

and preferably, the five polypeptide chains (chain 1 to chain 5) connect and/or associate with each other according to the orientation shown in Fig. 3.

In certain embodiments, the antigen-binding molecule of the present disclosure may comprise the following five polypeptide chains:

a polypeptide chain comprising the amino acid sequence of SEQ ID NO: 54 (chain 1),
a polypeptide chain comprising the amino acid sequence of SEQ ID NO: 55 (chain 2),
a polypeptide chain comprising the amino acid sequence of SEQ ID NO: 60 (chain 3), and
two polypeptide chains each comprising the amino acid sequence of SEQ ID NO: 57 (chain 4 and chain 5);

and preferably, the five polypeptide chains (chain 1 to chain 5) connect and/or associate with each other according to the orientation shown in Fig. 3.

In certain embodiments, the antigen binding molecule described herein binds to an epitope of CD3, CD137 or DLL3 that is conserved among the CD3, CD137 or DLL3 from different species. In certain embodiments, the antigen binding molecule in the present disclosure is a trispecific antigen binding molecule, i.e. it is capable of specifically binding to three different antigens - capable of binding to either one of CD3 or CD137 but does not bind to both antigens simultaneously, and is capable of specifically binding to DLL3.

Whether the antibody variable region in the present disclosure is capable of binding to antigens such as CD3, CD137, and DLL can be determined by a method known in the art.

This can be determined by, for example, an electrochemiluminescence method (ECL method) (BMC Research Notes 2011, 4: 281).

Specifically, for example, a low-molecular antibody composed of a region capable of binding to an antigen, for example, a Fab region, of a biotin-labeled antigen-binding molecule to be tested, or a monovalent antibody (antibody lacking one of the two Fab regions carried by a usual antibody) thereof is mixed with an antigen labeled with sulfo-tag (Ru complex), and the mixture is added onto a streptavidin-immobilized plate. In this operation, the biotin-labeled antigen-binding molecule to be tested binds to streptavidin on the plate. Light is developed from the sulfo-tag, and the luminescence signal can be detected using Sector Imager 600 or 2400 (MSD K.K.) or the like to thereby confirm the binding of the aforementioned region of the antigen-binding molecule to be tested to the antigen.

Alternatively, this assay may be conducted by ELISA, FACS (fluorescence activated cell sorting), ALPHAScreen (amplified luminescent proximity homogeneous assay screen), the BIACORE method based on a surface plasmon resonance (SPR) phenomenon, etc. (Proc. Natl. Acad. Sci. USA (2006) 103 (11), 4005-4010).

Specifically, the assay can be conducted using, for example, an interaction analyzer Biacore (Cytiva Japan Corp.) based on a surface plasmon resonance (SPR) phenomenon. The Biacore analyzer includes any model such as Biacore T100, T200, X100, A100, 4000, 3000, 2000, 1000, or C. Any sensor chip for Biacore, such as a CM7, CM5, CM4, CM3, C1, SA, NTA, L1, HPA, or Au chip, can be used as a sensor chip. Proteins for capturing the antigen-binding molecule in the present disclosure, such as protein A, protein G, protein L, anti-human IgG antibodies, anti-human IgG-Fab, anti-human L chain antibodies, anti-human Fc antibodies, antigenic proteins, or antigenic peptides, are immobilized onto the sensor chip by a coupling method such as amine coupling, disulfide coupling, or aldehyde coupling. An antigen is injected thereon as an analyte, and the interaction is measured to obtain a sensorgram. In this operation, the concentration of the antigen can be selected within the range of a few micro M to a few pM according to the interaction strength (e.g., KD) of the assay sample.

Alternatively, an antigen may be immobilized instead of the antigen-binding molecule onto the sensor chip, and then the antibody sample to be evaluated may be allowed to interact with it. Whether the antibody variable region of the antigen-binding molecule in the present disclosure has binding activity against the antigen can be confirmed on the basis of a dissociation constant (KD) value calculated from the sensorgram of the interaction or on the basis of the degree of increase in the sensorgram after the action of the antigen-binding molecule sample over the level before the action.

In some embodiments, binding activity or affinity of the antibody variable region in the present disclosure to the antigen of interest is assessed at 37°C (e.g., for CD137) or 25°C (e.g., for CD3) using e.g., Biacore T200 instrument (Cytiva) or Biacore 8K instrument (Cytiva). Anti-human Fc (e.g., Cytiva) is immobilized onto all flow cells of a CM4 sensor chip using amine coupling kit (e.g, Cytiva). The antigen binding molecules or antibody variable regions are captured onto the anti-Fc sensor surfaces, then the antigen is injected over the flow cell. The capture levels of the antigen binding molecules or antibody variable regions may be aimed at 200 resonance unit (RU). A recombinant human antigen may be injected at 2000 to 125 nM prepared by two-fold serial dilution, followed by dissociation. All antigen binding molecules or antibody variable regions and analytes are prepared in ACES pH 7.4 containing 20 mM ACES, 150 mM NaCl, 0.05% Tween 20, 0.005% NaN3. Sensor surface is regenerated each cycle with 3M $MgCl_2$. Binding affinity is determined by processing and fitting the data to 1:1 binding model using e.g., Biacore Insight Evaluation software, version 2.0 (Cytiva) or Biacore 8K Evaluation software (Cytiva). The KD values are calculated for assessing the specific binding activity or affinity of the antigen binding domains in the present disclosure.

The ALPHAScreen is carried out by the ALPHA technology using two types of beads (donor and acceptor) on the basis of the following principle: luminescence signals are detected only when these two beads are located in proximity through the biological interaction between a molecule bound with the donor bead and a molecule bound with the acceptor bead. A laser-excited photosensitizer in the donor bead converts ambient oxygen to singlet oxygen having an excited state. The singlet oxygen diffuses around the donor bead and reaches the acceptor bead located in proximity thereto to thereby cause chemiluminescent reaction in the bead, which finally emits light. In the absence of the interaction between the molecule bound with the donor bead and the molecule bound with the acceptor bead, singlet oxygen produced by the donor bead does not reach the acceptor bead. Thus, no chemiluminescent reaction occurs.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is injected on the surface of the sensor chip. Upon binding of the analyte to the ligand, the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). The amount of the analyte bound to the ligand captured on the sensor chip surface (amount of change in response on the sensorgram between before and after the interaction of the analyte) can be determined from the sensorgram. However, since the amount bound also depends on the amount of the ligand, the comparison must be performed under conditions where substantially the same amounts of the ligand are used. Kinetics, i.e., an association rate constant (ka) and a dissociation rate constant (kd), can be determined from the curve of the sensorgram, while affinity (KD) can be determined from the ratio between these constants. Inhibition assay is also preferably used in the BIACORE method. Examples of the inhibition assay are described in Proc. Natl. Acad. Sci. USA (2006) 103 (11), 4005-4010.

The term "does not bind to CD3 and CD137 (4-1BB) at the same time" or "does not bind to CD3 and CD137 (4-1BB) simultaneously" means that the antigen-binding domain or antibody variable region in the present disclosure cannot bind to CD137 in a state bound with CD3 whereas the antigen-binding domain or antibody variable region cannot bind to CD3 in a state bound with CD137. In this context, the phrase "not bind to CD3 and CD137 at the same time" also includes not cross-linking a cell expressing CD3 to a cell expressing CD137, or not binding to CD3 and CD137 each expressed on a different cell, at the same time. This phrase further includes the case where the variable region is capable of binding to both CD3 and CD137 at the same time when CD3 and CD137 are not expressed on cell membranes, as with soluble proteins, or both reside on the same cell, but cannot bind to CD3 and CD137 each expressed on a different cell, at the same time. Such an antibody variable region is not particularly limited as long as the antibody variable region has these functions. Examples thereof can include variable regions derived from an IgG-type antibody variable region by the alteration of a portion of its amino acids so as to bind to the desired antigen. The amino acid to be altered is selected from, for example, amino acids whose alteration does not cancel the binding to the antigen, in an antibody variable region binding to CD3 or CD137.

In this context, the phrase "expressed on different cells" merely means that the antigens are expressed on separate cells. The combination of such cells may be, for example, the same types of cells such as a T cell and another T cell, or may be different types of cells such as a T cell and an NK cell.

Whether the antigen-binding molecule in the present disclosure does "not bind to CD3 and CD137 at the same time" can be confirmed by: confirming the antigen-binding molecule to have binding activity against both CD3 and CD137; then allowing either CD3 or CD137 to bind in advance to the antigen-binding molecule comprising the variable region having this binding activity; and then determining the presence or absence of its binding activity against the other one by the method mentioned above. Alternatively, this can also be confirmed by determining whether the binding of the antigen-binding molecule to either CD3 or CD137 immobilized on an ELISA plate or a sensor chip is inhibited by the addition of the other one into the solution. In some embodiments, the binding of the antigen-binding molecule in the present disclosure to either CD3 or CD137 is inhibited by binding of the antigen-binding molecule to the other by at least 50%, preferably 60% or more, more preferably 70% or more, more preferably 80% or more, further preferably 90% or more, or even more preferably 95% or more.

In one aspect, while one antigen (e.g. CD3) is immobilized, the inhibition of the binding of the antigen-binding molecule to CD3 can be determined in the presence of the other antigen (e.g. CD137) by methods known in prior art (i.e. ELISA, BIACORE, and so on). In another aspect, while CD137 is immobilized, the inhibition of the binding of the antigen-binding molecule to CD137 also can be determined in the presence of CD3. When either one of two aspects mentioned above is conducted, the antigen-binding molecule in the present disclosure is determined not to bind to CD3 and CD137 at the same time if the binding is inhibited by at least 50%, preferably 60% or more, preferably 70% or more, further preferably 80% or more, further preferably 90% or more, or even more preferably 95% or more.

In some embodiments, the concentration of the antigen injected as an analyte is at least 1-fold, 2-fold, 5-fold, 10-fold, 30-fold, 50-fold, or 100-fold higher than the concentration of the other antigen to be immobilized.

In preferable manner, the concentration of the antigen injected as an analyte is 100-fold higher than the concentration of the other antigen to be immobilized and the binding is inhibited by at least 80%.

In one embodiment, the ratio of the KD value for the CD3 (analyte)-binding activity of the antigen-binding molecule to the CD137 (immobilized)-binding activity of the antigen-binding molecule (KD (CD3)/ KD (CD137)) is calculated and the CD3 (analyte) concentration which is 10-fold, 50-fold, 100-fold, or 200-fold of the ratio of the KD value (KD(CD3)/KD(CD137) higher than the CD137 (immobilized) concentration can be used for the competition measurement above. (e.g. 1-fold, 5-fold, 10-fold, or 20-fold higher concentration can be selected when the ratio of the KD value is 0.1. Furthermore, 100-fold, 500-fold, 1000-fold, or 2000-fold higher concentration can be selected when the ratio of the KD value is 10. )

In one aspect, while one antigen (e.g. CD3) is immobilized, the attenuation of the binding signal of the antigen-binding molecule to CD3 can be determined in the presence of the other antigen (e.g. CD137) by methods known in prior art (i.e. ELISA, ECL and so on). In another aspect, while CD137 is immobilized, the attenuation of the binding signal of the antigen-binding molecule to CD137 also can be determined in the presence of CD3. When either one of two aspects mentioned above is conducted, the antigen-binding molecule in the present disclosure is determined not to bind to CD3 and CD137 at the same time if the binding signal is attenuated by at least 50%, preferably 60% or more, preferably 70% or more, further preferably 80% or more, further preferably 90% or more, or even more preferably 95% or more.

In some embodiments, the concentration of the antigen injected as an analyte is at least 1-fold, 2-fold, 5-fold, 10-fold, 30-fold, 50-fold, or 100-fold higher than the concentration of the other antigen to be immobilized.

In preferable manner, the concentration of the antigen injected as an analyte is 100-fold higher than the concentration of the other antigen to be immobilized and the binding is inhibited by at least 80%.

In one embodiment, the ratio of the KD value for the CD3 (analyte)-binding activity of the antigen-binding molecule to the CD137 (immobilized)-binding activity of the antigen-binding molecule (KD (CD3)/ KD (CD137)) is calculated and the CD3 (analyte) concentration which is 10-fold, 50-fold, 100-fold, or 200-fold of the ratio of the KD value (KD(CD3)/KD(CD137) higher than the CD137 (immobilized) concentration can be used for the measurement above. (e.g. 1-fold, 5-fold, 10-fold, or 20-fold higher concentration can be selected when the ratio of the KD value is 0.1. Furthermore, 100-fold, 500-fold, 1000-fold, or 2000-fold higher concentration can be selected when the ratio of the KD value is 10. )

Specifically, in the case of using, for example, the ECL method, a biotin-labeled antigen-binding molecule to be tested, CD3 labeled with sulfo-tag (Ru complex), and an unlabeled CD137 are prepared. When the antigen-binding molecule to be tested is capable of binding to CD3 and CD137, but does not bind to CD3 and CD137 at the same time, the luminescence signal of the sulfo-tag is detected in the absence of the unlabeled CD137 by adding the mixture of the antigen-binding molecule to be tested and labeled CD3 onto a streptavidin-immobilized plate, followed by light development. By contrast, the luminescence signal is decreased in the presence of unlabeled CD137. This decrease in luminescence signal can be quantified to determine relative binding activity. This analysis may be similarly conducted using the labeled CD137 and the unlabeled CD3.

In the case of the ALPHAScreen, the antigen-binding molecule to be tested interacts with CD3 in the absence of the competing CD137 to generate signals of 520 to 620 nm. The untagged CD137 competes with CD3 for the interaction with the antigen-binding molecule to be tested. Decrease in fluorescence caused as a result of the competition can be quantified to thereby determine relative binding activity. The polypeptide biotinylation using sulfo-NHS-biotin or the like is known in the art. CD3 can be tagged with GST by an appropriately adopted method which involves, for example: fusing a polynucleotide encoding CD3 in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis. This analysis may be similarly conducted using the tagged CD137 and the untagged CD3.

Alternatively, a method using fluorescence resonance energy transfer (FRET) may be used. FRET is a phenomenon in which excitation energy is transferred directly between two fluorescent molecules located in proximity to each other by electron resonance. When FRET occurs, the excitation energy of a donor (fluorescent molecule having an excited state) is transferred to an acceptor (another fluorescent molecule located near the donor) so that the fluorescence emitted from the donor disappears (to be precise, the lifetime of the fluorescence is shortened) and instead, the fluorescence is emitted from the acceptor. By use of this phenomenon, whether or not bind to CD3 and CD137 at the same time can be analyzed. For example, when CD3 carrying a fluorescence donor and CD137 carrying a fluorescence acceptor bind to the antigen-binding molecule to be tested at the same time, the fluorescence of the donor disappears while the fluorescence is emitted from the acceptor. Therefore, change in fluorescence wavelength is observed. Such an antibody is confirmed to bind to CD3 and CD137 at the same time. On the other hand, if the mixing of CD3, CD137, and the antigen-binding molecule to be tested does not change the fluorescence wavelength of the fluorescence donor bound with CD3, this antigen-binding molecule to be tested can be regarded as antigen binding domain that is capable of binding to CD3 and CD137, but does not bind to CD3 and CD137 at the same time.

For example, a biotin-labeled antigen-binding molecule to be tested is allowed to bind to streptavidin on the donor bead, while CD3 tagged with glutathione S transferase (GST) is allowed to bind to the acceptor bead. The antigen-binding molecule to be tested interacts with CD3 in the absence of the competing second antigen to generate signals of 520 to 620 nm. The untagged second antigen competes with CD3 for the interaction with the antigen-binding molecule to be tested. Decrease in fluorescence caused as a result of the competition can be quantified to thereby determine relative binding activity. The polypeptide biotinylation using sulfo-NHS-biotin or the like is known in the art. CD3 can be tagged with GST by an appropriately adopted method which involves, for example: fusing a polynucleotide encoding CD3 in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis.

The tagging is not limited to the GST tagging and may be carried out with any tag such as, but not limited to, a histidine tag, MBP, CBP, a Flag tag, an HA tag, a V5 tag, or a c-myc tag. The binding of the antigen-binding molecule to be tested to the donor bead is not limited to the binding using biotin-streptavidin reaction. Particularly, when the antigen-binding molecule to be tested comprises Fc, a possible method involves allowing the antigen-binding molecule to be tested to bind via an Fc-recognizing protein such as protein A or protein G on the donor bead.

Also, the case where the variable region is capable of binding to CD3 and CD137 at the same time when CD3 and CD137 are not expressed on cell membranes, as with soluble proteins, or both reside on the same cell, but cannot bind to CD3 and CD137 each expressed on a different cell, at the same time can also be assayed by a method known in the art.

Specifically, the antigen-binding molecule to be tested has been confirmed to be positive in ECL-ELISA for detecting binding to CD3 and CD137 at the same time is also mixed with a cell expressing CD3 and a cell expressing CD137. The antigen-binding molecule to be tested can be shown to be incapable of binding to CD3 and CD137 expressed on different cells, at the same time unless the antigen-binding molecule and these cells bind to each other at the same time. This assay can be conducted by, for example, cell-based ECL-ELISA. The cell expressing CD3 is immobilized onto a plate in advance. After binding of the antigen-binding molecule to be tested thereto, the cell expressing CD137 is added to the plate. A different antigen expressed only on the cell expressing CD137 is detected using a sulfo-tag-labeled antibody against this antigen. A signal is observed when the antigen-binding molecule binds to the two antigens respectively expressed on the two cells, at the same time. No signal is observed when the antigen-binding molecule does not bind to these antigens at the same time.

Alternatively, this assay may be conducted by the ALPHAScreen method. The antigen-binding molecule to be tested is mixed with a cell expressing CD3 bound with the donor bead and a cell expressing CD137 bound with the acceptor bead. A signal is observed when the antigen-binding molecule binds to the two antigens expressed on the two cells respectively, at the same time. No signal is observed when the antigen-binding molecule does not bind to these antigens at the same time.

Alternatively, this assay may also be conducted by an Octet interaction analysis method. First, a cell expressing CD3 tagged with a peptide tag is allowed to bind to a biosensor that recognizes the peptide tag. A cell expressing CD137 and the antigen-binding molecule to be tested are placed in wells and analyzed for interaction. A large wavelength shift caused by the binding of the antigen-binding molecule to be tested and the cell expressing CD137 to the biosensor is observed when the antigen-binding molecule binds to the two antigens expressed on the two cells respectively, at the same time. A small wavelength shift caused by the binding of only the antigen-binding molecule to be tested to the biosensor is observed when the antigen-binding molecule does not bind to these antigens at the same time.

Instead of these methods based on the binding activity, assay based on biological activity may be conducted. For example, a cell expressing CD3 and a cell expressing CD137 are mixed with the antigen-binding molecule to be tested, and cultured. The two antigens expressed on the two cells respectively are mutually activated via the antigen-binding molecule to be tested when the antigen-binding molecule binds to these two antigens at the same time. Therefore, change in activation signal, such as increase in the respective downstream phosphorylation levels of the antigens, can be detected. Alternatively, cytokine production is induced as a result of the activation. Therefore, the amount of cytokines produced can be measured to thereby confirm whether or not to bind to the two cells at the same time. Alternatively, cytotoxicity against a cell expressing CD137 is induced as a result of the activation. Alternatively, the expression of a reporter gene is induced by a promoter which is activated at the downstream of the signal transduction pathway of CD137 or CD3 as a result of the activation. Therefore, the cytotoxicity or the amount of reporter proteins produced can be measured to thereby confirm whether or not to bind to the two cells at the same time.

In another aspect, competition assays may be used to identify an antigen-binding molecule that competes with a certain reference antigen-binding molecule for binding to CD3 and CD137. In certain embodiments, such an antigen-binding molecule binds to the same site on CD3 and CD137 (epitope, for example, a linear or a conformational epitope) as that bound by the reference antigen-binding molecule. A detailed illustrative method for mapping the epitope to which a polypeptide binds is provided by Morris (1996) "Epitope Mapping Protocols" in Methods in Molecular Biology vol. 66 (Humana Press, Totowa, NJ). By analyzing the site on the antigen (epitope) to which the reference antigen-binding molecule binds using the epitope mapping method, and by using the peptide containing the identified epitope, an antigen-binding molecule that binds thereto may be prepared and thereby an antigen-binding molecule that binds to the same site as the site on the antigen to which the reference antigen-binding molecule binds can be obtained.

An exemplary competition assay is described below. An immobilized antigen (for example, CD3 and CD137) is incubated in a solution comprising a first labeled antigen-binding molecule (substance) that binds to the antigen and a second unlabeled antigen-binding molecule (substance) to be tested for its ability to compete with the first substance in binding to the antigen. As a control, the immobilized antigen is incubated in a solution comprising the first labeled substance but not the second unlabeled substance. After incubation under conditions that allow binding of the first substance to the antigen, excess unbound substances are removed and the amount of label bound to the immobilized antigen is measured. If the amount of label bound to the immobilized antigen is substantially reduced in the test sample as compared to the control sample, it indicates that the second molecule competes with the first substance in binding to the antigen. See Harlow and Lane (1988) Antibodies: A Laboratory Manual ch.14 (Cold Spring Harbor Laboratory, Cold Spring Harbor, NY).

Another exemplary competitive assay uses BIACORE (registered trademark) analysis to determine the ability of a test substance to compete with a second (reference) substance in binding to the antigen. In a further aspect of operating a BIACORE (registered trademark) instrument (for example, BIACORE (registered trademark) 3000) according to the manufacturer's recommendations, the antigen is captured on a CM5 BIACORE (registered trademark) chip using standard techniques known in the art to produce a surface coated with the antigen. Typically, 200-800 resonance units of the antigen are bound to the chip (an amount that provides an easily measurable level of binding but is easily saturable at the concentration of the test substance used). Two substances to be evaluated for their ability to compete with each other (i.e., test substance and reference substance) are mixed in a suitable buffer at a molar ratio of binding sites of 1:1 to produce a mixture. When calculating the concentration based on the binding site, the molecular weight of the test substance or reference substance is deemed as that obtained by dividing the total molecular weight of the corresponding substance by the number of antigen-binding sites on the substance. The concentration of each of the substances (i.e., test substance and reference substance) in the mixture must be sufficiently high to easily saturate the binding sites of the substances on the antigen molecules captured on the BIACORE (registered trademark) chip. The test substance and reference substance in the mixture are at the same molar concentration (based on binding), typically 1.00-1.5 micromolar (based on binding site). Separate solutions containing only the test substance and only the reference substance are also prepared. The test substance and reference substance in these solutions are in the same buffer as the mixture and they may have the same concentration and conditions. The mixture containing the test substance and reference substance is passed over a BIACORE (registered trademark) chip coated with the antigen and the total amount of binding is recorded. The chip is then treated to remove the bound test substance or reference substance without damaging the antigen bound to the chip. Typically, this is done by treating the chip with 30 mM HCl for 60 seconds. Thereafter, a solution containing only the test substance is passed over the surface coated with the antigen, and the amount of binding is recorded. Again, the chip is treated to remove all of the bound substances without damaging the antigen bound to the chip. Thereafter, a solution containing only the reference substance is passed over the surface coated with the antigen, and the amount of binding is recorded. Next, the theoretical maximum binding value of the mixture containing the test substance and reference substance is calculated, which is the sum of the binding of each substance (i.e., test and reference) when it passes alone over the antigen surface. If the actually recorded binding of the mixture is smaller than this theoretical maximum value, the test substance and reference substance are competing with each other in binding to the antigen. Therefore, in general, a competing test substance is a substance that binds to the antigen in the assay so that in the presence of the reference substance during the above-described BIACORE (registered trademark) blocking assay, the recorded binding is between 80% and 0.1% (for example, 80% > to 4%) of the theoretical maximum binding value (as defined above) for the test substance and reference substance in combination, and in particular, between 75% and 0.1% (for example, 75% to 4%) of the theoretical maximum binding value, and more specifically, between 70% and 0.1% (for example, 70% to 4%) of the theoretical maximum binding value.

Another exemplary competitive assay uses BIACORE (registered trademark) analysis to determine the ability of a test substance to compete with a second (reference) substance in binding to the antigen. In a further aspect of operating a BIACORE (registered trademark) instrument (for example, BIACORE (registered trademark) 3000) according to the manufacturer's recommendations, the reference substance is captured on a BIACORE (registered trademark) chip using standard techniques known in the art, to create a surface coated with the reference substance. Typically, 200-800 resonance units of the reference substance are bound to the chip (an amount that provides an easily measurable level of antigen binding). The antigen and a test substance to be evaluated for its ability to compete with the reference substance are mixed in an appropriate buffer to produce a mixture. A separate solution containing only the antigen is also prepared. The antigen in this solution is in the same buffer as the mixture and can be at the same concentration and conditions. A solution containing only the antigen is passed over a BIACORE (registered trademark) chip coated with the reference substance and the total amount of binding is recorded. For chip regeneration, the chip is treated to remove the reference substance coated on the chip surface. For example, when the reference substance is coated via an antibody to a BIACORE (registered trademark) chip onto which Protein A is immobilized by covalent bonding, treatment is performed with 10 mM Glycine-HCl to remove the antibody bound to Protein A. The mixture containing the test substance and the antigen is then passed over a BIACORE (registered trademark) chip coated with the reference substance and the total amount of binding is recorded. When the total amount of binding recorded when passing the mixture containing the test substance and the antigen is smaller compared to the total amount of binding recorded when passing a solution containing only the antigen, the test substance and reference substance are competing substances. Although not limited, competition between a test substance and a reference substance may mean that the value obtained by dividing (the total amount of binding when a test mixture containing the test substance and the antigen is passed) by (the total amount of binding when a solution containing only the antigen is passed) is 0.8 or less, 0.7 or less, 0.6 or less, or 0.5 or less.

In one aspect, the antigen-binding molecule carried by a substrate includes embodiments produced by methods well-known to those skilled in the art as described below. Without limitation, the antigen-binding molecule can be produced using recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" (also called "knobs-in-holes" or "KiH") engineering (see, e.g., U.S. Patent No. 5,731,168). Antigen-binding molecules may also be made by engineering electrostatic steering effects for making antibody Fc-heterodimeric molecules (WO 2009/089004A1 ); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included in the antigen-binding molecules herein (see, e.g. US 2006/0025576A1).

Antigen-Binding Molecule Variants

In certain embodiments, amino acid sequence variants of the antigen-binding molecules in the present disclosure are also contemplated. For example, it may be desirable to improve the binding affinity and/or other biological properties of the antigen-binding molecule. Amino acid sequence variants of an antigen-binding molecule may be prepared by introducing appropriate modifications into the nucleotide sequence encoding the antigen-binding molecule, or by peptide synthesis. Such modifications include, for example, deletions from, and/or insertions into and/or substitutions of residues within the amino acid sequences of the antigen-binding molecule. Any combination of deletion, insertion, and substitution can be made to arrive at the final construct, provided that the final construct possesses the desired characteristics, e.g., antigen-binding.

a) Substitution, Insertion, and Deletion Variants

In certain embodiments, the antigen-binding molecules in the present disclosure include antigen-binding molecule variants having one or more amino acid substitutions. Sites of interest for substitutional mutagenesis include the HVRs and FRs. Conservative substitutions are shown the table below under the heading of "preferred substitutions." More substantial changes are provided in the table under the heading of "exemplary substitutions," and as further described below in reference to amino acid side chain classes. Amino acid substitutions may be introduced into an antigen-binding molecule of interest and the products screened for a desired activity, e.g., retained/improved antigen binding, decreased immunogenicity, or improved ADCC or CDC.

[Table 5]

| Original Residue | Exemplary Substitutions | Preferred Substitutions |
| --- | --- | --- |
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: norleucine, methionine (Met), alanine (Ala), valine (Val), leucine (Leu), isoleucine (Ile);
(2) neutral hydrophilic: cysteine (Cys), serine (Ser), threonine (Thr), asparagine (Asn), glutamine (Gln);
(3) acidic: aspartic acid (Asp), glutamic acid (Glu);
(4) basic: histidine (His), lysine (Lys), arginine (Arg);
(5) residues that influence chain orientation: glycine (Gly), proline (Pro);
(6) aromatic: tryptophan (Trp), tyrosine (Tyr), phenylalanine (Phe).

Non-conservative substitutions will entail exchanging a member of one of these classes for another class.

One type of substitutional variant involves substituting one or more hypervariable region residues of a parent antigen-binding molecule (e.g. a humanized or human antibody). Generally, the resulting variant(s) selected for further study will have modifications (e.g., improvements) in certain biological properties (e.g., increased affinity, reduced immunogenicity) relative to the parent antigen-binding molecule and/or will have substantially retained certain biological properties of the parent antigen-binding molecule. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antigen-binding molecule affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antigen-binding molecule variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antigen-binding molecule to bind antigen. For example, conservative alterations (e.g., conservative substitutions described herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antigen-binding molecule that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antigen-binding molecule with antigen is affected. Further substitutions may be introduced at the amino acid locations demonstrating functional sensitivity to the initial substitutions. Alternatively, or additionally, a crystal structure of a complex of antigens and an antigen-binding molecule may be analyzed to identify contact points between the antigen-binding molecule and antigen. Such contact residues and neighboring residues may be targeted or eliminated as candidates for substitution. Variants may be screened to determine whether they contain the desired properties.

In connection with an antigen-binding molecule in the present disclosure, examples of the desired property (activity) can include, but are not particularly limited to, binding activity, neutralizing activity, cytotoxic activity, agonist activity, antagonist activity, and enzymatic activity. The agonist activity is an activity of intracellularly transducing signals, for example, through the binding of an antibody to an antigen such as a receptor to induce change in some physiological activity. Examples of the physiological activity can include, but are not limited to, proliferative activity, survival activity, differentiation activity, transcriptional activity, membrane transport activity, binding activity, proteolytic activity, phosphorylating/dephosphorylating activity, redox activity, transfer activity, nucleolytic activity, dehydration activity, cell death-inducing activity, and apoptosis-inducing activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antigen-binding molecule with an N-terminal methionyl residue. Other insertional variants of the antigen-binding molecule include the fusion of an enzyme (e.g. for ADEPT) or a polypeptide which increases the plasma half-life of the antigen-binding molecule to the N- or C-terminus of the antigen-binding molecule.

b) Glycosylation variants

In certain embodiments, an antigen-binding molecule herein is altered to increase or decrease the extent to which the antigen-binding molecule is glycosylated. Addition or deletion of glycosylation sites to an antigen-binding molecule may be conveniently accomplished by altering the amino acid sequence such that one or more glycosylation sites is created or removed.

Where the antigen-binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antigen-binding molecule of the present disclosure may be made in order to create antigen-binding molecule variants with certain improved properties.

In one embodiment, antigen-binding molecule variants having a carbohydrate structure that lacks fucose attached (directly or indirectly) to an Fc region are also suitably used for the antigen-binding molecule in the present disclosure. For example, the amount of fucose in such antigen-binding molecule may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antigen-binding molecules. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antigen-binding molecule variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antigen-binding molecules include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antigen-binding molecule variants with bisected oligosaccharides, e.g., in which a biantennary oligosaccharide attached to the Fc region of the antigen-binding molecule is bisected by GlcNAc, are also further encompassed by the antigen-binding molecules in the present disclosure. Such antigen-binding molecule variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antigen-binding molecule variants with at least one galactose residue in the oligosaccharide attached to the Fc region are also encompassed by the antigen-binding molecules in the present disclosure. Such antigen-binding molecule variants may have improved CDC function. Such antigen-binding molecule variants are described, e.g., in WO 1997/30087 (Patel et al.); WO 1998/58964 (Raju, S.); and WO 1999/22764 (Raju, S.).

c) Fc region variants

In certain embodiments, one or more amino acid modifications may be introduced into the Fc region of an antigen-binding molecule herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antigen-binding molecule variant that possesses some but not all effector functions, which make it a desirable candidate for applications in which the half life of the antigen-binding molecule in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antigen-binding molecule lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1™ non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al. Proc. Nat'l Acad. Sci. USA 95:652-656 (1998). Clq binding assays may also be carried out to confirm that the antigen-binding molecule is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, e.g., Petkova, S.B. et al., Int'l. Immunol. 18(12):1759-1769 (2006)).

Antigen-binding molecules with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antigen-binding molecule variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antigen-binding molecule variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934Al (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

d) Cysteine engineered antigen-binding molecule variants

In certain embodiments, it may be desirable to create cysteine engineered antigen-binding molecules, e.g., "thioMAbs," in which one or more residues of an antigen-binding molecule are substituted with cysteine residues. In particular embodiments, the substituted residues occur at accessible sites of the antigen-binding molecule. By substituting those residues with cysteine, reactive thiol groups are thereby positioned at accessible sites of the antigen-binding molecule and may be used to conjugate the antigen-binding molecule to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen-binding molecules may be generated as described, e.g., in U.S. Patent No. 7,521,541.

e) Antigen-Binding Molecule Derivatives

In certain embodiments, an antigen-binding molecule herein may be further modified to contain additional nonproteinaceous moieties that are known in the art and readily available. The moieties suitable for derivatization of the antigen-binding molecule include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antigen-binding molecule may vary, and if more than one polymer are attached, they can be the same or different molecules. In general, the number and/or type of polymers used for derivatization can be determined based on considerations including, but not limited to, the particular properties or functions of the antigen-binding molecule to be improved, whether the antigen-binding molecule derivative will be used in a therapy under defined conditions, etc.

In another embodiment, an antigen-binding molecule may be a conjugate with a nonproteinaceous moiety that may be selectively heated by exposure to radiation. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antigen binding molecule-nonproteinaceous moiety are killed.

Recombinant Methods and Compositions

Antigen-binding molecules may be produced using recombinant methods and compositions, e.g., as described in U.S. Patent No. 4,816,567. In one embodiment, isolated nucleic acid encoding an antigen-binding molecule in the present disclosure (a polypeptide comprising an antigen-binding domain described herein) is provided. Such nucleic acid may encode an amino acid sequence comprising the VL and/or an amino acid sequence comprising the VH of the antigen-binding molecule (e.g., the light and/or heavy chains of the antigen-binding molecule). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antigen-binding molecule and an amino acid sequence comprising the VH of the antigen-binding molecule, or (2) a first vector comprising a nucleic acid that encodes an amino acid sequence comprising the VL of the antigen-binding molecule and a second vector comprising a nucleic acid that encodes an amino acid sequence comprising the VH of the antigen-binding molecule. In one embodiment, the host cell is eukaryotic, e.g. a Chinese Hamster Ovary (CHO) cell or lymphoid cell (e.g., Y0, NS0, Sp2/0 cell). In one embodiment, an antigen-binding molecule can be obtained through culturing a host cell comprising a nucleic acid encoding the antigen-binding molecule, as provided above, under conditions suitable for expression of the antigen-binding molecule, and optionally recovering the antigen-binding molecule from the host cell (or host cell culture medium).

For recombinant production of an antigen-binding molecule in the present disclosure, nucleic acid encoding an antigen-binding molecule, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antigen-binding molecule).

Suitable host cells for cloning or expression of antigen-binding molecule-encoding vectors include prokaryotic or eukaryotic cells described herein. For example, antigen-binding molecules may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antigen-binding molecule may be isolated from the bacterial cell paste in a soluble fraction and can be further purified.

In addition to prokaryotes, eukaryotic microbes such as filamentous fungi or yeast are suitable cloning or expression hosts for antigen-binding molecule-encoding vectors, including fungi and yeast strains whose glycosylation pathways have been "humanized," resulting in the production of an antigen-binding molecule with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antigen-binding molecule are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIES™ technology for producing antigen-binding molecules in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod.

23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antigen-binding molecule production, see, e.g., Yazaki and Wu, Methods in Molecular Biology, Vol. 248 (B.K.C. Lo, ed., Humana Press, Totowa, NJ), pp. 255-268 (2003).

Recombinant production of an antigen-binding molecule described herein could be done with methods similar to those described above, by using a host cell that comprises (e.g., has been transformed with) one or plural vectors comprising nucleic acid that encodes an amino acid sequence comprising the whole antigen-binding molecule or part of the antigen-binding molecule.

Assays

Antigen-binding molecules in the present disclosure may be identified, screened for, or characterized for their physical/chemical properties and/or biological activities by various assays known in the art.

a) Binding assays and other assays

In one aspect, an antigen-binding molecule in the present disclosure is tested for its antigen binding activity, e.g., by known methods such as ELISA, Western blot, etc.

b) Activity assays

In one aspect, an antigen-binding molecule may exert any biological activity. Biological activity may include, e.g., activity of holding two antigen molecules at spatially close positions, activity of regulating interaction between two antigen molecules, activity of promoting activation of an receptor by a ligand, activity of promoting catalytic reaction of an enzyme with a substrate, promoting interaction between a cell expressing a first antigen and a cell expressing a second antigen, activity of promoting damage of a target cell by a cell with cytotoxic activity (e.g., a T cell, NK cell, monocyte, or macrophage), activity of regulating activation of two antigen molecules which are activated by association with each other, and resistance to protease cleavage . Antigen-binding molecules having such biological activity in vivo and/or in vitro are also suitably used.

Furthermore, an antigen-binding molecule in the present disclosure can exert various biological activities depending on the type of an antigen molecule to which the antigen-binding molecule binds. Examples of such antigen-binding molecules include an antigen-binding molecule which binds to a T cell receptor (TCR) complex (e.g., CD3) and has activity of inducing T cell activation (agonist activity); and an antigen-binding molecule which binds to a molecule of TNF receptor superfamily (e.g., OX40 or 4-1BB) or of other co-stimulatory molecules (e.g., CD28 or ICOS) and has activity of promoting the above-mentioned activation (agonist activity). In certain embodiments, such biological activity exerted through the binding to an antigen molecule is enhanced or diminished by the linking of two or more antigen-binding domains comprised in the antigen-binding molecule in the present disclosure. Without being limited by theory, in certain embodiments, such enhancement or diminishment may be achieved because the interaction between two or more antigen molecules is regulated through the binding to the antigen-binding molecule in the present disclosure (e.g., the association between two or more antigen molecules is promoted).

In certain embodiments, an antigen-binding molecule in the present disclosure is tested for such biological activity. Whether two antigen molecules are held spatially close can be evaluated using techniques such as crystal structure analysis, electron microscopy, and electron tomography-based structural analysis of a complex composed of antigens and an antigen-binding molecule. Whether two antigen-binding domains are spatially close to each other or whether the mobility of two antigen-binding domains is reduced can also be evaluated by the above-mentioned techniques. In particular, as for techniques to analyze the three-dimensional structure of IgG molecules using electron tomography, see, for example, Zhang et al., Sci. Rep. 5:9803 (2015). In electron tomography, the frequency of occurrence of structures that a subject molecule may form can be shown by histograms, enabling distributional evaluation of structural changes such as reduced mobility of domains. For example, when the relationship between values that can be taken by structure-related parameters, such as distance and angle between two domains, and their frequency of occurrence is shown by histograms, one can determine that the mobility of the two domains is decreased if their areas of distribution are decreased. Activity exerted through interaction and such of two antigen molecules can be evaluated by selecting and using an appropriate activity measurement system from known ones according to the type of target antigen molecules. The effect on protease cleavage can be evaluated using methods known to those skilled in the art, or methods described in the Examples below.

## VI. Preparations, Pharmaceutical Compositions, Pharmaceutical Formulations

The present disclosure relates to preparations comprising an antigen-binding molecule which comprises at least one disulfide bond formed between amino acid residues in regions other than a hinge region, produced by the method described herein. Preparations of an antigen-binding molecule as described herein may be made in the form of a pharmaceutical composition or a pharmaceutical formulation by mixing such an antigen-binding molecule having the desired degree of purity with one or more optional pharmaceutically acceptable carriers (Remington's Pharmaceutical Sciences 16th edition, Osol, A. Ed. (1980)). Thus, the present disclosure relates to pharmaceutical compositions or pharmaceutical formulations comprising a preparation comprising an antigen-binding molecule which comprises at least one disulfide bond formed between amino acid residues in regions other than a hinge region, produced by the method described herein. Pharmaceutical compositions or pharmaceutical formulations in the present disclosure are prepared in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antigen-binding molecule formulations are described in US Patent No. 6,267,958. Aqueous antigen-binding molecule formulations include those described in US Patent No. 6,171,586 and WO2006/044908, the latter formulations including a histidine-acetate buffer.

The pharmaceutical composition or pharmaceutical formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antigen-binding molecule, which matrices are in the form of shaped articles, e.g. films, or microcapsules.

The formulations to be used for in vivo administration are generally sterile. Sterility may be readily accomplished, e.g., by filtration through sterile filtration membranes.

All documents cited herein are incorporated herein by reference.

The following are examples of methods and compositions of the present disclosure. It is understood that various other embodiments may be practiced, given the general description provided above.

[Examples]

## [Example 1] Validation of effectiveness using two types of reducing agents, Cys and TCEP

[Summary]

The inventors developed methods for obtaining an antibody solution having high proportion of LINC format by efficiently applying reduction and oxidation reactions to antigen-binding molecules having artificial cysteine residues in the Fab regions to crosslink the Fabs by the disulfide bond.

[Development method]

Techniques to steadily obtain the LINC format in high-yield were developed by inserting the two steps of "allowing a reduction solution to flow → removing the reducing agent and oxidizing" into the "Protein A affinity chromatography" technique, universally used for primary purification from a cell culture solution (harvested cell culture fluid: HCCF). Details of each step of Protein A affinity chromatography in the method of the present invention are shown in Table 6.

[Table 6]

| | | Solution composition | Flow volume (CV) |
|---|---|---|---|
| Protein A | Antibody adsorption | Cell culture solution (HCCF), 10-20 g/L resin | - |
| | Platform cleansing (removal of impurities) | 25 mmol/L NaCl, 25 mmol/L Tris-HCl (pH 7. 7) | 2 |
| | | 400 mmol/L Na$_2$SO$_4$, 25 mmol/L Tris-HCl (pH 7. 7) | 3 |
| | | 25 mmol/L NaCl, 25 mmol/L Tris-HCl (pH 7. 7) | 2 |
| | Reduction step | Reducing agent solution | 10 |
| | Reducing agent removal oxidation step | 25 mmol/L NaCl, 25 mmol/L Tris-HCl (pH 7.7) | 5 |
| | Antibody elution and recovery | 50 mmol/L Acetic acid | 5 |

[Analysis method]

The proportion of antibodies in the LINC format in a sample as a result of Fab crosslinking was defined as the "LINC ratio".

LINC ratio was calculated as in WO2021/157679. Specifically, the crosslinked LINC format and the non-crosslinked unLINC format (open-type) were separated by non-reducing SDS-PAGE, and the respective abundance ratios were compared.

The procedure for non-reducing SDS-PAGE is the following:

1) a sample diluted to 0.1 mg/mL using PBS was mixed with an equal amount of a sample buffer solution (2ME-) (x2) and then heated at 70°C for 10 minutes;
2) 10 uL of this was loaded onto a 4% SDS-PAGE gel, and subjected to electrophoresis in a Tris/Glycine/SDS buffer at 125 V for 90 minutes;
3) after immobilization in 40% MeOH and 10% AcOH for 10 minutes, this was stained using CBB solution for one minute and then decolorized using MilliQ for 10 minutes; and
4) ChemiDoc Touch MP Imaging system (Bio-Rad Corporation) was used to obtain the image data and to quantify each band; specifically, image data of the decolorized gels were obtained using the instrument itself and each of the bands were quantified using the attached image analysis software ImageQuant LAS400.

Regarding Mab1 (DUAL/LINC (1+2) format, trivalent antibody, chain 1: SEQ ID NO: 54, chain 2: SEQ ID NO: 55, chain 3: SEQ ID NO: 56, chain 4 and chain 5: SEQ ID NO: 57), the culture solution including the antibody was loaded at 10 g/L resin onto a Protein A column (MabSelect SuRe, 1 mL), and chromatography was performed by passing solutions in the order shown in Table 6.

The sequences of the amino acid residues at positions 1-22 of SEQ ID NO: 54, the amino acid residues at positions 1-19 of SEQ ID NO: 55, the amino acid residues at positions 1-19 of SEQ ID NO: 56, and the amino acid residues at positions 1-19 of SEQ ID NO: 57 are expression signal sequences. In the LINC form, a disulfide bond is formed between the amino acid residue at EU numbering position 191 of chain 1 and the amino acid residue at EU numbering position 191 of chain 3. The amino acid residue at EU numbering position 191 of chain 1 corresponds to the amino acid residue at position 444 in SEQ ID NO: 54. The amino acid residue at EU numbering position 191 of chain 3 corresponds to the amino acid residue at position 221 in SEQ ID NO: 56.

In the reduction step, a solution containing Cysteine (Cys) or Tris(2-carboxyethyl)phosphine (TCEP) was allowed to flow. For each of the solutions, the concentration, buffer composition, and pH were as shown in Table 7. In the reduction step, at a rate of column residence time of 12 minutes, the solution was allowed to flow at 10-times the column volume, and the reaction was carried out for 120 minutes.

[Table 7]

| Reducing agent | Reducing agent concentration (mmol/L) | Buffer composition | pH |
|---|---|---|---|
| Cys | 0.1 mmol/L<br>1 mmol/L<br>10 mmol/L<br>100 mmol/L | 50 mmol/L Tris-HCl | pH7<br><br>pH8 |
| TCEP | 0.001 mmol/L<br>0.01 mmol/L<br>0.1 mmol/L<br>1 mmol/L | 20 mmol/L Tris-HCl | pH7<br><br>pH8 |

In the reducing-agent removal/oxidation step, 25 mM NaCl, 25 mM Tris-HCl, pH7.7, containing neither an oxidizing agent nor a reducing agent, was allowed to flow. At a rate of column residence time of 4 minutes, the solution was allowed to flow at 5-times the column volume, and the reaction was carried out for 20 minutes.

[Results]

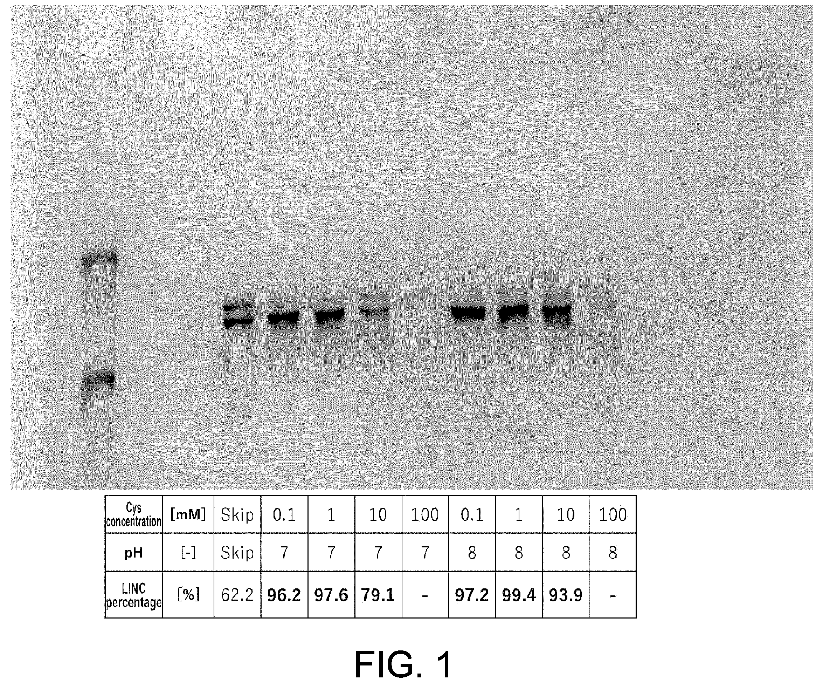

As a result, Cys under conditions of pH 7.0 and 8.0 and at a concentration in the range of 0.1-10 mmol/L was confirmed to increase the LINC ratio (Fig. 1). Furthermore, the chromatographic yield was also confirmed to be about the same (85-104%) as that of the control (92%) which skipped the redox reaction.

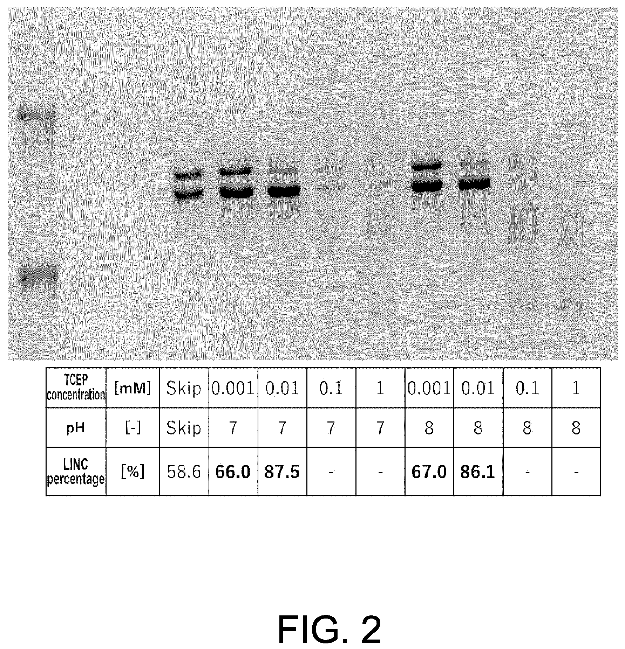

Furthermore, in the case of TCEP as well, conditions where the reducing agent concentration was 0.001-0.01 mmol/L were confirmed to increase the LINC ratio (Fig. 2).

[Conclusion]

Methods of the present invention are techniques that enable the LINC format to be obtained easily as well as quickly and in high yield, and they were found to be applicable to a broad range of conditions (the type of reducing agent can range from Cys with weak reducing power to TCEP with very strong reducing power, the reducing agent concentration may be 1 $\mu$mol/L to 100 mmol/L, and additionally, the ranges of pH, reaction time, and such may also be broad).

[Example 2] Validation of effectiveness for membrane chromatography devices

[Summary]

It was confirmed that the present technique can also be applied to Protein A affinity chromatography that uses a membrane chromatography device.

[Development method]

Table 8 shows details of each step of the membrane Protein A affinity chromatography in the methods of the present invention. Techniques to steadily obtain the LINC format in high yield were developed by introducing two steps which are "allowing a reducing solution to flow $\rightarrow$ removing the reducing agent and oxidizing".

[Table 8]

| | | Solution composition | Flow volume (MV) | Flow rate (MV/min) | Residence time (s) |
|---|---|---|---|---|---|
| Protein A | Antibody adsorption | Cell culture solution (HCCF), 25 g/L resin | - | 5 | 12 |
| | Platform cleansing (removal of impurities) | 25 mmol/L NaCl, 25 mmol/L Tris-HCl (pH 7.7±0.2) | 10 | 5 | 12 |
| | | 400 mmol/L Na$_2$SO$_4$, 25 mmol/L Tris-HCl (pH 7.7±0.2) | 10 | 5 | 12 |
| | | 25 mmol/L NaCl, 25 mmol/L Tris-HCl (pH 7.7±0.2) | 10 | 5 | 12 |
| | Reduction step | Reducing agent solution | 15 | 2 | 30 |
| | Reducing agent removal/ oxidation step | 25 mmol/L NaCl, 25 mmol/L Tris-HCl (pH 7.7±0.2) | 15 | 2 | 30 |
| | Antibody elution and recovery | 50 mmol/L Acetic acid | 10 | 5 | 12 |

[Analysis method]

The proportion of antibodies in the LINC format in a sample as a result of Fab crosslinking was defined as the "LINC ratio".

The LINC form with Fab crosslinking is not digested, but the non-crosslinked unLINC form (Open form) is digested by FabALACTICA (IdgE, a protease produced by Genovis) (a protease that digests the antibody amino acid sequence KSCDKT/HTCPPCP between T and H ("/" indicates the digestion site)), and this molecular characteristic was utilized. For analysis of protease-treated samples, capillary SDS gel electrophoresis (CE-SDS) and hydrophobic interaction chromatography (HIC) were used. Specifically, sample analysis and LINC ratio calculation were carried out by the following procedure:

1) The protease, IdgE, was added to antibody-containing sample solutions.
2) The mixed samples were incubated at 37°C for 18 hours.

In the case of analysis by CE-SDS:

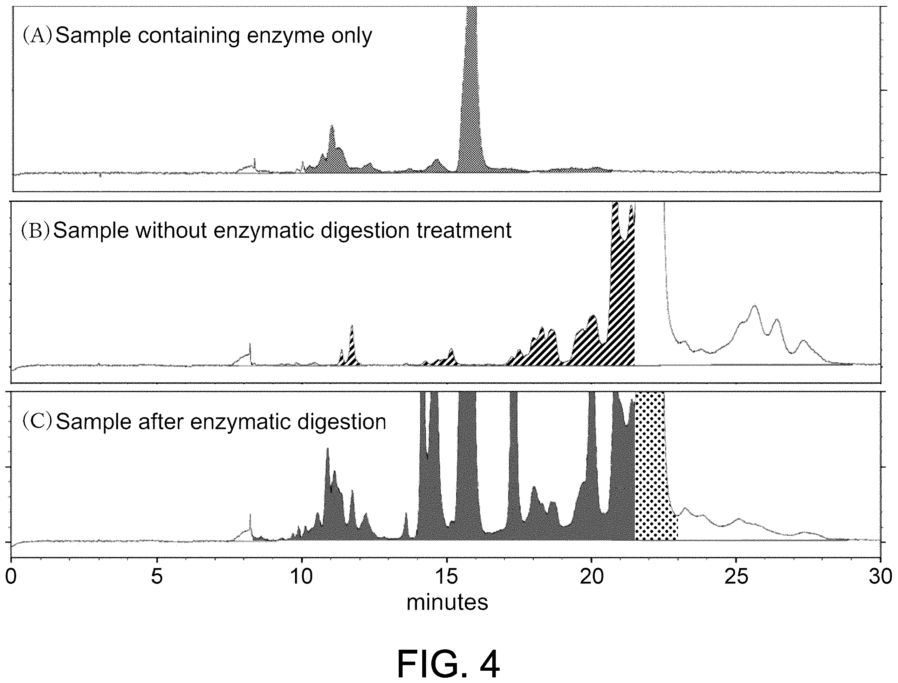

3) The enzymatically digested sample, the sample without enzymatic digestion treatment, and the sample containing the enzyme alone were analyzed by CE-SDS.
4) Computational processing was performed using an electropherogram for the obtained (A) sample containing the enzyme alone, (B) sample without enzymatic digestion treatment, and (C) each sample after enzymatic digestion. (A) and (B) were subtracted from (C) (Fig. 4).
5) The proportion of peak area derived from the LINC form relative to all of the peak areas detected in the subtracted electropherogram obtained in 4) was calculated, and this value was made to be the LINC ratio.

The equation for calculating the LINC ratio is the following:

$$\text{LINC\%} = \frac{\boxtimes \times 100}{\left(\boxed{\text{Pre}} - \boxed{\text{Pre}} - \boxed{\text{Pre}}\right) + \boxtimes}$$

LINC form-derived peak

:

Peaks derived from the enzymatically digested sample containing the unLINC form-derived peaks and the enzyme-derived peaks

:

Peaks derived from the sample prior to enzymatic digestion without the unLINC form-derived peaks

:

Peaks derived from the sample containing the enzyme alone

:

In the case of analysis by HIC:

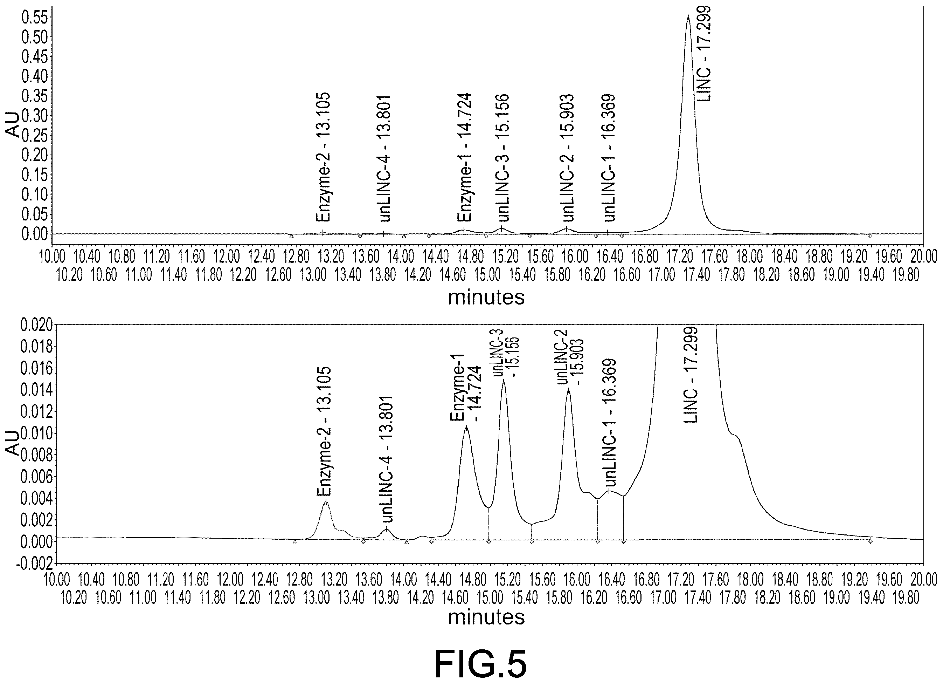

3) The enzymatically digested sample was analyzed by HIC. Mobile phase A used in HIC was a 20 mM phosphate buffer containing 1.5 M ammonium sulfate at pH7.0, and Mobile phase B was a 20 mM phosphate buffer at pH7.0. The gradient for liquid chromatography was as follows: increasing the proportion of Mobile phase B from 2% to 100% over 15 minutes. The column temperature was set to 25 ± 5°C, and the UV detection wavelength was set to 220 nm.
4) The proportion of LINC form-derived peak area relative to the peak areas on the obtained UV chromatogram was calculated, and this value was made to be the LINC ratio (see Fig. 5).

The equation for calculating the LINC ratio is the following:

$$(LINC\%) = (\text{value of area of LINC}) \times 100 \, / \, (Total)$$

(Total) = (value of area of LINC) + (value of area of unLINC-1) + (value of area of unLINC-2) + (value of area of un-LINC-3) + (value of area of unLINC-4)

[Validation of effectiveness for membrane chromatography]

Regarding Mab2 (DUAL/LINC (1+2) format, trivalent antibody, chain 1: SEQ ID NO: 54, chain 2: SEQ ID NO: 55, chain 3: SEQ ID NO: 60, chains 4 and 5: SEQ ID NO: 57), the culture solution including the antibody was loaded on to a membrane chromatography device (Sartbind A, 1.2 mL) at 25 g/L resin, and chromatography was performed.
The sequences of amino acid residues at positions 1 to 22 of SEQ ID NO: 54, the amino acid residues at positions 1 to 19 of SEQ ID NO: 55, the amino acid residues at positions 1 to 19 of SEQ ID NO: 60, and the amino acid residues at positions 1 to 19 of SEQ ID NO: 57 are expression signal sequences. Mab2 from which the expression signal sequences have been removed has chain 1 having the amino acid sequence of SEQ ID NO: 45, chain 2 having the amino acid sequence of SEQ ID NO: 40, chain 3 having the amino acid sequence of SEQ ID NO: 44, and chains 4 and 5 having the amino acid sequence of SEQ ID NO: 42.
In the LINC form, a disulfide bond is formed between the amino acid residue at EU numbering position 191 of chain 1 and the amino acid residue at EU numbering position 191 of chain 3. The amino acid residue at EU numbering position 191 of chain 1 corresponds to the amino acid residue at position 444 in SEQ ID NO: 54, and to the amino acid residue at position 422 in SEQ ID NO: 45. The amino acid residue at EU numbering position 191 of chain 3 corresponds to the amino acid residue at position 221 in SEQ ID NO: 60, and to the amino acid residue at position 202 in SEQ ID NO: 44.
In the reduction step, a solution containing 0.15 mmol/L of the reducing agent (Cys) was allowed to flow. The buffer composition was 0.15 mmol/L L-Cysteine and 50 mmol/L Tris-HCl at pH 8.0, the device residence time of the solution was 30 seconds, the amount that was allowed to flow was 15-times the device volume, and the reaction was carried out for 7.5 minutes.
In the reducing-agent removal/oxidation step, a solution of 25 mM NaCl and 25 mM Tris-HCl at pH7.7 containing neither an oxidizing agent nor a reducing agent was allowed to flow. At a device residence time of 30 seconds, the solution was allowed to flow at 15-times the device volume, and the reaction was carried out for 7.5 minutes.
As a result, the LINC ratio was confirmed to increase under the set conditions (Table 9). Furthermore, the yield by chromatographic purification was confirmed to be comparable to the control which skipped the redox reaction.

[Table 9] LINC ratio of the samples obtained in the Example (the results from the HIC method are shown)

| | LINC ratio (%) |
|---|---|
| Ctrl (reduction reaction skipped) | 70.1 |
| Sample to which the present invention was applied | 83.5 |

[Conclusion]

The methods of the present invention are techniques that allow the LINC format to be obtained easily and quickly, in high yield, and it was confirmed that they can be applied to not only column chromatography but also chromatography in the form of membrane.

[Industrial Applicability]

Methods of the present disclosure are useful in the easy and quick high-yielding production and purification of antigen-binding molecules having appropriate disulfide bonds in regions other than the hinge region.

**Claims**

1. A method for producing a preparation comprising an antigen-binding molecule having at least one disulfide bond formed between amino acid residues in regions other than a hinge region,
   wherein the method comprises subjecting to chromatography in the presence of a reducing agent a mixture which comprises:

   an antigen-binding molecule having at least one disulfide bond formed between amino acid residues in regions other than a hinge region, and
   a mis-disulfide bonded form and/or a non-disulfide bonded form of the antigen-binding molecule.

2. A method for producing a preparation comprising an antigen-binding molecule having at least one disulfide bond formed between amino acid residues in regions other than a hinge region, wherein the method comprises:

   (a) contacting a mixture which comprises:

   an antigen-binding molecule having at least one disulfide bond formed between amino acid residues in regions other than a hinge region, and
   a mis-disulfide bonded form and/or a non-disulfide bonded form of the antigen-binding molecule

   with a reducing agent in chromatography; and
   (b) removing the reducing agent.

3. The method of claim 1 or 2, wherein the antigen-binding molecule comprises two or more polypeptide chains, and the at least one disulfide bond formed between amino acid residues in regions other than a hinge region is formed between the polypeptide chains.

4. The method of claim 3, wherein either or both of the amino acid residues in regions other than a hinge region are mutated, substituted, or introduced cysteine residues.

5. The method of any one of claims 1 to 4, wherein the chromatography comprises an affinity chromatography matrix, an ion exchange chromatography matrix, a hydrophobic interaction chromatography matrix, a multimodal chromatography matrix including both ion exchange chromatography and hydrophobic interaction chromatography, or a hydroxyapatite matrix.

6. The method of claim 5, wherein the affinity chromatography matrix is selected from the group consisting of a protein A matrix, a protein G matrix, a protein L matrix, a sequenceselective peptide matrix, and a matrix that selectively binds to the antigen-binding molecule.

7. The method of claim 5, wherein the ion exchange chromatography matrix is a cation exchange ligand or an anion exchange ligand.

8. The method of claim 5, wherein the hydrophobic interaction chromatography matrix is a hydrophobic ligand.

9. The method of claim 5, wherein the multimodal chromatography matrix is a matrix with a combination of a cation exchange ligand and a hydrophobic ligand, or a matrix with a combination of an anion exchange ligand and a hydrophobic ligand.

10. The method of claim 5, wherein the hydroxyapatite chromatography matrix is hydroxyapatite or a derivative thereof.

11. The method of any one of claims 5 to 10, wherein the chromatography matrix is loaded into a column for column chromatography or applied onto a membrane for membrane chromatography.

12. The method of any one of claims 1 to 11, wherein the reducing agent is selected from the group consisting of a monothiol, a dithiol, a phosphine, and an inorganic reagent, and a combination of two or more thereof.

13. The method of any one of claims 1 to 12, wherein the reducing agent is cysteine and/or TCEP.

14. The method of any one of claims 1 to 13, wherein the reducing agent is TCEP and the concentration of the reducing agent is 0.00001 mM to 10.0 mM.

15. The method of any one of claims 1 to 13, wherein the reducing agent is cysteine and the concentration of the reducing agent is 0.01 mM to 100 mM.

16. The method of any one of claims 2 to 15, wherein the removal of the reducing agent comprises contacting the antigen-binding molecule with a solution that does not contain the reducing agent.

17. The method of any one of claims 1 to 16, wherein the antigen-binding molecule comprises a first antigen-binding domain and a second antigen-binding domain which can be linked with each other via at least one disulfide bond.

18. The method of claim 17, wherein the at least one disulfide bond formed between amino acid residues in regions other than a hinge region is formed between the first antigen-binding domain and the second antigen-binding domain.

19. The method of claim 17 or 18, wherein the at least one disulfide bond formed between amino acid residues in regions other than a hinge region is formed between a heavy chain of the first antigen-binding domain and a heavy chain of the second antigen-binding domain.

20. The method of any one of claims 17 to 19, wherein the at least one disulfide bond formed between amino acid residues in regions other than a hinge region is formed between a CH1 region of the first antigen-binding domain and a CH1 region of the second antigen-binding domain.

21. The method of any one of claims 17 to 20, wherein the at least one disulfide bond formed between amino acid residues in regions other than a hinge region is formed between an amino acid residue at EU numbering position 191 in a heavy chain of the first antigen-binding domain and an amino acid residue at EU numbering position 191 in a heavy chain of the second antigen-binding domain.

22. The method of any one of claims 17 to 21, wherein the first antigen-binding domain and the second antigen-binding domain bind to a first antigen and a second antigen, respectively, which are proteins present on the surface of a cell, and wherein the antigen-binding molecule has activity of promoting interaction between the cell expressing the first antigen and the cell expressing the second antigen.

23. The method of claim 22, wherein the cell expressing the first antigen is a cell with cytotoxic activity and the cell expressing the second antigen is a target cell thereof, and wherein the antigen-binding molecule promotes damage to the target cell by the cell with cytotoxic activity.

24. The method of claim 23, wherein the cell with cytotoxic activity is a T cell, a NK cell, a monocyte, or a macrophage.

25. The method of any one of claims 22 to 24, wherein the first and second antigens are independently selected from the group consisting of a receptor belonging to the cytokine receptor superfamily, a G protein-coupled receptor, an ion channel-type receptor, a tyrosine kinase-type receptor, an immune checkpoint receptor, an antigen receptor, a CD antigen, a costimulatory molecule, and a cell adhesion molecule.

26. The method of any one of claims 17 to 25, wherein the first antigen-binding domain and the second antigen-binding domain are each capable of binding to CD3 and/or CD137.

27. The method of any one of claims 1 to 26, wherein the antigen-binding molecule further comprises a third antigen-binding domain.

28. The method of claim 27, wherein the third antigen-binding domain is fused to either the first antigen-binding domain or the second antigen-binding domain.

29. The method of claim 27 or 28, wherein the third antigen-binding domain is a Fab or an scFv.

30. The method of any one of claims 27 to 29, wherein the third antigen-binding domain is fused, at its C-terminus, to the N-terminus of the Fab heavy chain (VH region) of either the first antigen-binding domain or the second antigen-binding domain, optionally via a peptide linker.

31. The method of any one of claims 27 to 30, wherein the first antigen-binding domain, the second antigen-binding domain, and the third antigen-binding domain are each a Fab molecule, wherein the third antigen-binding domain is fused, at the C-terminus of its Fab heavy chain (CH1 region), to the N-terminus of the Fab heavy chain (VH region) of either the first antigen-binding domain or the second antigen-binding domain, optionally via a peptide linker.

32. The method of claim 30 or 31, wherein the peptide linker comprises an amino acid sequence selected from the group consisting of the amino acid sequences of SEQ ID NO: 18, SEQ ID NO: 19, and SEQ ID NO: 20.

33. The method of any one of claims 27 to 32, wherein the third antigen-binding domain is a crossover Fab molecule in which the variable regions of the Fab light chain and the Fab heavy chain are exchanged, and wherein the first antigen-binding domain and the second antigen-binding domain are conventional Fab molecules.

34. The method of any one of claims 27 to 33, wherein the third antigen-binding domain is capable of binding to an antigen expressed on a cancer cell or cancer tissue.

35. The method of any one of claims 27 to 34, wherein the third antigen-binding domain is capable of binding to DLL3, preferably human DLL3.

36. The method of any one of claims 1 to 35, wherein the antigen-binding molecule further comprises an Fc region.

37. The method of any one of claims 17 to 36, wherein the first antigen-binding domain and the second antigen-binding domain each comprise an antibody variable region that may be identical to or different from each other, and comprise an antibody variable region independently selected from the group consisting of (a1) to (a4) below:

   (a1) an antibody variable region comprising:

      a heavy chain variable region comprising:

         heavy chain complementarity determining region (CDR) 1 comprising the amino acid sequence of SEQ ID NO: 27,
         heavy chain CDR 2 comprising the amino acid sequence of SEQ ID NO: 28, and
         heavy chain CDR 3 comprising the amino acid sequence of SEQ ID NO: 29; and

      a light chain variable region comprising:

         light chain CDR 1 comprising the amino acid sequence of SEQ ID NO: 30,
         light chain CDR 2 comprising the amino acid sequence of SEQ ID NO: 31, and
         light chain CDR 3 comprising the amino acid sequence of SEQ ID NO: 32;

(a2) an antibody variable region comprising:

a heavy chain variable region comprising:

heavy chain complementarity determining region (CDR) 1 comprising the amino acid sequence of SEQ ID NO: 33,
heavy chain CDR 2 comprising the amino acid sequence of SEQ ID NO: 34, and
heavy chain CDR 3 comprising the amino acid sequence of SEQ ID NO: 35; and

a light chain variable region comprising:

light chain CDR 1 comprising the amino acid sequence of SEQ ID NO: 30,
light chain CDR 2 comprising the amino acid sequence of SEQ ID NO: 31, and
light chain CDR 3 comprising the amino acid sequence of SEQ ID NO: 32;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

38. The method of any one of claims 17 to 37, wherein the first antigen-binding domain and the second antigen-binding domain each comprise an antibody variable region that may be identical to or different from each other, and comprise an antibody variable region independently selected from the group consisting of (a1) to (a4) below:

(a1) an antibody variable region comprising:

a heavy chain variable region comprising the amino acid sequence of SEQ ID NO: 36, and
a light chain variable region comprising the amino acid sequence of SEQ ID NO: 37;

(a2) an antibody variable region comprising:

a heavy chain variable region comprising the amino acid sequence of SEQ ID NO: 38, and
a light chain variable region comprising the amino acid sequence of SEQ ID NO: 37;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

39. The method of any one of claims 27 to 38, wherein the third antigen-binding domain comprises an antibody variable region independently selected from the group consisting of (a1) to (a4) below:

(a1) an antibody variable region comprising:

a heavy chain variable region comprising:

heavy chain complementarity determining region (CDR) 1 comprising the amino acid sequence of SEQ ID NO: 46,
heavy chain CDR 2 comprising the amino acid sequence of SEQ ID NO: 47, and
heavy chain CDR 3 comprising the amino acid sequence of SEQ ID NO: 48; and

a light chain variable region comprising:

light chain CDR 1 comprising the amino acid sequence of SEQ ID NO: 49,
light chain CDR 2 comprising the amino acid sequence of SEQ ID NO: 50, and
light chain CDR 3 comprising the amino acid sequence of SEQ ID NO: 51;

(a2) an antibody variable region comprising:

a heavy chain variable region comprising the amino acid sequence of SEQ ID NO: 52, and
a light chain variable region comprising the amino acid sequence of SEQ ID NO: 53;

(a3) an antibody variable region that binds to the same epitope as that to which the antibody variable region of (a1) or (a2) binds; and
(a4) an antibody variable region that competes with the antibody variable region of (a1) or (a2) for binding to an antigen.

40. The method of any one of claims 17 to 39, wherein the first antigen-binding domain and the second antigen-binding domain each comprise an antibody variable region comprising:

a heavy chain variable region comprising:

heavy chain complementarity determining region (CDR) 1 comprising the amino acid sequence of SEQ ID NO: 27,
heavy chain CDR 2 comprising the amino acid sequence of SEQ ID NO: 28, and
heavy chain CDR 3 comprising the amino acid sequence of SEQ ID NO: 29; and

a light chain variable region comprising:

light chain CDR 1 comprising the amino acid sequence of SEQ ID NO: 30,
light chain CDR 2 comprising the amino acid sequence of SEQ ID NO: 31, and
light chain CDR 3 comprising the amino acid sequence of SEQ ID NO: 32.

41. The method of any one of claims 17 to 40, wherein the first antigen-binding domain and the second antigen-binding domain each comprise an antibody variable region comprising:

a heavy chain variable region comprising the amino acid sequence of SEQ ID NO: 36, and
a light chain variable region comprising the amino acid sequence of SEQ ID NO: 37.

42. The method of any one of claims 27 to 41, wherein the third antigen-binding domain comprises an antibody variable region comprising:

a heavy chain variable region comprising:

heavy chain complementarity determining region (CDR) 1 comprising the amino acid sequence of SEQ ID NO: 46,
heavy chain CDR 2 comprising the amino acid sequence of SEQ ID NO: 47, and
heavy chain CDR 3 comprising the amino acid sequence of SEQ ID NO: 48; and

a light chain variable region comprising:

light chain CDR 1 comprising the amino acid sequence of SEQ ID NO: 49,
light chain CDR 2 comprising the amino acid sequence of SEQ ID NO: 50, and
light chain CDR 3 comprising the amino acid sequence of SEQ ID NO: 51.

43. The method of any one of claims 27 to 42, wherein the third antigen-binding domain comprises an antibody variable region comprising:

a heavy chain variable region comprising the amino acid sequence of SEQ ID NO: 52, and
a light chain variable region comprising the amino acid sequence of SEQ ID NO: 53.

44. The method of any one of claims 27 to 43, wherein the first antigen-binding domain and the second antigen-binding domain each comprise an antibody variable region comprising:

a heavy chain variable region comprising the amino acid sequence of SEQ ID NO: 36, and
a light chain variable region comprising the amino acid sequence of SEQ ID NO: 37, and wherein the third antigen-binding domain comprises an antibody variable region comprising:

a heavy chain variable region comprising SEQ ID NO: 52, and
a light chain variable region comprising SEQ ID NO: 53.

45. The method of any one of claims 17 to 44, wherein the first antigen-binding domain and the second antigen-binding domain are each a Fab having a cysteine residue at EU numbering position 191 in the heavy chain, and have a disulfide bond formed by the two cysteine residues.

46. The method of any one of claims 1 to 45, wherein the antigen-binding molecule comprises any one combination of five polypeptide chains selected from the group consisting of:

(a1) a polypeptide chain comprising the amino acid sequence of SEQ ID NO: 39 (chain 1),

a polypeptide chain comprising the amino acid sequence of SEQ ID NO: 40 (chain 2),
a polypeptide chain comprising the amino acid sequence of SEQ ID NO: 41 (chain 3), and
two polypeptide chains each comprising the amino acid sequence of SEQ ID NO: 42 (chain 4 and chain 5);

(a2) a polypeptide chain comprising the amino acid sequence of SEQ ID NO: 43 (chain 1),

a polypeptide chain comprising the amino acid sequence of SEQ ID NO: 40 (chain 2),
a polypeptide chain comprising the amino acid sequence of SEQ ID NO: 44 (chain 3), and
two polypeptide chains each comprising the amino acid sequence of SEQ ID NO: 42 (chain 4 and chain 5); and

(a3) a polypeptide chain comprising the amino acid sequence of SEQ ID NO: 45 (chain 1),

a polypeptide chain comprising the amino acid sequence of SEQ ID NO: 40 (chain 2),
a polypeptide chain comprising the amino acid sequence of SEQ ID NO: 44 (chain 3), and
two polypeptide chains each comprising the amino acid sequence of SEQ ID NO: 42 (chain 4 and chain 5);

wherein, preferably, the five polypeptide chains (chains 1 to 5) are connected and/or associated with each other according to the orientation shown in Fig. 3.

47. A preparation produced by the method of any one of claims 1 to 46, which comprises an antigen-binding molecule having at least one disulfide bond formed between amino acid residues in regions other than a hinge region.

48. A pharmaceutical composition comprising a preparation produced by the method of any one of claims 1 to 46, wherein the preparation comprises an antigen-binding molecule having at least one disulfide bond formed between amino acid residues in regions other than a hinge region.

FIG. 1

| TCEP concentration | [mM] | Skip | 0.001 | 0.01 | 0.1 | 1 | 0.001 | 0.01 | 0.1 | 1 |
|---|---|---|---|---|---|---|---|---|---|---|
| pH | [-] | Skip | 7 | 7 | 7 | 7 | 8 | 8 | 8 | 8 |
| LINC percentage | [%] | 58.6 | **66.0** | **87.5** | - | - | **67.0** | **86.1** | - | - |

# FIG. 2

Trivalent Ab (1+2 Dual/LINC)

LINC (engineered disulfide linkage)

Chain 3

Chain 1

Chain 2

Fv A (Outer)

Fv B (Inner)

Linker

Chain 5

Fc region

Fv C

Chain 4

Variable heavy chain domain (VH)
Variable light chain domain (VL)
Constant heavy chain domain 1 (CH1)
Constant light chain domain (CL)

# FIG. 3

(A) Sample containing enzyme only

(B) Sample without enzymatic digestion treatment

(C) Sample after enzymatic digestion

minutes

FIG. 4

EP 4 624 489 A1

FIG.5

# EP 4 624 489 A1

## INTERNATIONAL SEARCH REPORT

| International application No. |
| --- |
| **PCT/JP2023/042131** |

### A. CLASSIFICATION OF SUBJECT MATTER

*C07K 16/00*(2006.01)i; *A61K 39/395*(2006.01)i; *A61P 35/00*(2006.01)i; *C07K 16/28*(2006.01)i; *C07K 16/46*(2006.01)i; *C12N 15/13*(2006.01)n
FI: C07K16/00 ZNA; C07K16/28; C07K16/46; A61K39/395 N; A61K39/395 T; A61P35/00; C12N15/13

According to International Patent Classification (IPC) or to both national classification and IPC

### B. FIELDS SEARCHED

Minimum documentation searched (classification system followed by classification symbols)

C07K16/00; A61K39/395; A61P35/00; C07K16/28; C07K16/46; C12N15/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2021/201087 A1 (CHUGAISEIYAKU KABUSHIKIKAISHA) | 1-48 |
|  | claims 1-3, 5, 6, 8, 9, 12, 16, paragraphs [0020]-[0023], [0131]-[0136], [0314], fig. 1 |  |
| Y | claims 1-3, 5, 6, 8, 9, 12, 16, paragraphs [0020]-[0023], [0131]-[0136], [0314], fig. 1 | 1-48 |
| Y | GHOSE, S. et al. Modeling on-column reduction of trisulfide bonds in monoclonal antibodies during protein A chromatography. Journal of Chromatography A. 2017, vol. 1479, pp. 81-86, <http://dx.doi.org/10.1016/j.chroma.2016.11.019> abstract, section of 2.3.1 | 1-48 |
| Y | AONO, H. et al. Efficient on-column conversion of IgG1 trisulfide linkages to native disulfides in tandem with Protein A affinity chromatography. Journal of Chromatography A. 2010, vol. 1217, pp. 5225-5232, <doi:10.1016/j.chroma.2010.06.029> abstract, section of 2.2 | 1-48 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 February 2024** | **20 February 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
|---|
| **PCT/JP2023/042131** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2023/042131**

| Patent document cited in search report | Publication date (day/month/year) | Patent family member(s) | Publication date (day/month/year) |
|---|---|---|---|
| WO 2021/201087 A1 | | JP 2023-519776 A | |
| | | US 2023/0121511 A1 | |
| | | CN 115335410 A | |
| | | KR 10-2022-0161375 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020027330 A
- WO 2021157679 A
- WO 2021200898 A
- WO 2021201087 A
- WO 2020037016 A
- WO 2006047340 A
- WO 9316185 A
- US 5571894 A
- US 5587458 A
- US 5869046 A
- EP 404097 A
- WO 199301161 A
- US 6248516 B1
- WO 1988001649 A
- US 4946778 A
- US 5260203 A
- WO 2006132352 A
- WO 2015143414 A
- US 20110123527 A1
- WO 2009011941 A
- WO 2019131988 A
- WO 2011093097 A
- WO 2013126746 A
- US 5731168 A
- US 7695936 B
- WO 2016164480 A
- WO 2006106905 A
- WO 2011028952 A
- WO 2014018572 A
- WO 2008119353 A
- WO 2011131746 A
- WO 2012058768 A
- WO 2013063702 A
- WO 2012023053 A
- WO 2007114325 A
- WO 98050431 A
- WO 95033844 A
- WO 9308829 A
- WO 2009089004 A1
- US 4676980 A
- US 20060025576 A1
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 5500362 A
- WO 5821337 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 4816567 A
- US 5648237 A
- US 5789199 A
- US 5840523 A
- US 5959177 A
- US 6040498 A
- US 6420548 B
- US 7125978 B
- US 6417429 B
- US 20050260186 A
- US 20060104968 A
- US 6267958 B
- US 6171586 B
- WO 2006044908 A

**Non-patent literature cited in the description**

- Removal of Cysteinylation from an Unpaired Sulfhydryl in the Variable Region of a Recombinant Monoclonal IgG1 Antibody Improves Homogeneity, Stability, and Biological Activity. *Pharm Sci.*, February 2008, vol. 97 (2), 775-90
- **SAMBROOK et al.** Molecular Cloning: A Laboratory Manual. Cold Spring Harbor Laboratory Press, 2001
- Current Protocols in Molecular Biology. 2003
- PCR 2: A Practical Approach. Methods in Enzymology. Academic Press, Inc., 1995
- Antibodies, A Laboratory Manual. 1988
- Animal Cell Culture. 1987
- Oligonucleotide Synthesis. 1984
- Methods in Molecular Biology. Humana Press
- Cell Biology: A Laboratory Notebook. Academic Press, 1998
- **J. P. MATHER** ; **P.E. ROBERTS**. Introduction to Cell and Tissue Culture. Plenum Press, 1998
- Cell and Tissue Culture: Laboratory Procedures. J. Wiley and Sons, August 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY** ; **P. TRAVERS**. Immunobiology. 1997
- **P. FINCH**. *Antibodies*, 1997
- Antibodies: A Practical Approach. IRL Press, 1988
- Monoclonal Antibodies: A Practical Approach. Oxford University Press, 2000
- **E. HARLOW** ; **D. LANE**. Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **KABAT et al.** Sequences of Proteins of Immunological Interest. Public Health Service, National Institutes of Health, 1991
- **KUNKEL et al.** *Proc. Natl. Acad. Sci. USA*, 1985, vol. 82, 488-492
- *Annu Rev. Biophys. Biomol. Struct.*, 2006, vol. 35, 225-249
- *Proc. Natl. Acad. Sci. U.S.A.*, 2003, vol. 100 (11), 6353-6357
- **KINDT et al.** Kuby Immunology. W.H. Freeman and Co., 2007, 91
- **PORTOLANO et al.** *J. Immunol.*, 1993, vol. 150, 880-887
- **CLARKSON et al.** *Nature*, 1991, vol. 352, 624-628
- **CHOTHIA** ; **LESK**. *J. Mol. Biol.*, 1987, vol. 196, 901-917
- **MACCALLUM et al.** *J. Mol. Biol.*, 1996, vol. 262, 732-745
- **KABAT et al.** Sequences of Proteins of Immunological Interest. 1991, vol. 1-3
- **BREKKE et al.** *Immunol (See Table 1 of Today*, 1995, vol. 16, 85-90
- **HUDSON et al.** *Nat Med*, 2003, vol. 9, 129-134
- **PLUCKTHUN**. The Pharmacology of Monoclonal Antibodies. Springer-Verlag, 1994, vol. 113, 269-315
- **HOLLINGER et al.** *Proc Natl Acad Sci USA*, 1993, vol. 90, 6444-6448
- *Science*, 1988, vol. 240 (4855), 1038-1041
- *Proc. Natl. Acad. Sci. U.S.A.*, 1988, vol. 85 (16), 5879-5883
- *J Immunol. Methods*, 1999, vol. 231 (1-2), 177-189
- *Journal of Immunology*, 1994, vol. 152 (11), 5368-5374
- *Protein Engineering*, 1996, vol. 9 (3), 299-305
- *Methods in Molecular Biology*, 2012, vol. 911, 65-78
- *Biochimica et Biophysica Acta - Proteins and Proteomics*, 2006, vol. 1764 (8), 1307-1319
- *Journal of Applied Microbiology*, 2014, vol. 117 (2), 528-536
- *Journal of Biomolecular Screening*, 2016, vol. 21 (1), 35-43
- *Journal of Biological Chemistry*, 2016, vol. 291 (24), 12641-12657
- *AIDS*, 2016, vol. 30 (11), 1691-1701
- *J. Rheumatol*, 2007, vol. 34, 11
- *Hybridomas*, 1990, vol. 1 (1), 47-54
- *Blood*, 2007, vol. 109, 1185-1192
- **PERCZEL et al.** *Protein Engrg.*, 1991, vol. 4, 669-679
- **SPOERRY C et al.** *PLoS ONE*, 2016, vol. 11 (10), e0164809
- **RIDGWAY et al.** *Prot Eng*, 1996, vol. 9, 617-621
- **CARTER**. *J Immunol Meth*, 2001, vol. 248, 7-15
- **CARTER**. *J Immunol Methods*, 2001, vol. 248, 7-15
- *Protein Engineering Design & Selection*, 2010, vol. 23, 195-202
- *Nat Biotechnol.*, February 2014, vol. 32 (2), 191-8
- **CHRISTOPH et al.** *Nature Biotechnology*, 2013, vol. 31, 753-758
- *BMC Research Notes*, 2011, vol. 4, 281

- *Proc. Natl. Acad. Sci. USA*, 2006, vol. 103 (11), 4005-4010
- Epitope Mapping Protocols. **MORRIS**. Methods in Molecular Biology. Humana Press, 1996, vol. 66
- **HARLOW** ; **LANE**. Antibodies: A Laboratory Manual. Cold Spring Harbor Laboratory, 1988
- **MILSTEIN** ; **CUELLO**. *Nature*, 1983, vol. 305, 537
- **TRAUNECKER et al.** *EMBO J.*, 1991, vol. 10, 3655
- **BRENNAN et al.** *Science*, 1985, vol. 229, 81
- **KOSTELNY et al.** *J. Immunol.*, 1992, vol. 148 (5), 1547-1553
- **HOLLINGER et al.** *Proc. Natl. Acad. Sci. USA*, 1993, vol. 90, 6444-6448
- **GRUBER et al.** *J. Immunol.*, 1994, vol. 152, 5368
- **TUTT et al.** *J. Immunol.*, 1991, vol. 147, 60
- **CHOWDHURY**. *Methods Mol. Biol.*, 2008, vol. 207, 179-196
- **HOOGENBOOM et al.** Methods in Molecular Biology. Human Press, 2001, vol. 178, 1-37
- **CUNNINGHAM** ; **WELLS**. *Science*, 1989, vol. 244, 1081-1085
- **WRIGHT et al.** *TIBTECH*, 1997, vol. 15, 26-32
- **OKAZAKI et al.** *J. Mol. Biol.*, 2004, vol. 336, 1239-1249
- **YAMANE-OHNUKI et al.** *Biotech. Bioeng.*, 2004, vol. 87, 614
- **RIPKA et al.** *Arch. Biochem. Biophys.*, 1986, vol. 249, 533-545
- **KANDA, Y. et al.** *Biotechnol. Bioeng.*, 2006, vol. 94 (4), 680-688
- **RAVETCH** ; **KINET**. *Annu. Rev. Immunol.*, 1991, vol. 9, 464, 457-492
- **HELLSTROM, I. et al.** *Proc. Nat'l Acad. Sci. USA*, 1986, vol. 83, 7059-7063
- **HELLSTROM, I et al.** *Proc. Nat'l Acad. Sci. USA*, 1985, vol. 82, 1499-1502
- **BRUGGEMANN, M. et al.** *J. Exp. Med.*, 1987, vol. 166, 1351-1361
- **CLYNES et al.** *Proc. Nat'l Acad. Sci. USA*, 1998, vol. 95, 652-656
- **GAZZANO-SANTORO et al.** *J. Immunol. Methods*, 1996, vol. 202, 163
- **CRAGG, M.S. et al.** *Blood*, 2003, vol. 101, 1045-1052
- **CRAGG, M.S.** ; **M.J. GLENNIE**. *Blood*, 2004, vol. 103, 2738-2743
- **PETKOVA, S.B. et al.** *Int'l. Immunol.*, 2006, vol. 18 (12), 1759-1769
- **SHIELDS et al.** *J. Biol. Chem.*, 2001, vol. 9 (2), 6591-6604
- **IDUSOGIE et al.** *J. Immunol.*, 2000, vol. 164, 4178-4184
- **GUYER et al.** *J. Immunol.*, 1976, vol. 117, 587
- **KIM et al.** *J. Immunol.*, 1994, vol. 24, 249
- **DUNCAN** ; **WINTER**. *Nature*, 1988, vol. 322, 738-40
- **KAM et al.** *Proc. Natl. Acad. Sci. USA*, 2005, vol. 102, 11600-11605
- **CHARLTON**. Methods in Molecular Biology. Humana Press, 2003, vol. 248, 245-254
- **GERNGROSS**. *Nat. Biotech.*, 2004, vol. 22, 1409-1414
- **LI et al.** *Nat. Biotech.*, 2006, vol. 24, 210-215
- **GRAHAM et al.** *J. Gen Virol.*, 1977, vol. 36, 59
- **MATHER**. *Biol. Reprod.*, 1980, vol. 23, 243-251
- **MATHER et al.** *Annals N.Y. Acad. Sci.*, 1982, vol. 383, 44-68
- **URLAUB et al.** *Proc. Natl. Acad. Sci. USA*, 1980, vol. 77, 4216
- **YAZAKI** ; **WU**. Methods in Molecular Biology. Humana Press, 2003, vol. 248, 255-268
- **ZHANG et al.** *Sci. Rep.*, 2015, vol. 5, 9803
- Remington's Pharmaceutical Sciences. 1980